# EUROPÄISCHE PATENTSCHRIFT

(11) **EP 1 778 668 B1**
(45) Veröffentlichungstag und Bekanntmachung des Hinweises auf die Patenterteilung: **03.07.2013**
(21) Anmeldenummer: 05771218.4
(22) Anmeldetag: 11.08.2005
(51) Int. Cl.: C07D 401/04

(54) **VERFAHREN ZUR HERSTELLUNG VON DIHYDROPTERIDINONEN**
METHOD FOR THE PRODUCTION OF DIHYDROPTERIDINONES
PROCEDE DE PRODUCTION DE DIHYDROPTERIDINONES

(30) Priorität: 14.08.2004 EP 04019365; 27.01.2005 EP 05001611
(43) Veröffentlichungstag der Anmeldung: 02.05.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: LINZ, Guenter, 88441 MITTELBIBERACH (DE); KRAEMER, Gerd F., 88436 EBERHARDZELL (DE); GUTSCHERA, Ludwig, 89079 ULM (DE); ASCHE, Geert, 88400 Biberach (DE)
(74) Vertreter: Simon, Elke Anna Maria
(86) Internationale Anmeldenummer: PCT/EP2005/008734
(87) Internationale Veröffentlichungsnummer: WO 2006/018220

(56) Entgegenhaltungen:
- WO-A-03/020722
- WO-A-2004/076454

## Beschreibung

Die Erfindung betrifft ein Verfahren zur Herstellung von Dihydropteridinonen der allgemeinen Formel (I) 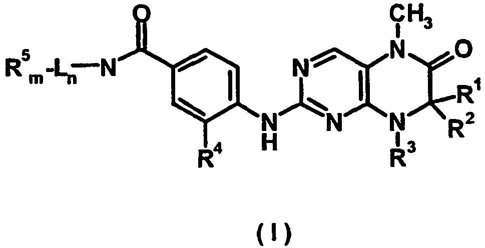 wobei die Reste L, R¹, R², R³, R⁴ und R⁵ die in den Ansprüchen und der Beschreibung genannten Bedeutungen haben

### Hintergrund der Erfindung

Pteridinon-Derivate sind als Wirkstoffe mit antiproliferativer Wirkung aus dem Stand der Technik bekannt. WO 03/020722 beschreibt die Verwendung von Dihydropteridinonderivaten zur Behandlung von Tumorerkrankungensowie Verfahren zu deren Herstellung.

Es ist die Aufgabe der vorliegenden Erfindung ein verbessertes Verfahren zur Herstellung der erfindungsgemäßen Dihydropteridinone bereitzustellen.

### Detaillierte Beschreibung der Erfindung

Die vorliegende Erfindung löst die vorstehend genannte Aufgabe über das im folgenden beschriebene Syntheseverfahren, welches im Vergleich zu dem in WO 03/020722 beschriebenen Verfahren ein konvergentes Verfahren darstellt. Gegenstand der Erfindung ist somit ein Verfahren zur Herstellung von Dihydropteridinonen der allgemeinen Formel (I) 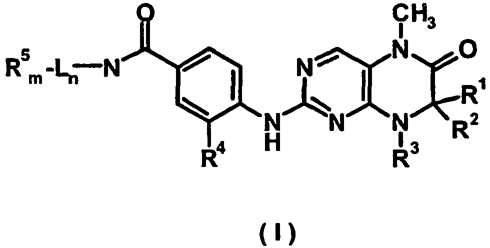 worin
R¹, R² gleich oder verschieden Wasserstoff oder gegebenenfalls substituiertes C₁-C₆-Alkyl,
oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl, C₅-C₁₂-Spirocycloalkyl, C₃-C₁₂-Heterocycloalkyl, das 1 bis 2 Heteroatome enthält, und C₃-C₁₂-Heterocycloalkenyl, das 1 bis 2 Heteroatome enthält, oder
R¹ und R³ oder R² und R³ gemeinsam eine gesättigte oder ungesättigte C₃-C₄-Alkylbrücke, die gegebenenfalls 1 Heteroatom enthalten kann,
R⁴ ein Rest ausgewählt aus der Gruppe bestehend aus Wasserstoff, -CN, Hydroxy, -NR₆R₇ und Halogen, oder
ein Rest ausgewählt aus der Gruppe bestehend aus gegebenenfalls substituiertem C₁-C₆-Alkyl, C₂-C₆-Alkenyl, C₂-C₆-Alkinyl, C₁-C₅-Alkyloxy, C₂-C₅-Alkenyloxy, C₂-C₅-Alkinyloxy, C₁-C₆-Alkylthio, C₁-C₆-Alkylsulfoxo und C₁-C₆-Alkylsulfonyl,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, - C₆-C₁₄-Aryl-C₁-C₄-Alkyl, gegebenenfalls verbrücktem C₃-C₁₂-Cycloalkyl und Heteroaryl, das gegebenenfalls 1 oder 2 Stickstoffatome enthält,
- n: 0 oder 1
- m: 1 oder 2
R⁵ ein Rest, ausgewählt aus der Gruppe bestehend aus gegebenenfalls substituiertem Morpholinyl, Piperidinyl, Piperazinyl, Piperazinylcarbonyl, Pyrrolidinyl, Tropenyl, R⁸-Diketomethylpiperazinyl, Sulfoxomorpholinyl, Sulfonylmorpholinyl, Thiomorpholinyl, -NR⁸R⁹ und Azacycloheptyl,
R⁶, R⁷ gleich oder verschieden, Wasserstoff oder C₁-C₄-Alkyl,
und
R⁸, R⁹ unsubstituierte Stickstoffsubstituenten an R⁵, gleich oder verschieden, entweder Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkysulfonyl- und C₆-C₁₄-Aryl-C₁-C₄-Alkylsulfonyl-, bedeuten,
wobei eine Verbindung der Formel (II) 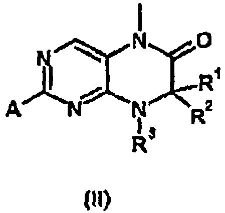 worin
R¹ bis R³ die angegebene Bedeutung aufweisen und A eine Abgangsgruppe ist,
mit einer Verbindung der Formel (III), 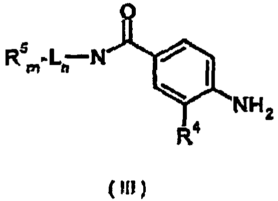 worin
R⁴, R⁵, L und m, n die angegebene Bedeutung aufweiten können, in Gegenwart organischer Sulfowsäuren umfasstet wird.

Ein weiterer Gegenstand der Erfindung ist ein Verfahren zur Herstellung von Dihydropteridinonen der allgemeinen Formel (I) wie vorhin beschrieben, wobei R₅ ein Morpholinyl oder Piperazinyl Rest darstellt, der mit einer Alkyl- oder CycloalkylGruppe, mono- oder di-substituiert sein kann, bevorzugt Cyclopropylmethyl oder Dimethyl.

Ein weiterer Gegenstand der Erfindung ist ein Verfahren zur Herstellung von Dihydropteridinonen der allgemeinen Formel (I) wie vorhin beschrieben, wobei die Dihydropteridinone ausgewählt ist aus der Gruppe bestehend aus folgende Dihydropteridinone der allgemeinen Formel (I)

| | | | | | | |
|---|---|---|---|---|---|---|
| 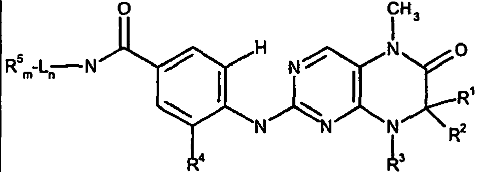 | | | | | | |

| Bsp. | R¹ | R² | Konfig. R¹ od. R² | R³ | R⁴ | Lₙ-R⁵ₘ |
|---|---|---|---|---|---|---|
| 27 | H | 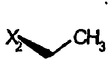 | R | 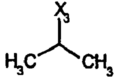 | 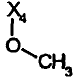 | 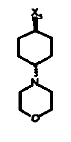 |
| 44 | H | 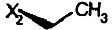 | R | 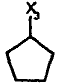 | H | 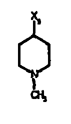 |
| 55 | H | 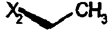 | R | 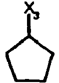 | 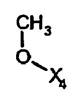 | 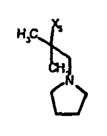 |
| 58 | H | 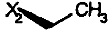 | R | 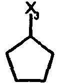 | 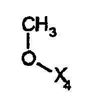 | 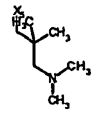 |
| 102 | H | 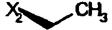 | R | 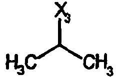 | 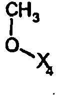 | 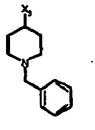 |
| 103 | H | 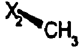 | R | 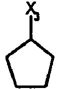 | 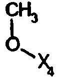 | 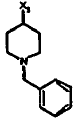 |
| 105 | H | 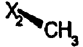 | R | 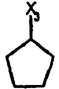 | 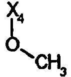 | 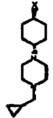 |
| 110 | H | 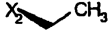 | R | 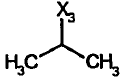 | 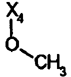 | 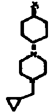 |
| 115 | H | 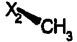 | R | 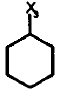 | 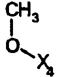 | 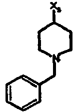 |
| 133 | H | 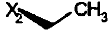 | R | 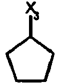 | 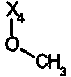 | 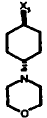 |
| 134 | H | 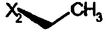 | R | 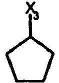 | 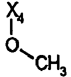 | 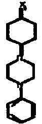 |
| 234 | H | 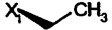 | R | 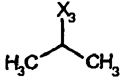 | 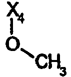 | 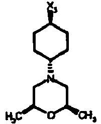 |
| 240 | H | 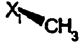 | R | 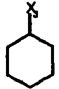 | 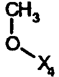 | 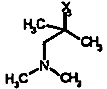 |

wobei die in der Tabelle verwendeten Abkürzungen X₁, X₂, X₃, X₄ und X₅ stehen jeweils für eine Verknüpfung mit einer Position in der unter der Tabelle aufgeführten allgemeinen Formel anstelle der entsprechenden Reste R¹, R², R³, R⁴ und L-R⁵.

Bevorzugt ist ein Verfahren, worin die Reaktion in Gegenwart saurer Katalysatoren, wie anorganische und organische Säuren, durchgeführt wird.

Weiterhin bevorzugt ist ein Verfahren, worin als saure Katalysatoren organische Sulfonsäuren, vorzugsweise Methansulfonsäure, Ethansulfonsäure, Ethan-1,2-disulfonsäure, Benzolsulfonsäure oder p-Toluolsulfonsäure, eingesetzt werden. Besonders bevorzugt ist ein Verfahren, worin die Menge an zugesetztem sauren Katalysator zwischen 0,001 und 2 Equivalenten liegt.

Enthält die Verbindung der Formel (I) ein oder mehrere basiche Gruppen im Rest R⁵ₘ-Lₙ dann müssen zur Protonierung und damit Blockierung der basischen Gruppe bzw. der basischen Gruppen zusätzlich zur katalytischen Menge an Säure ein weiteres Equivalent Säure oder entsprechend mehrere Equivalente Säure zugesetzt werden. In diesem Fall ist ein Verfahren besonders bevorzugt bei dem die Menge an zugesetztem sauren Katalysator über einem Equivalent liegt.

Weiterhin besonders bevorzugt ist ein Verfahren, bei dem die Reaktion in einem Lösungsmittel ausgewählt aus der Gruppe bestehend aus Amiden, wie Dimethylformanid, Dimethylacetamid oder N-Methylpyrrolidinon, Harnstoffen wie 1,3-Dimethyl-3,4,5,6-tetrahydro-2(1H)-pyrimidon (DMPU oder Dimethylpropylharnstoff), Sulfoxiden wie Dimethylsulfoxid oder Sulfonen wie Sulfolan, primären Alkoholen wie Methanol, Ethanol, 1-Propanol, 1-Butanol oder 1-Pentanol, sekundären Alkoholen wie 2-Propanol oder 2-Butanol, isomeren sekundären Alkoholen des Pentans oder Hexans, tertiären Alkoholen des Butans, Pentans oder Hexans, Acetonitril und 2-PropylnitriL Besonders bevorzugt sind sekundäre Alkohole wie 2-Propanol, 2-Butanol oder 2-Methyl-4-pentanol.

Insbesondere bevorzugt ist ein Verfahren, worin die Reaktionstemperatur zwischen 18 °C bis 180 °C, vorzugsweise zwischen 100°C bis 150 °C beträgt.

Die Umsetzungen können auch unter Druck in niedrigsiedenden Lösungsmitteln oder mit Hilfe von Mikrowellen als Energiequelle durchgeführt werden.

Die Aufarbeitung der Umsetzungen erfolgt nach gängigen Methoden z:B. über extraktive Reinigungsschritte oder Fäll-und Kristallisations-Prozesse.

Die erfindungsgemäßen Verbindungen können in Form der einzelnen optischen Isomeren, Mischungen der einzelnen Enantiomeren, Diastereomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen

Als Alkylgruppen sowie Alkylgruppen, welche Bestandteil anderer Reste sind, werden verzweigte und unverzweigte Alkylgruppen mit 1 bis 12 Kohlenstoffatomen bevorzugt 1 - 6, besonders bevorzugt 1-4 Kohlenstoffatomen bezeichnet, beispielsweise werden genannt: Methyl, Ethyl, Propyl, Butyl, Pentyl, Hexyl, Heptyl, Octyl, Nonyl, Decyl und Dodecyl. Sofern nicht anders genannt, sind von den vorstehend genannten Bezeichnungen Propyl, Butyl, Pentyl, Hexyl, Heptyl, Octyl, Nonyl, Decyl und Dodecyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl.

In den vorstehend genannten Alkylgruppen können gegebenenfalls ein oder mehrere Wasserstoffatome durch andere Reste ersetzt sein. Beispielsweise können diese Alkylgruppen durch Fluor substituiert sein. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 1 bis 5 Kohlenstoffatomen, beispielsweise Methylen, Etyhlen, Propylen-, Isopropylen-, n-Butylen, iso-Butyl, sec. Butyl und tert.-Butyl etc. Brücken bezeichnet. Besonders bevorzugt sind Methylen, Etyhlen, Propylen- und Butylen-Brücken. In den genannten Alkylbrücken können gegebenenfalls 1 bis 2 C-Atome durch ein oder mehrere Heteroatome ausgewählt aus der Gruppe Sauerstoff, Stickstoff oder Schwefel ersetzt sein

Als Alkenylgruppen (auch soweit sie Bestandteil anderer Reste sind) werden verzweigte und unverzweigte Alkylengruppen mit 2 bis 10 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 3-Butenyl, 1-Methyl-1-Propenyl, 1-Methyl-2-Propenyl, 2-Methyl-1-Propenyl, 2-Methyl-2-Propenyl und 1-Ethyl-1-Ethenyl.

In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor substituiert sein. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkylgruppen Fluor substituiert sein. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂, CN, OMe, -OCHF₂, -OCF₃, -NH₂, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -O-Methyl oder -O-Ethyl, -CONH₂.

Als Heteroarylreste, in denen bis zu zwei C-Atome durch ein oder zwei Stickstoffatome ersetzt sind werden beispielsweise, Pyrrol, Pyrazol, Imidazol, Triazol, Pyridin, Pyrimidin, genannt, wobei jeder der vorstehend genannten Heteroarylringe gegebenenfalls ferner an einen Benzolring anneliert sein kann, vorzugsweise Benzimidazol, und wobei diese Heterocyclen soweit nicht anders beschrieben, beispielsweise einen oder mehrere der nachfolgend genannten Substituenten tragen können: F, Cl, Br, OH, OMe, Methyl, Ethyl, CN, CONH₂, NH₂, gegebenenfalls substituiertes Phenyl, gegebenenfalls substituiertes Heteroaryl, vorzugsweise gegebenenfalls substituiertes Pyridyl.

Als Cycloalkylreste werden Cycloalkylreste mit 3 - 12 Kohlenstoffatomen beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet, wobei jeder der vorstehend genannten Cycloalkylreste gegebenenfalls ferner einen oder mehrere Substituenten tragen kann, beispielsweise: OH, NO₂, CN, OMe, -OCHF₂, - OCF₃, -NH₂ oder Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder-0-Ethyl, -COOH, -COO-C₁-C₄-Alkyl, vorzugsweise -COO-Methyl oder -COO-Ethyl oder -CONH₂. Besonders bevorzugte Substituenten der Cycloalkylreste sind =O, OH, NH₂, Methyl oder F.

Als Cycloalkenylreste werden Cycloalkylreste mit 3 - 12 Kohlenstoffatomen, die mindestens eine Doppelbindung aufweisen, beispielsweise Cyclopropenyl, Cyclobutenyl, Cyclopentenyl, Cyclohexenyl oder Cycloheptenyl, vorzugsweise Cyclopropenyl, Cyclopententyl oder Cyclohexenyl bezeichnet, wobei jeder der vorstehend genannten Cycloalkenylreste gegebenenfalls ferner einen oder mehrere Substituenten tragen kann.

"=O" bedeutet ein über eine Doppelbindung verknüpftes Sauerstoffatom.

Als Heterocycloalkylreste werden, soweit in den Definitionen nicht anders beschrieben, 3 bis 12 gliedrige, vorzugsweise 5- ,6- oder 7-gliedrige, gesättigte oder ungesättigte Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Morpholin, Pyrrolidin, Piperidin oder Piperazin, genannt, wobei der Heterocyclus gegebenenfalls Substituenten tragen kann , beispielsweise C₁-C₄-Alkyl, vorzugsweise Methyl, Ethyl oder Propyl.

Als Polycycloalkylreste werden gegebenenfalls substituierte, Bi-, Tri,-Tetra- oder pentacyclische Cycloalkylreste, beispielsweise Pinan, 2.2.2-Octan, 2.2.1-Heptan oder Adamantan, bezeichnet. Als Polycycloalkenylreste werden gegebenenfalls verbrückte oder/und substituierte, 8-gliedrige Bi-, Tri, Ttetra- oder pentacyclische Cycloalkenyllreste, vorzugsweise Bicycloalkenyl- oder Tricycloalkenylreste, sofern sie mindestens eine Doppelbindung aufweisen, beispielsweise Norbornen, bezeichnet. Als Spiroalkylreste werden gegebenenfalls substituierte spirocyclische C₅-C₁₂ Alkylreste bezeichnet.

Als Halogen wird im allgemeinen Fluor, Chlor, Brom oder Jod bezeichnet, vorzugsweise Fluor, Chlor oder Brom, besonders bevorzugt Chlor.

Als Abgangsgruppe A wird eine Abgangsgruppe wie beispielsweise Fluor, Chlor, Brom, Iod, Methansulfonyl, Ethansulfonyl, Trifluormethansulfonyl oder p-Toluolsulfonyl, vorzugsweise Chlor bezeichnet.

Die Herstellung der Zwischenverbindungen (II) und (III) kann nach Literatur bekannten Methoden, beispielsweise analog den in WO 03/020722 beschriebenen Synthesen erfolgen

Die Herstellung der Zwischenverbindung (III) kann für den Fall von trans-Diamino substituierten Cyclohexanen auch nach den nachfolgend beschriebenen Verfahren hergestellt werden.

Analog zu vorstehend beschriebener Vorgehensweise werden u.a. die in Tabelle 1 aufgeführten Verbindungen der Formel (I) erhalten.

Die in Tabelle 1 verwendeten Abkürzungen X₁, X₂, X₃, X₄ und X₅ stehen jeweils für eine Verknüpfung mit einer Position in der unter Tabelle 1 aufgeführten allgemeinen Formel anstelle der entsprechenden Reste R¹, R², R³, R⁴ und Lₙ-R⁵ₘ.

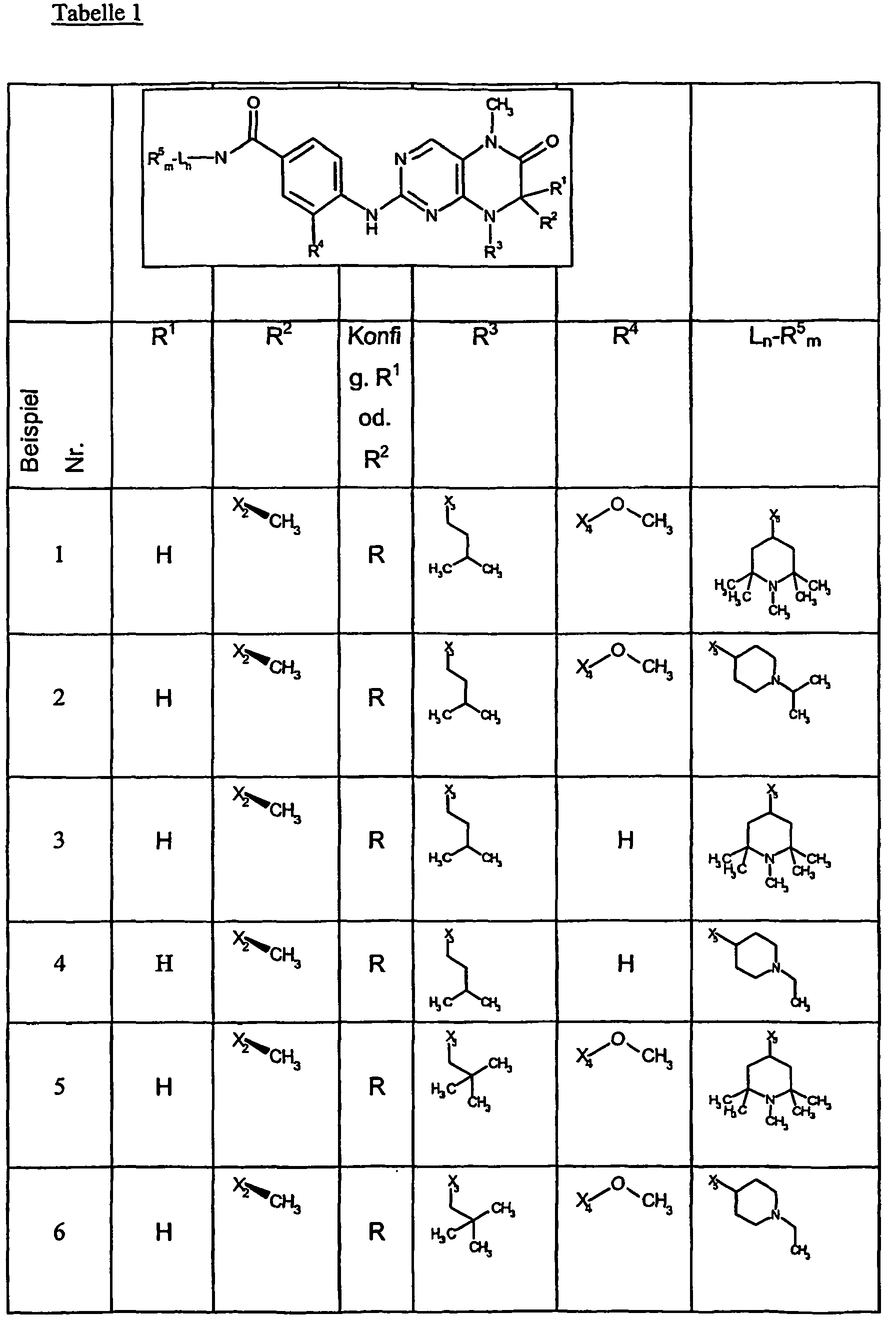

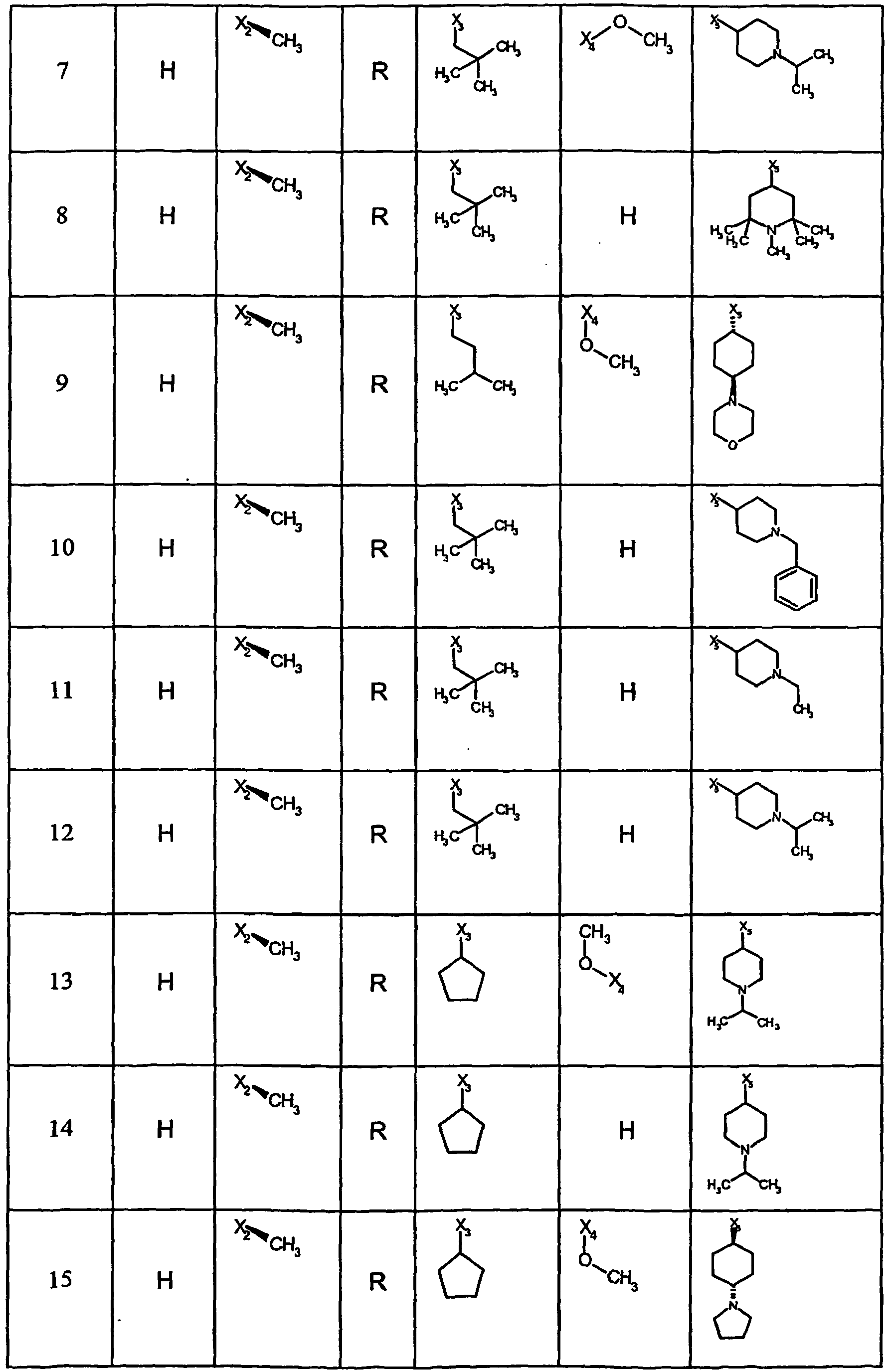

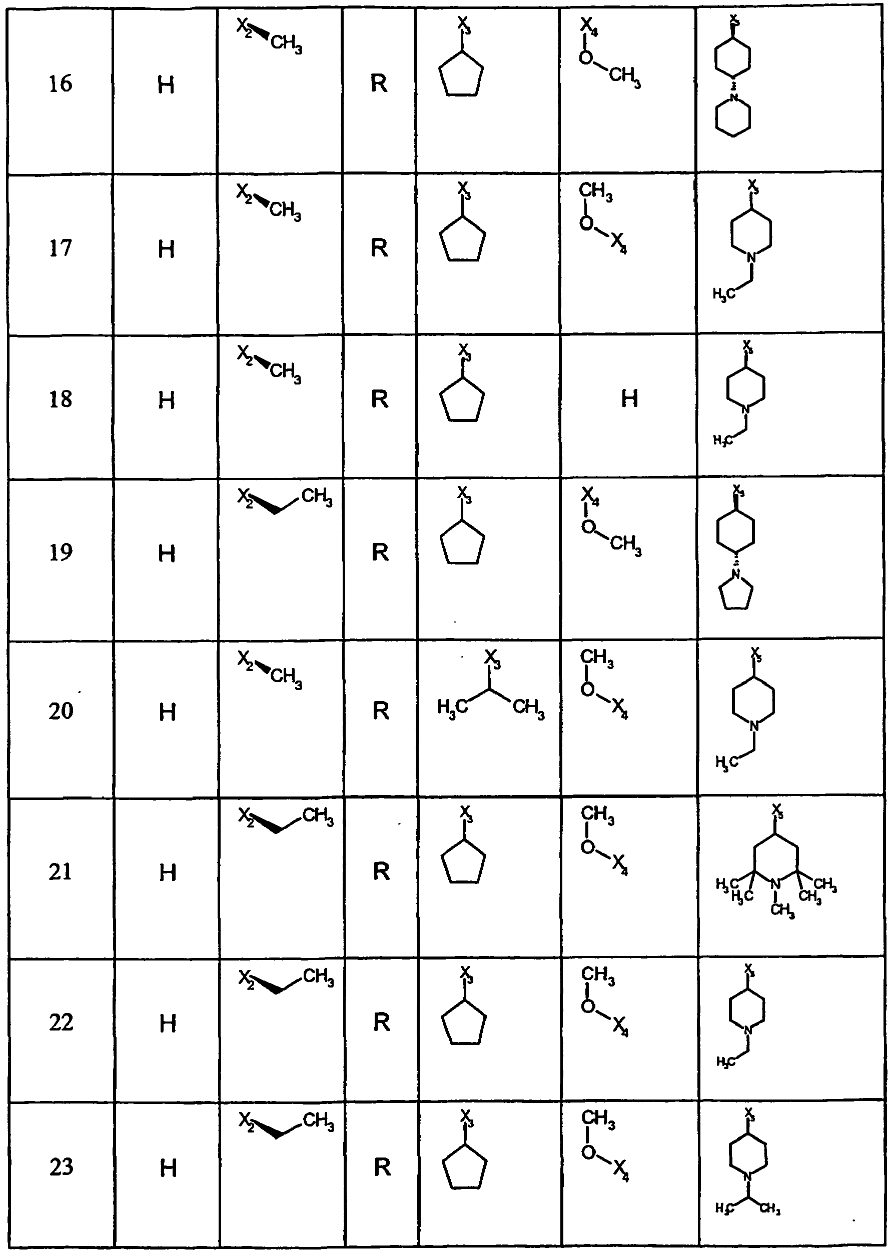

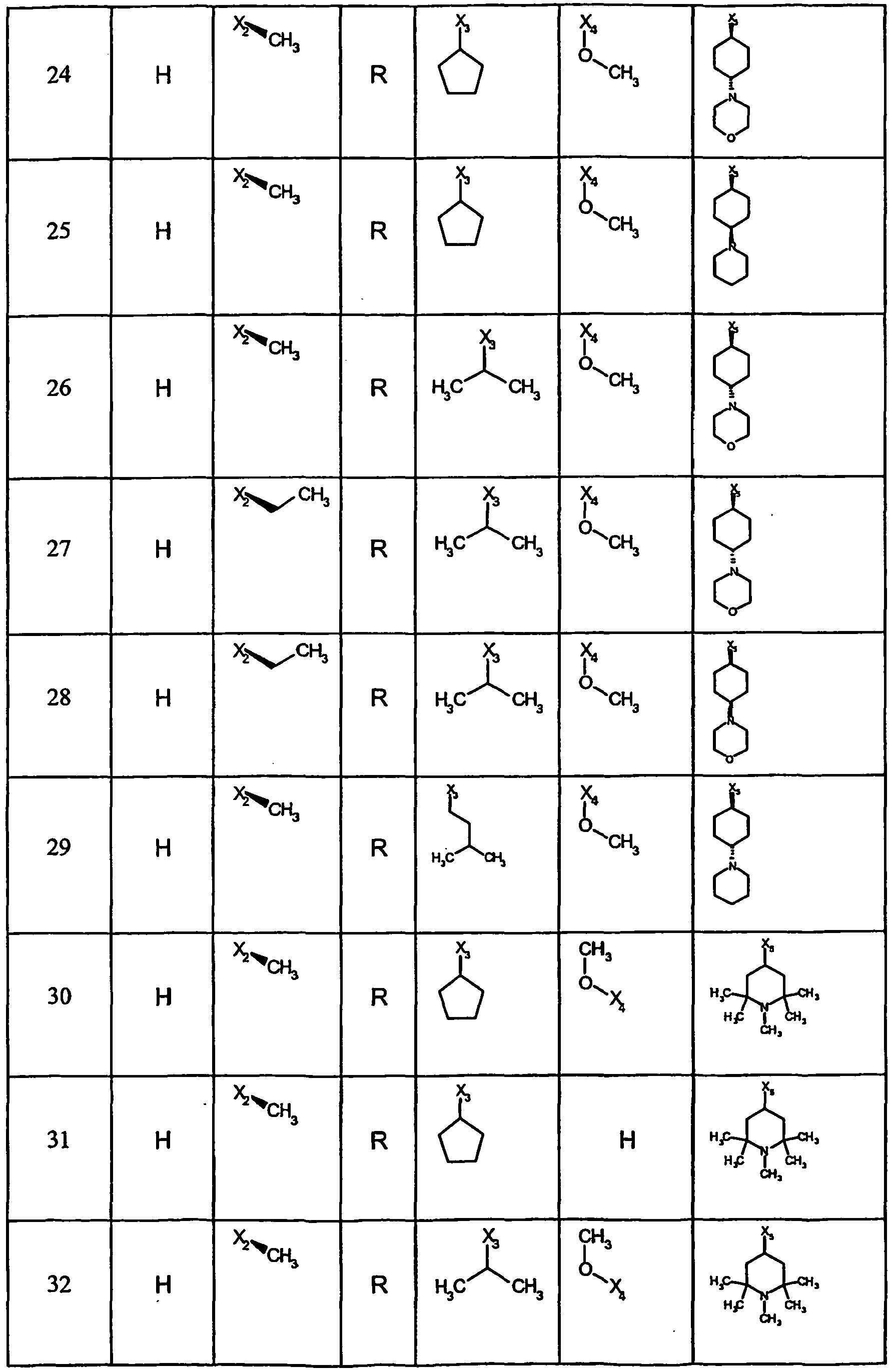

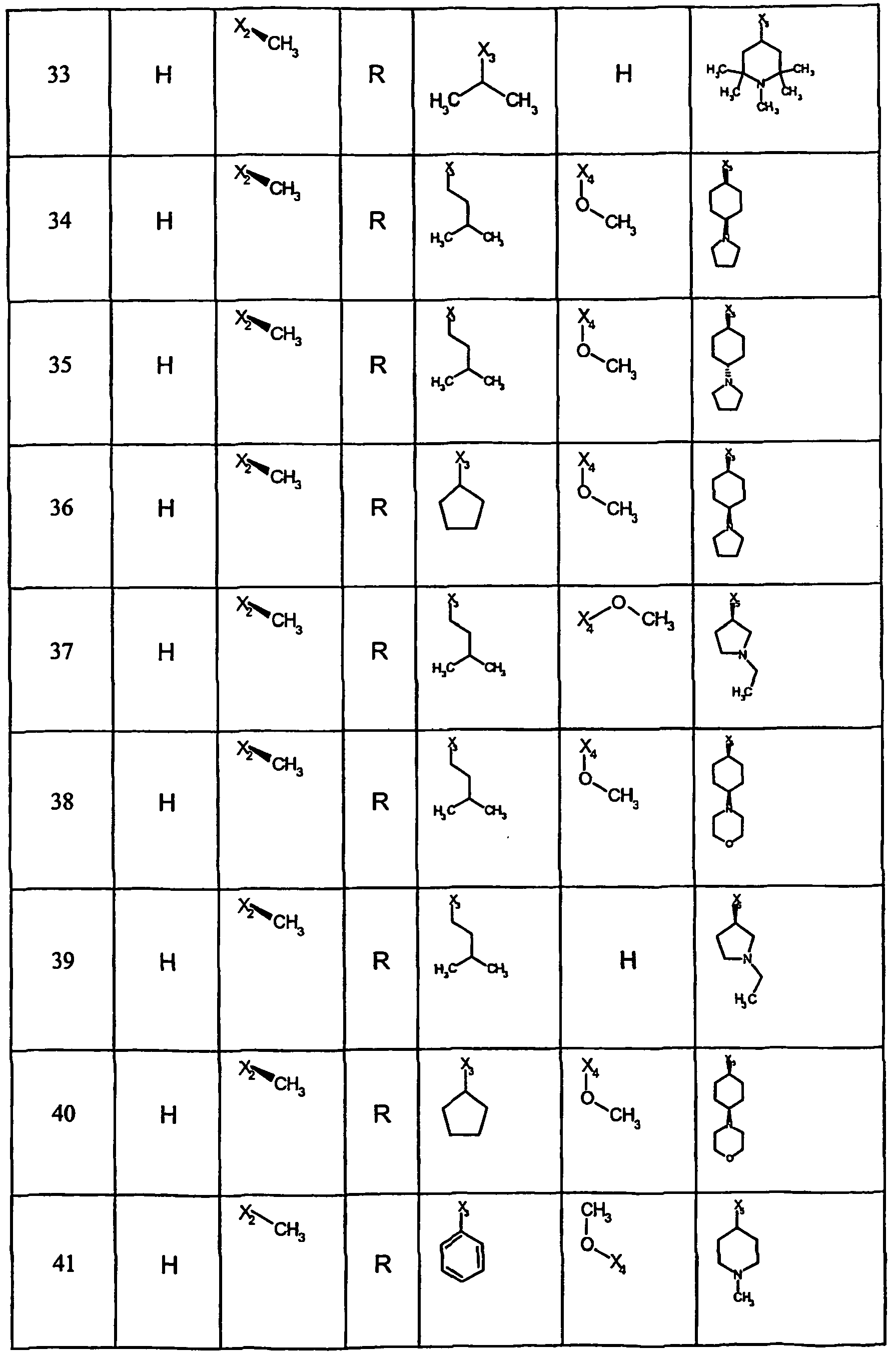

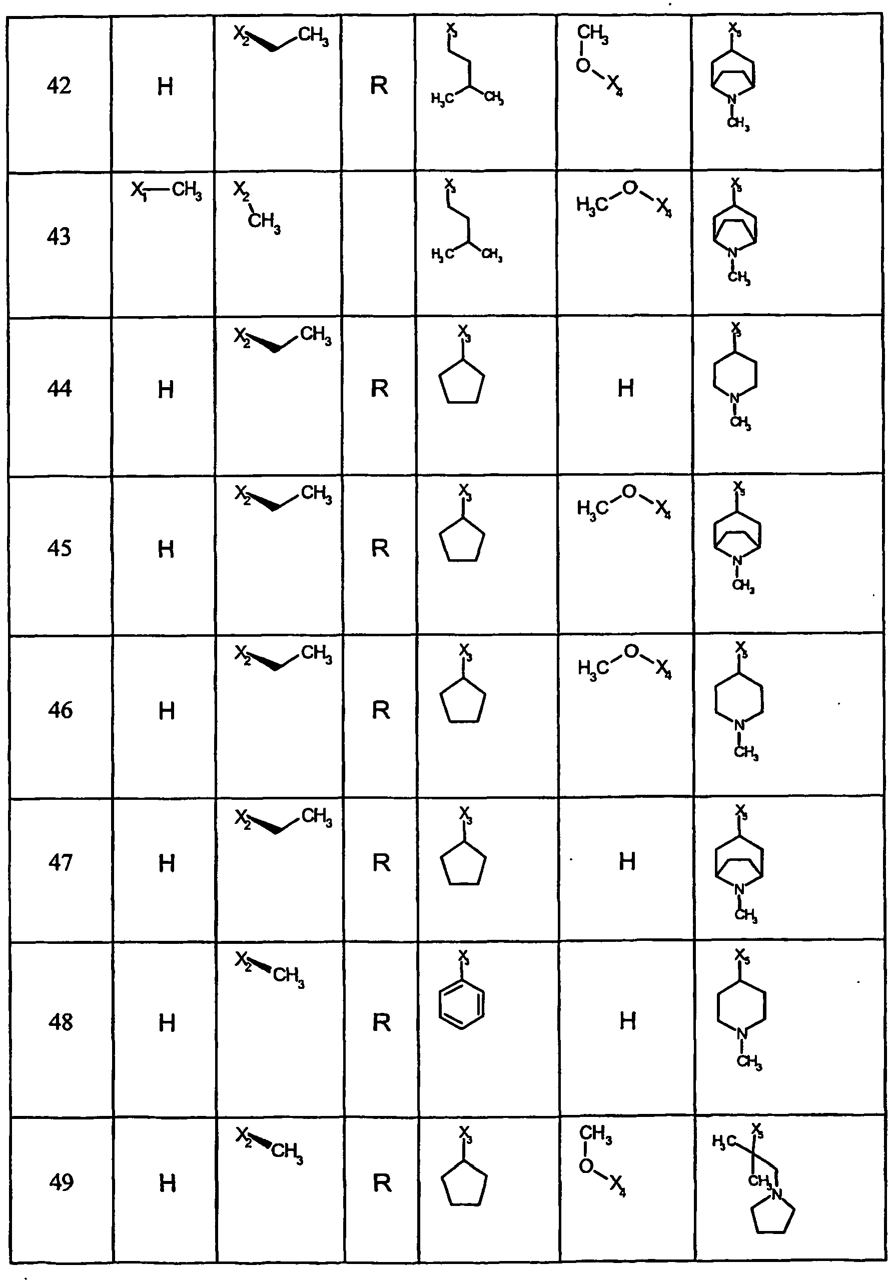

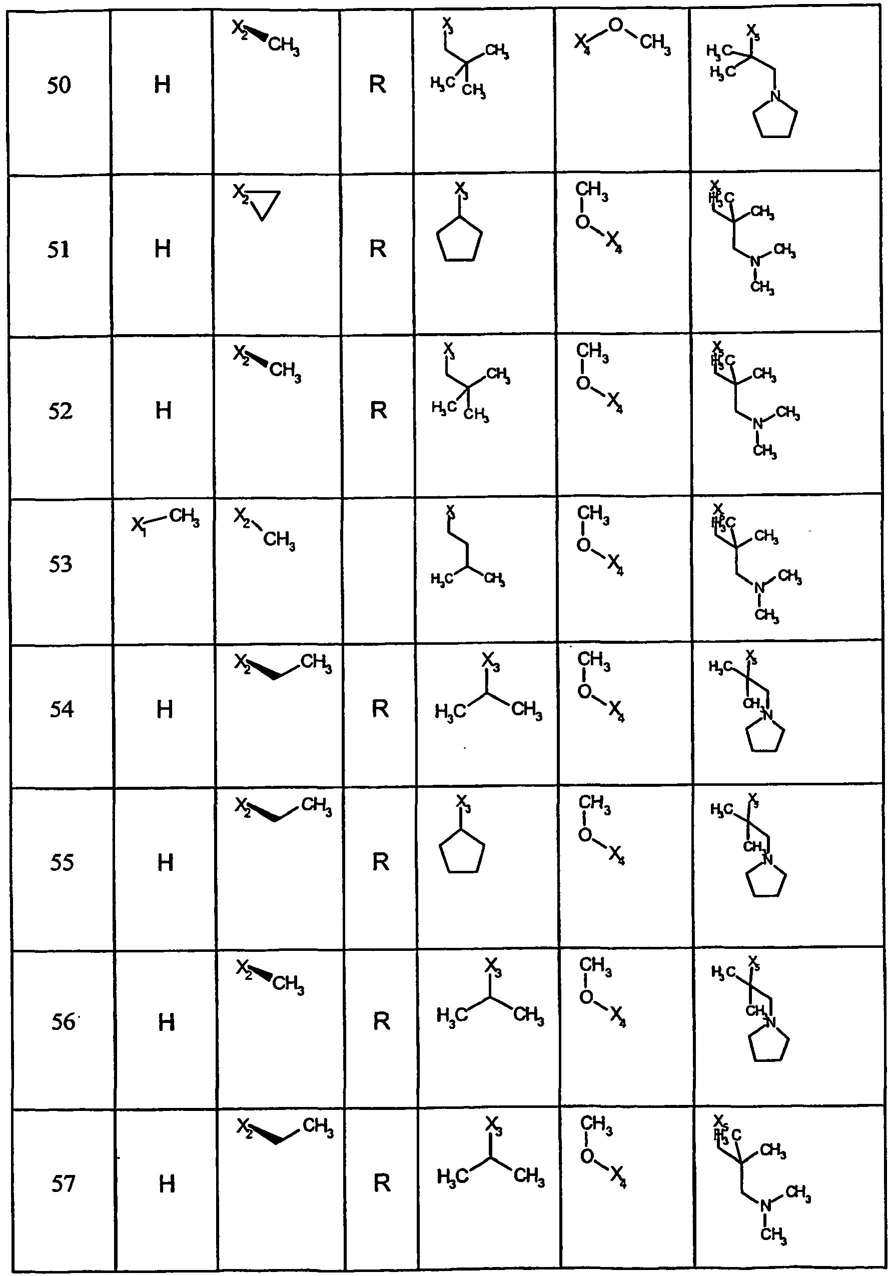

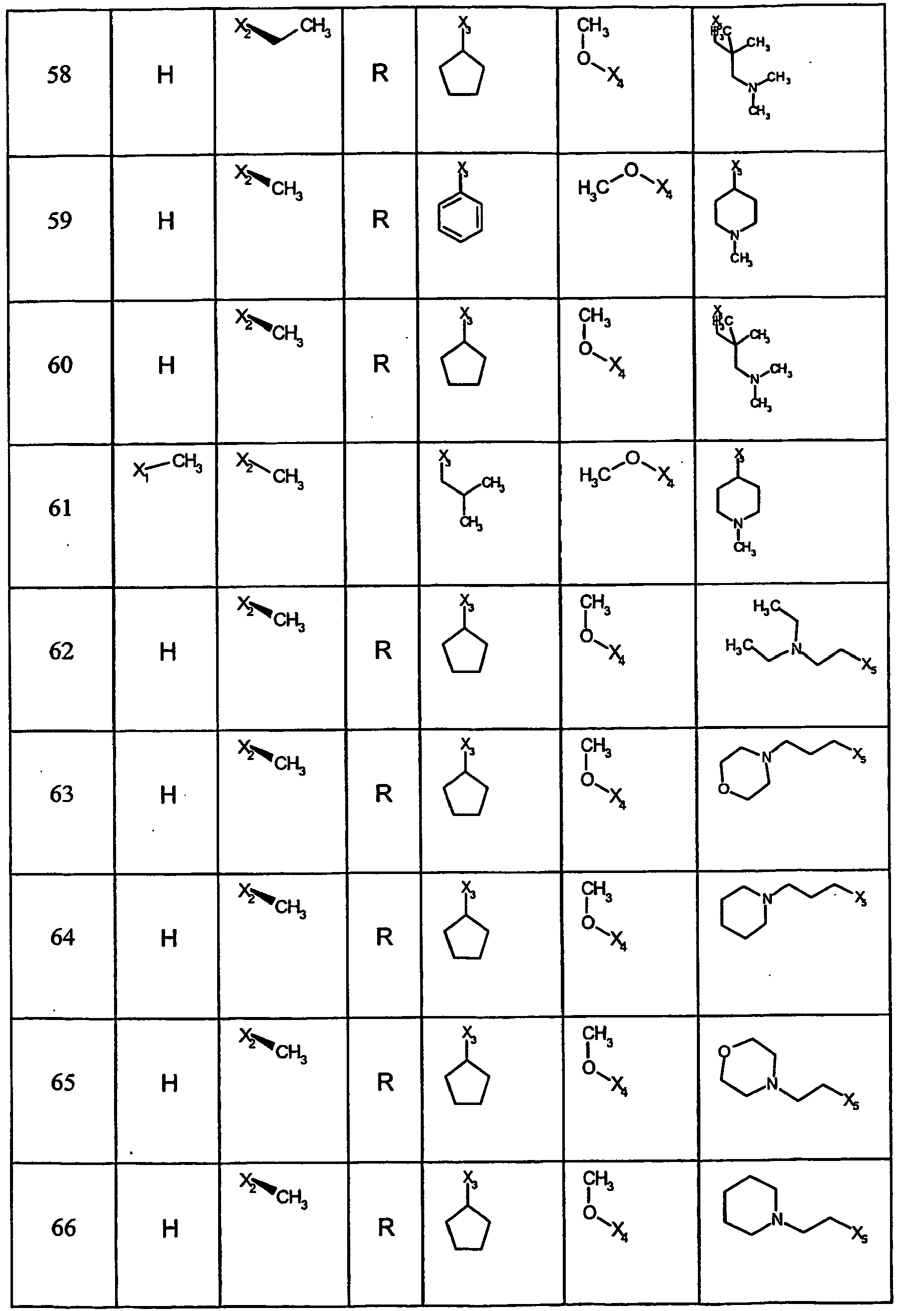

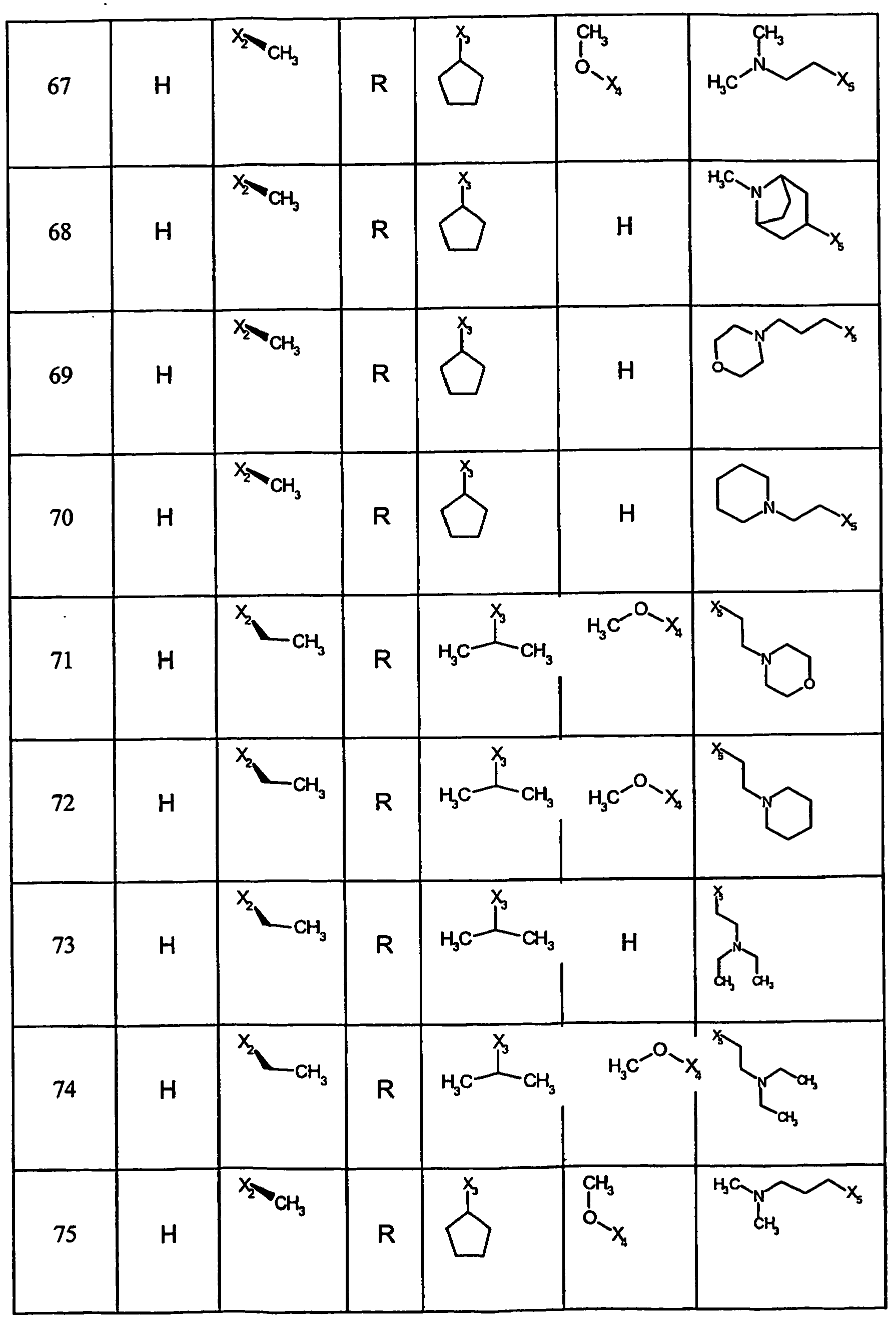

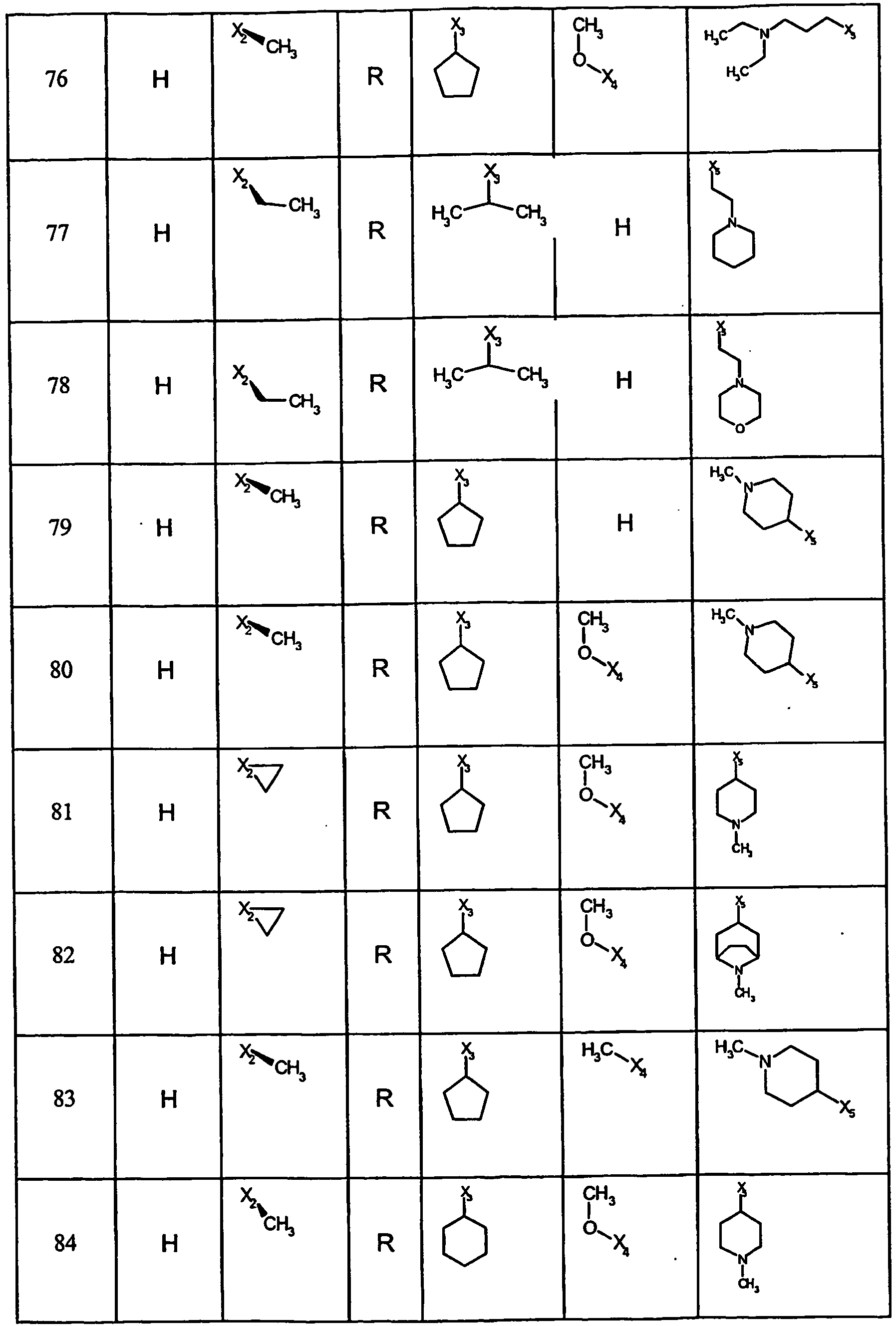

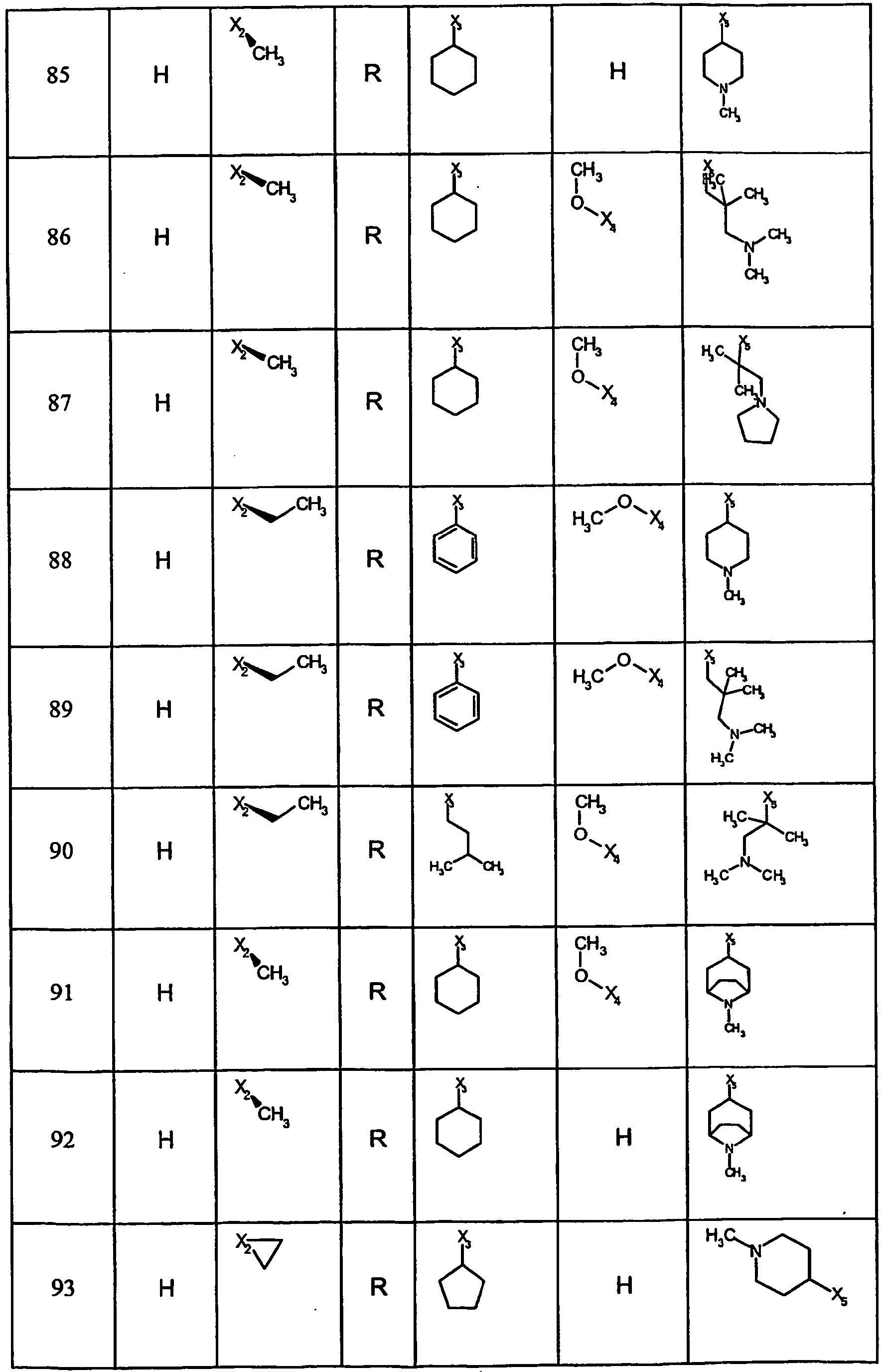

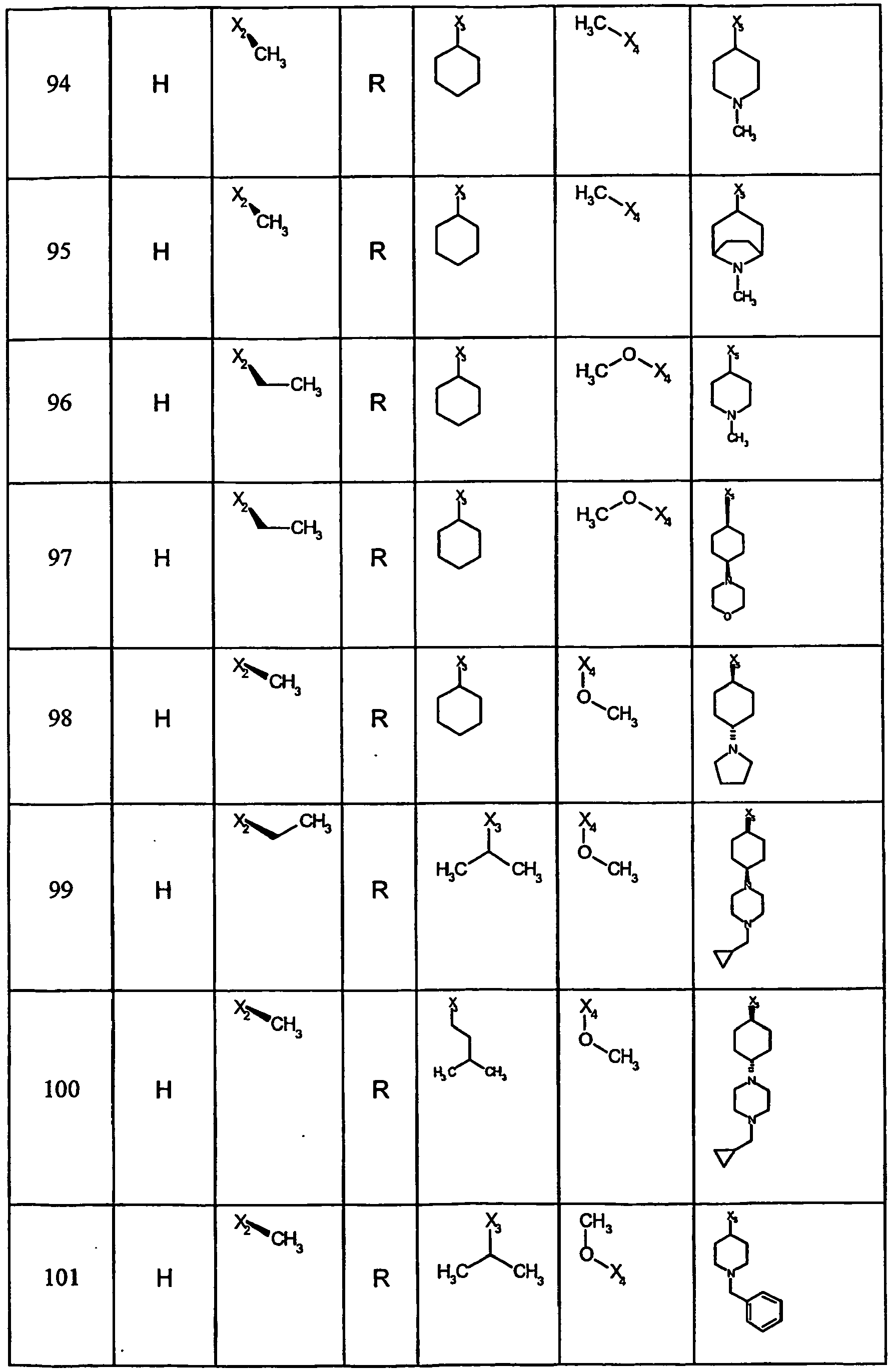

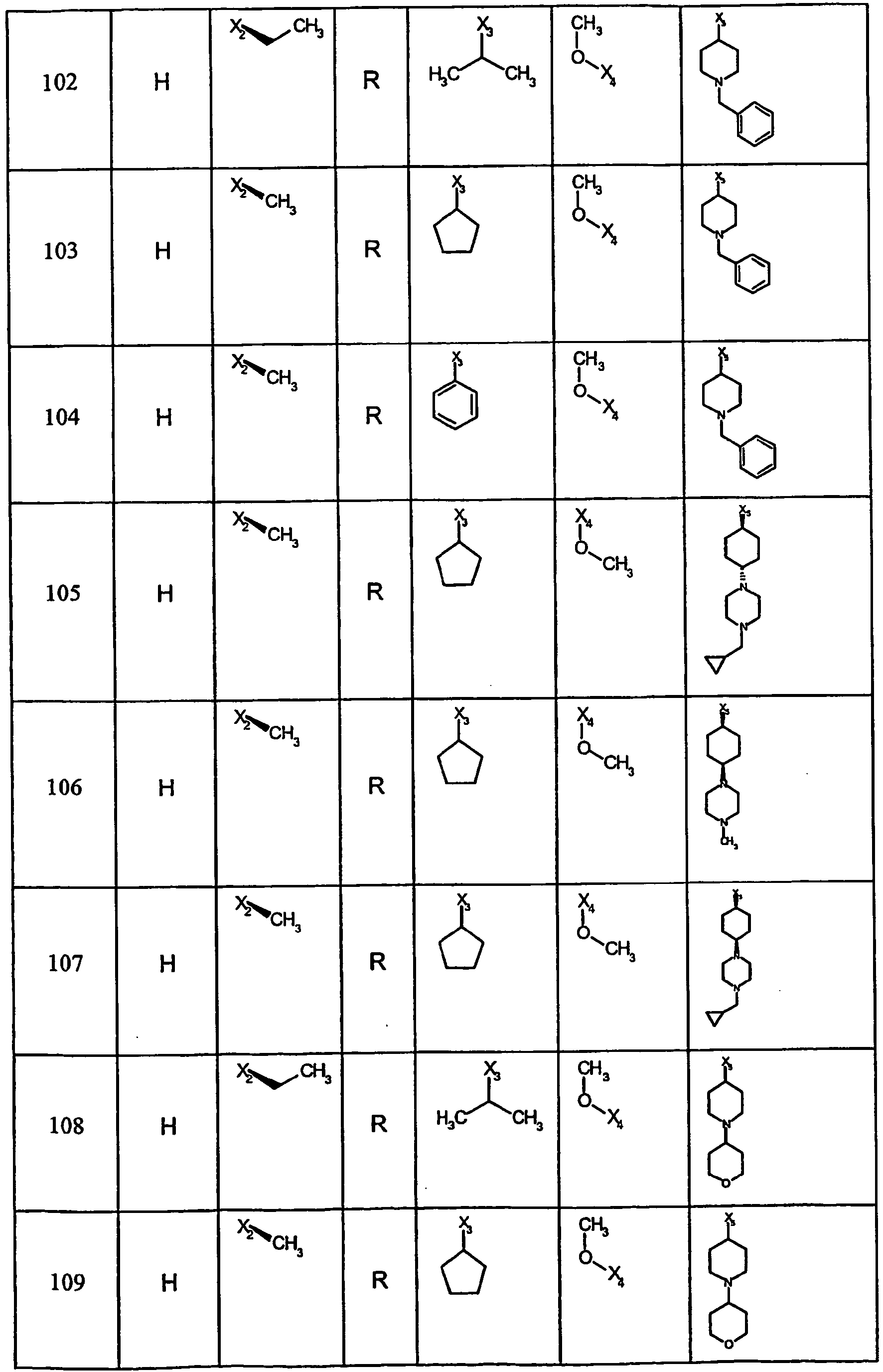

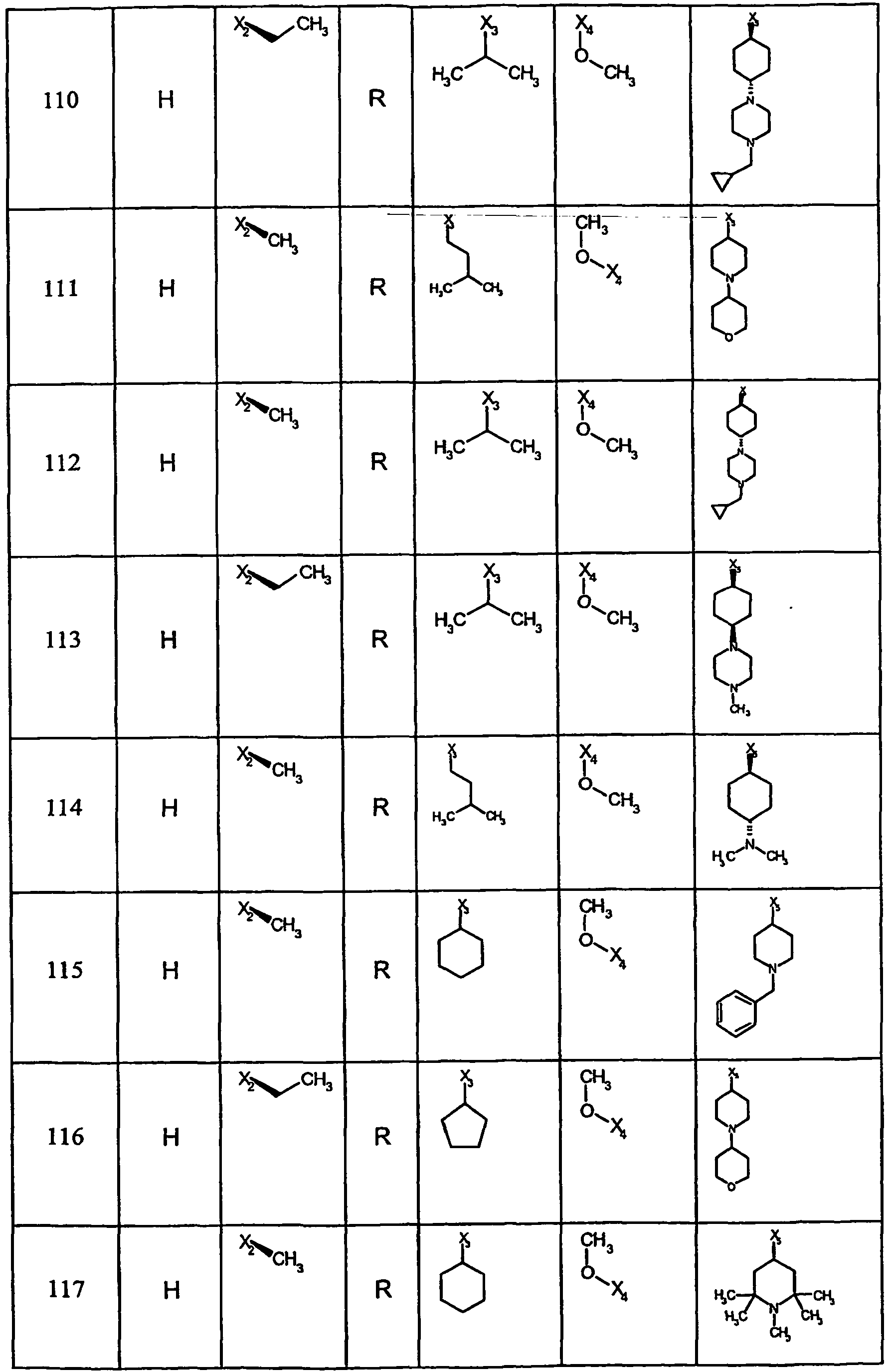

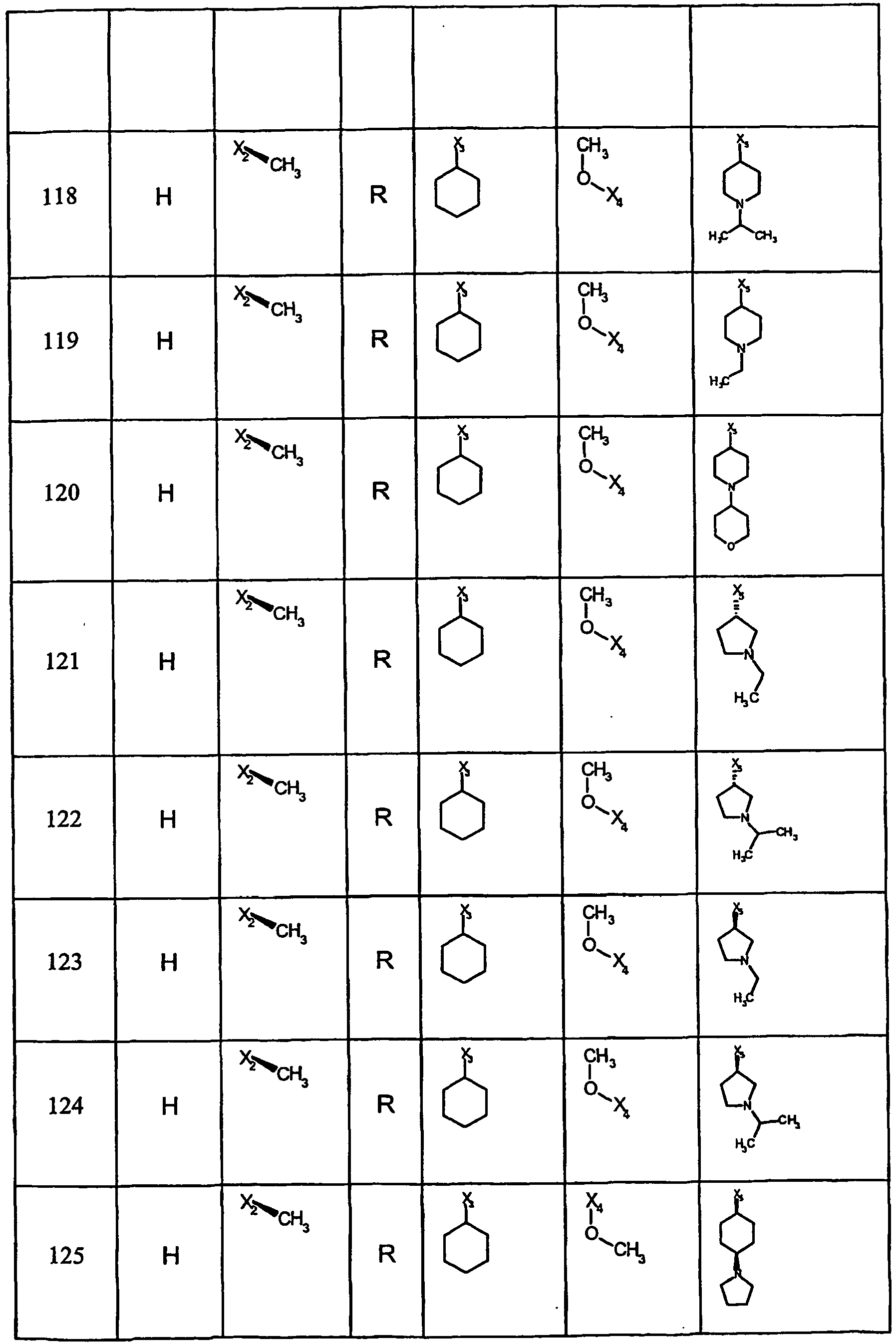

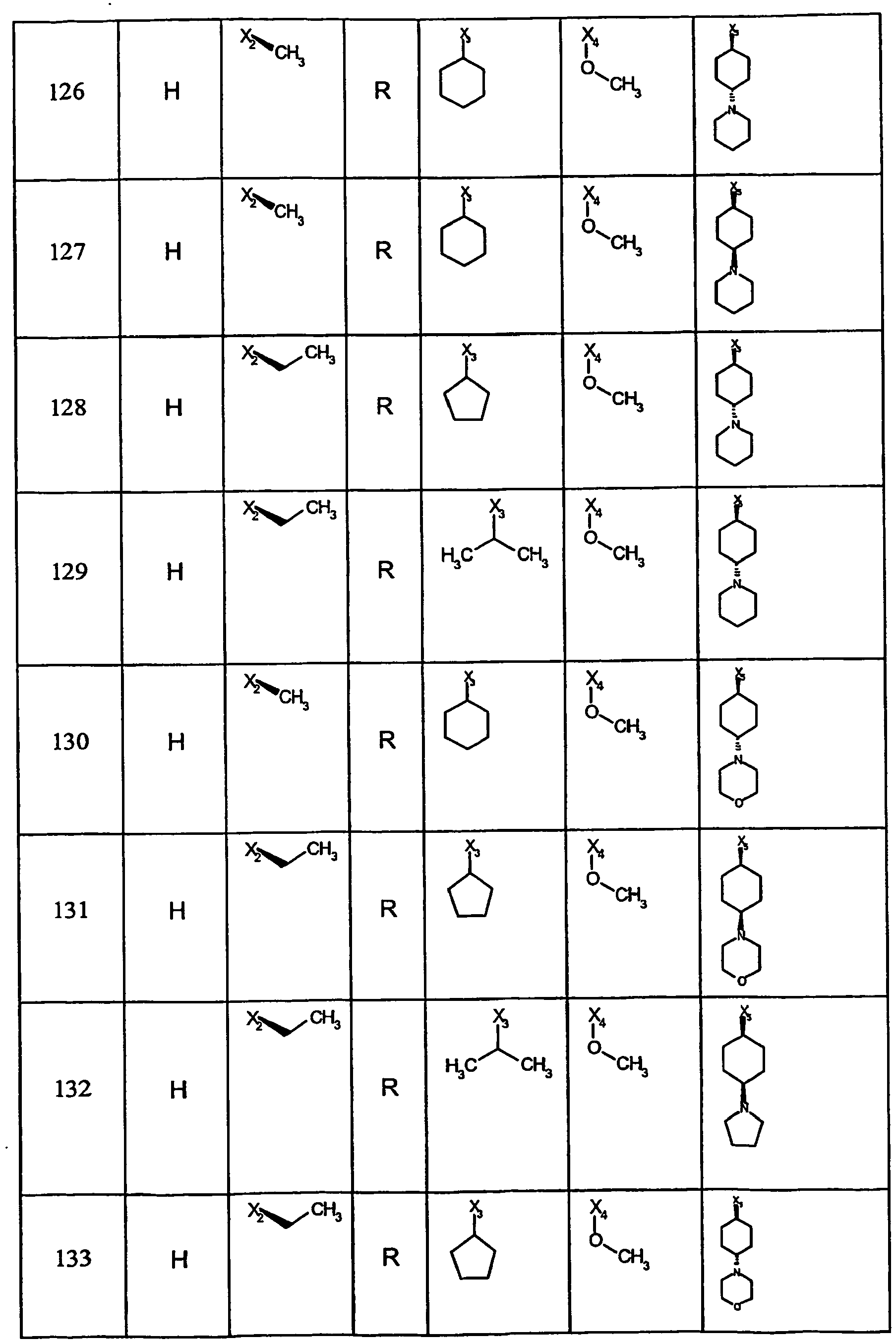

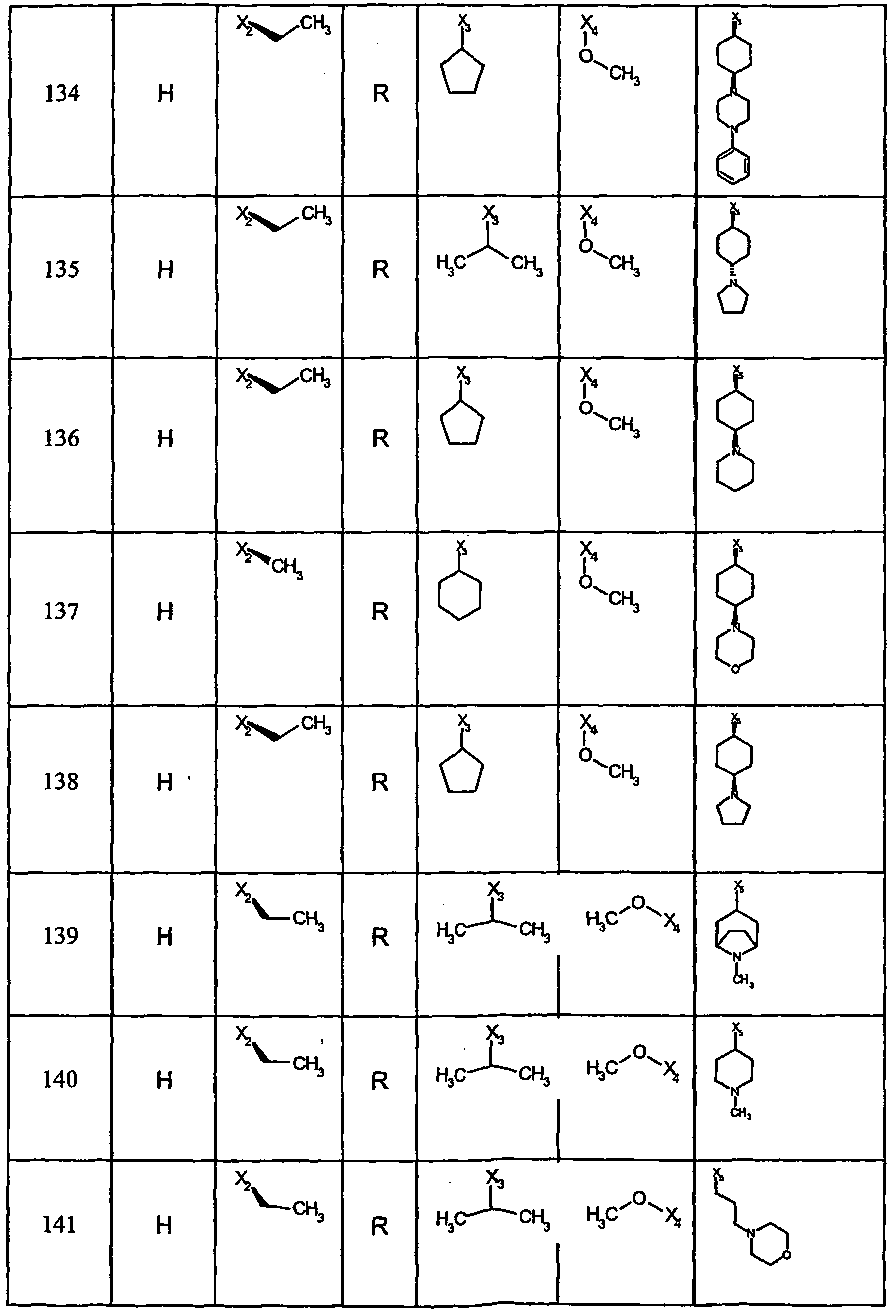

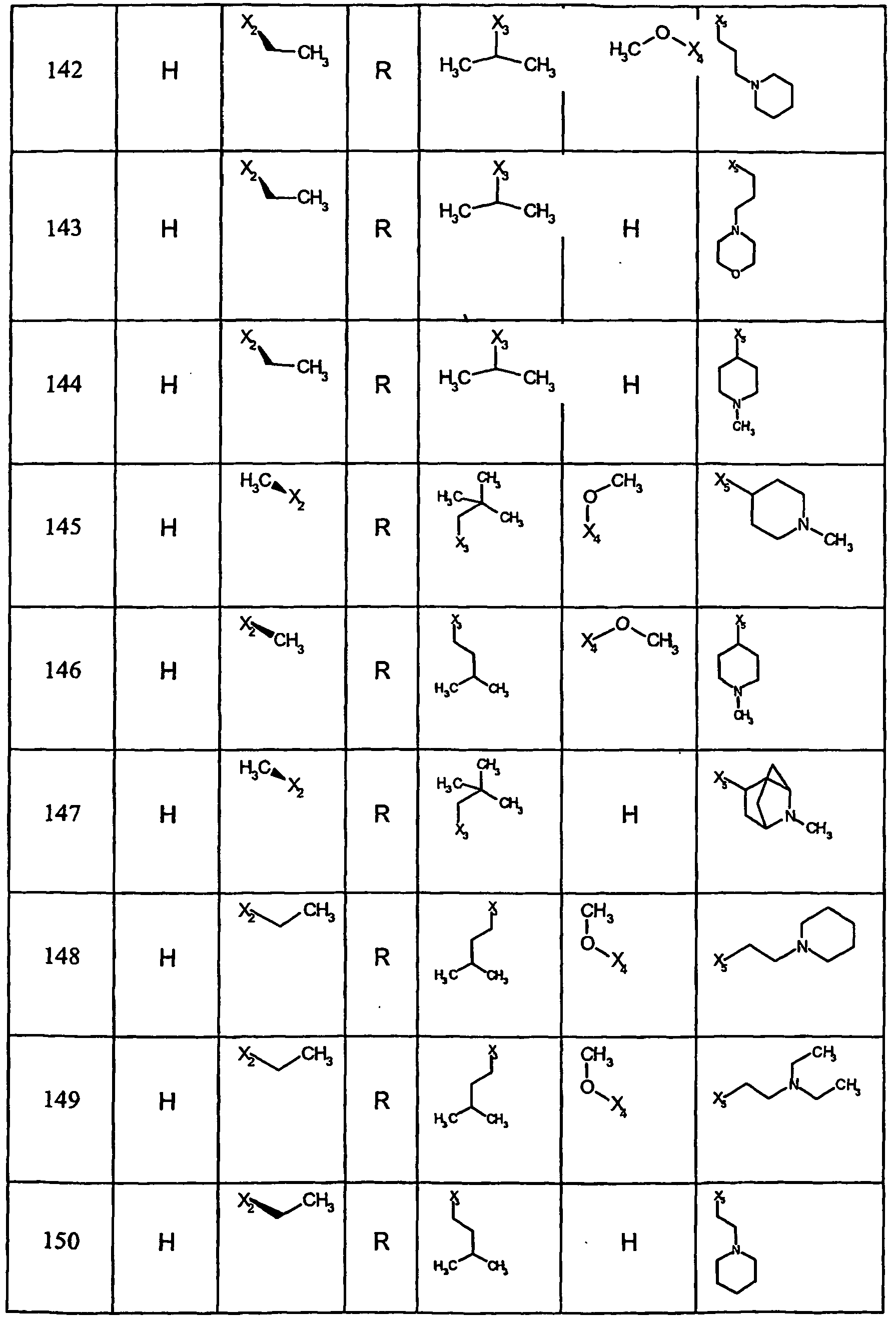

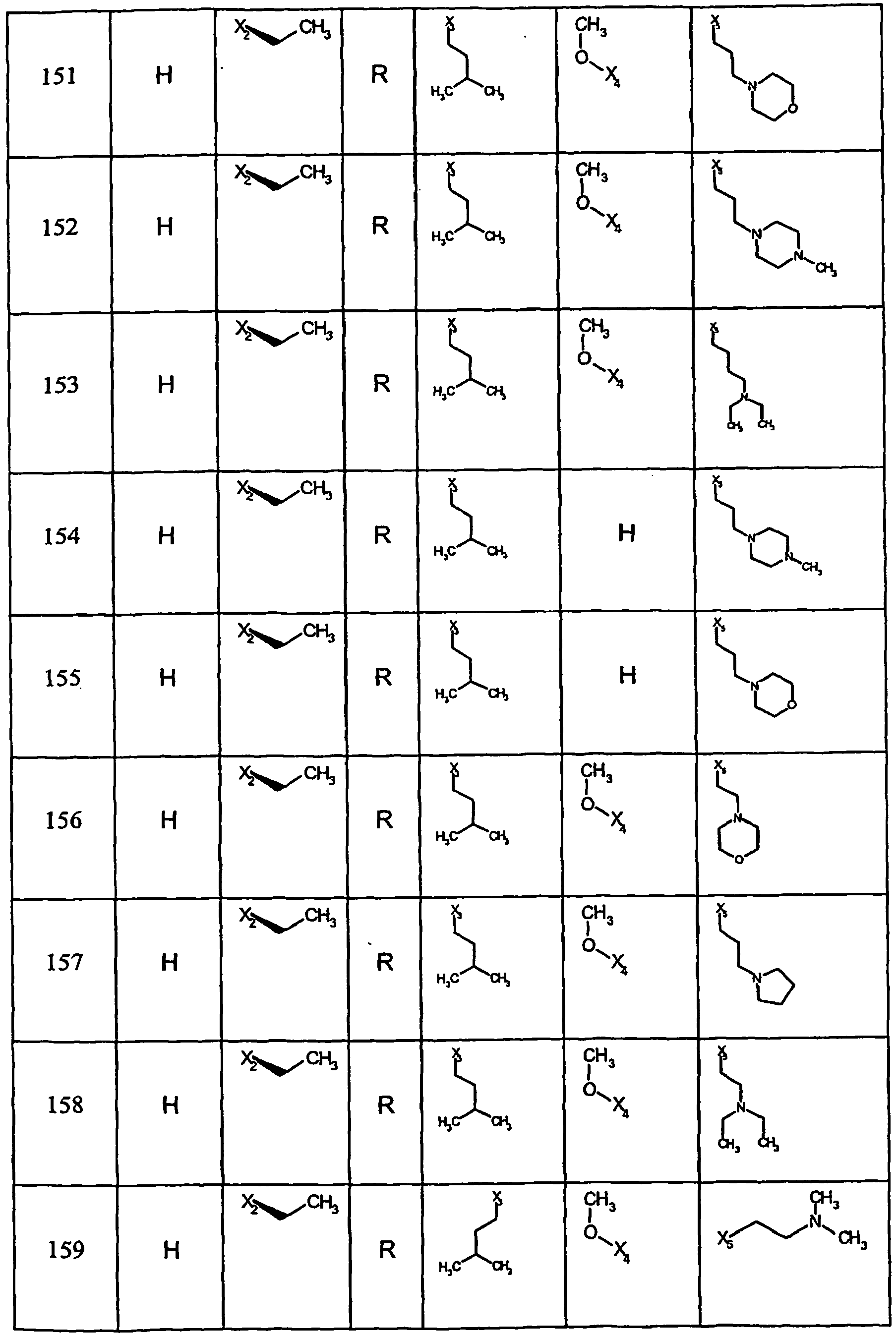

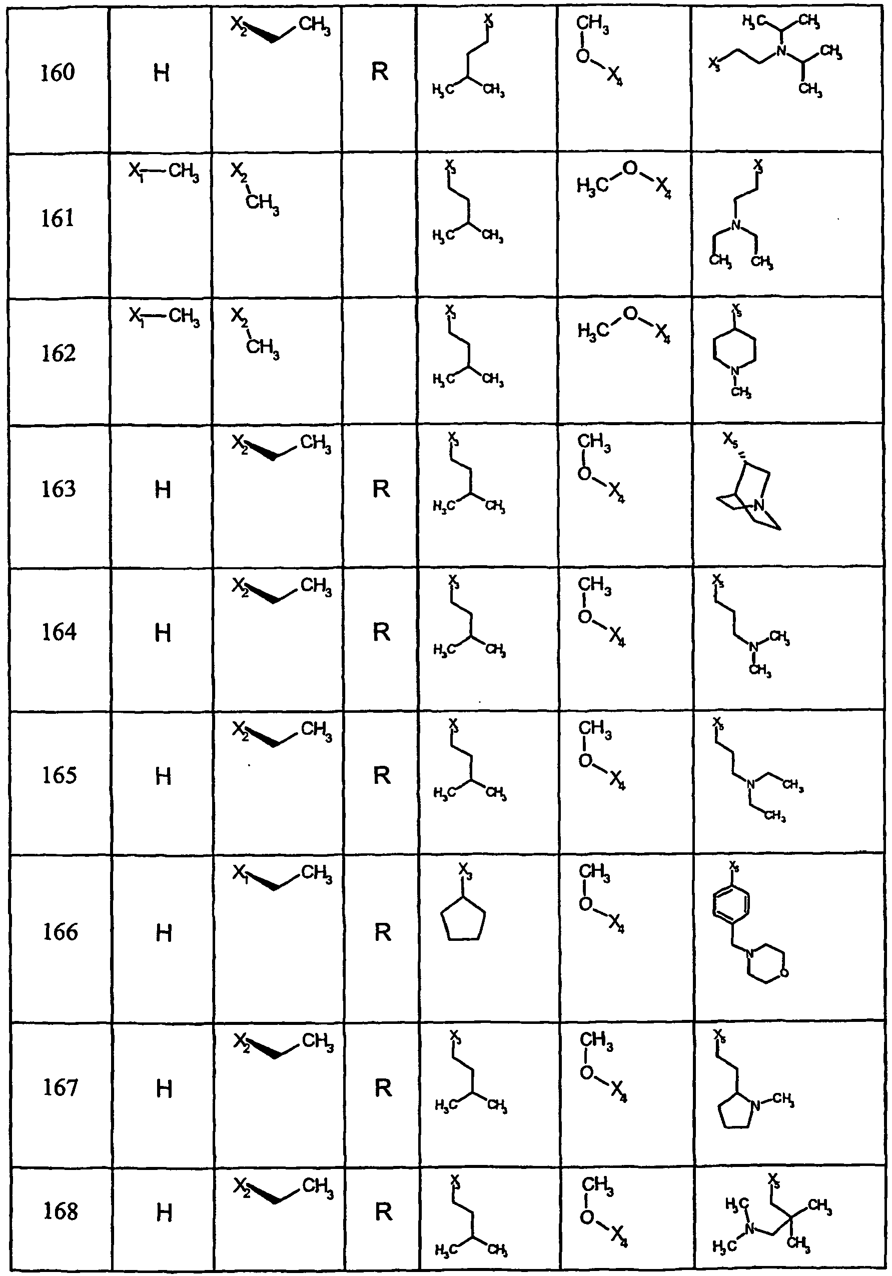

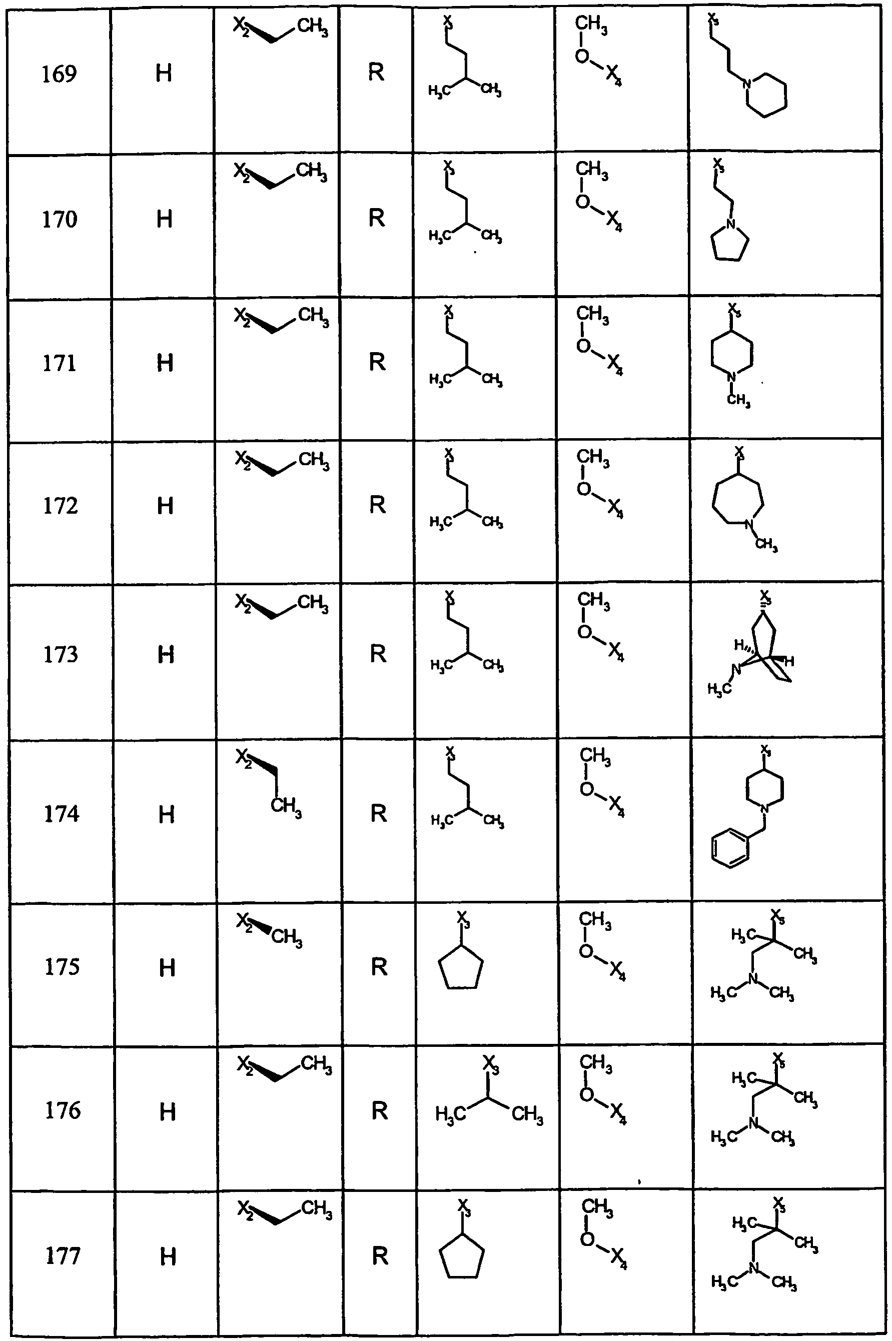

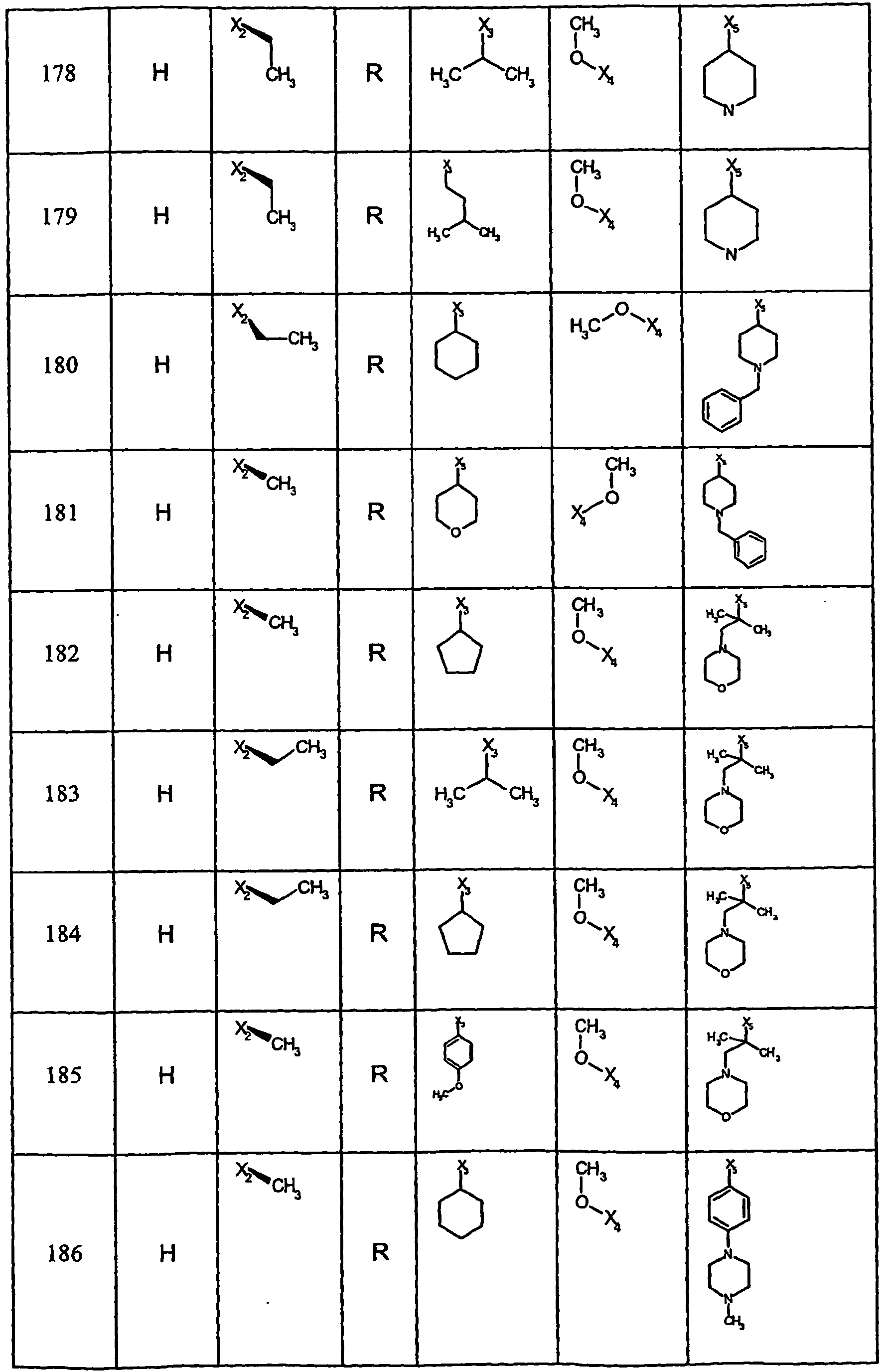

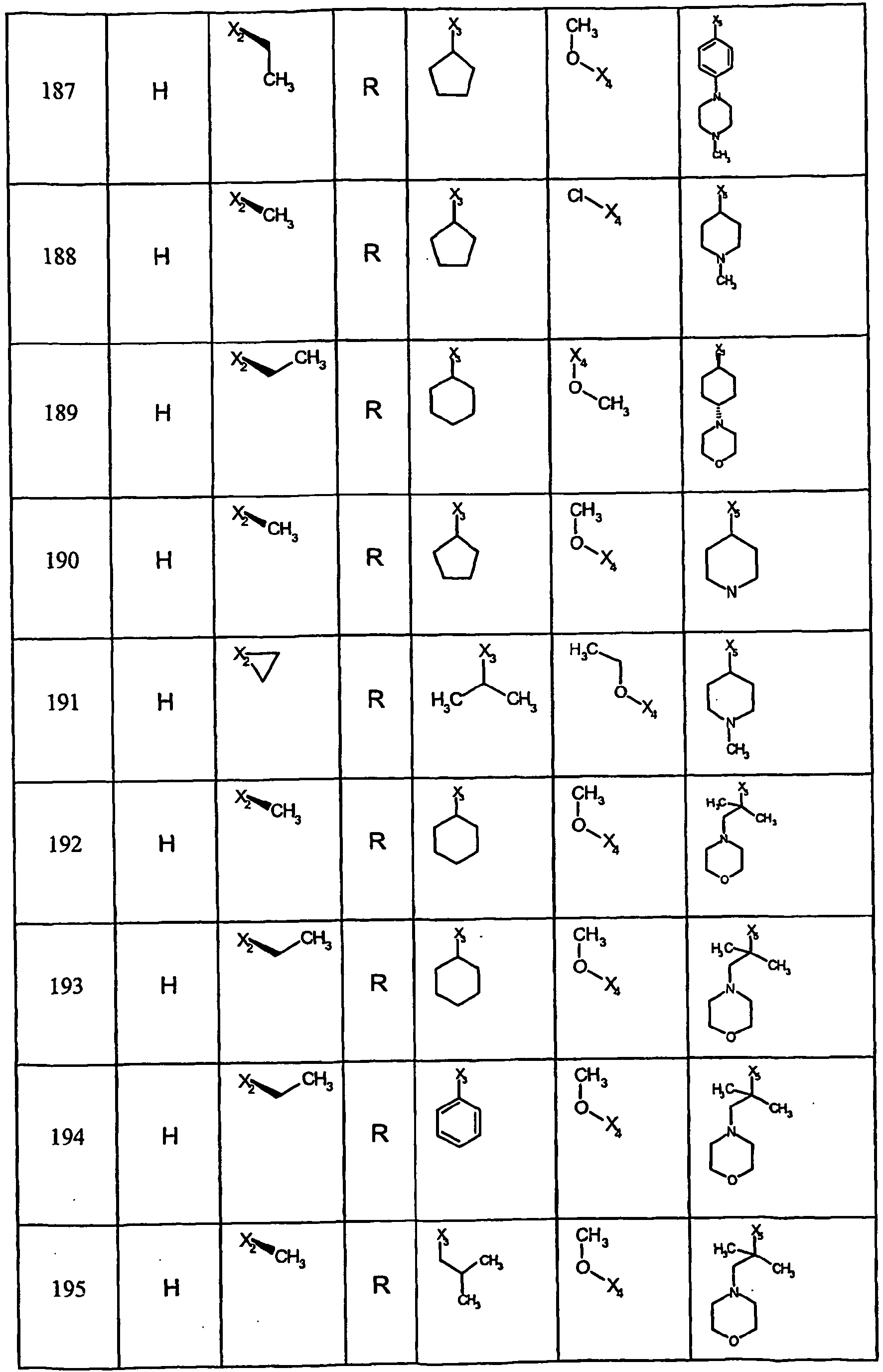

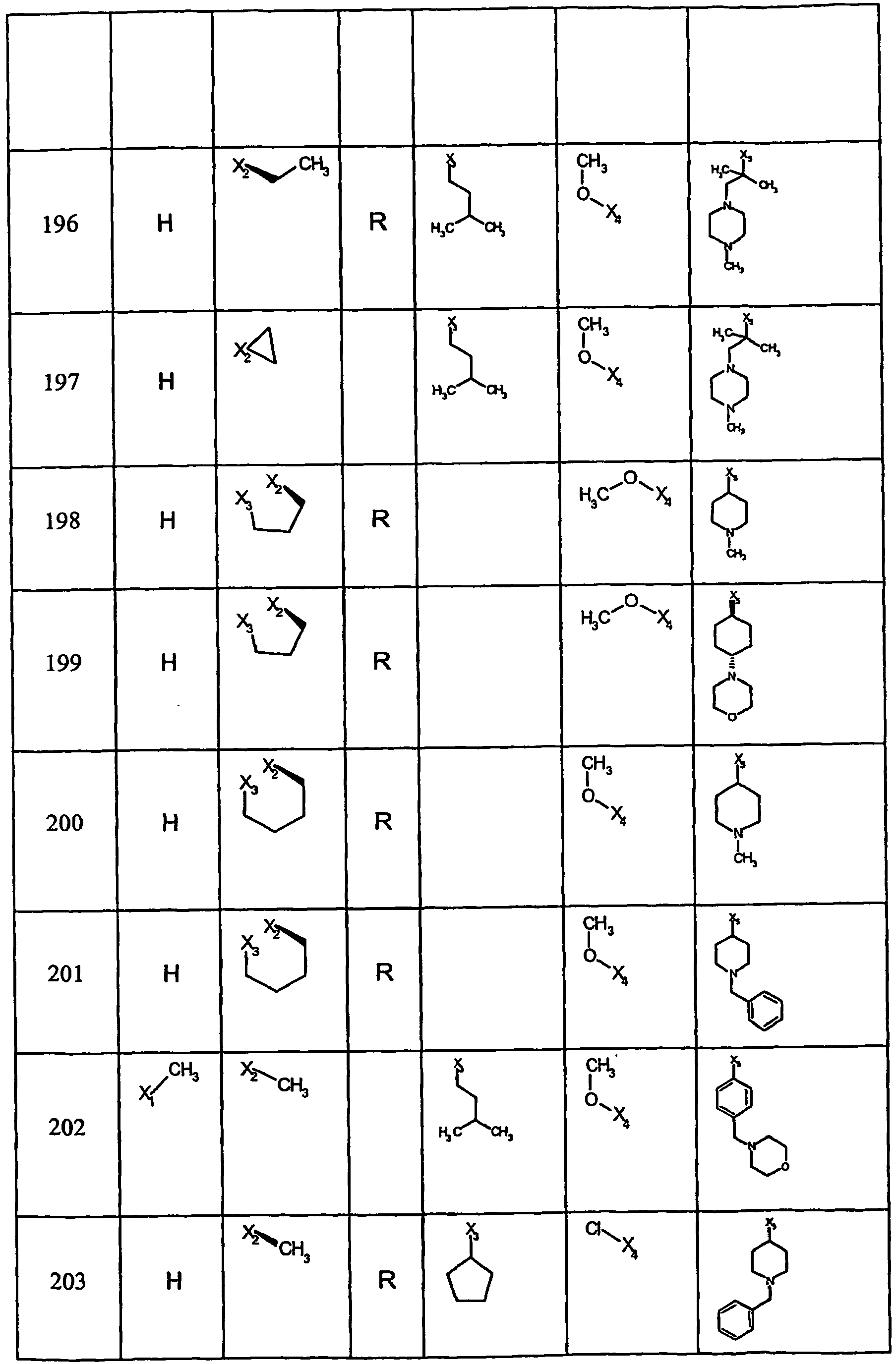

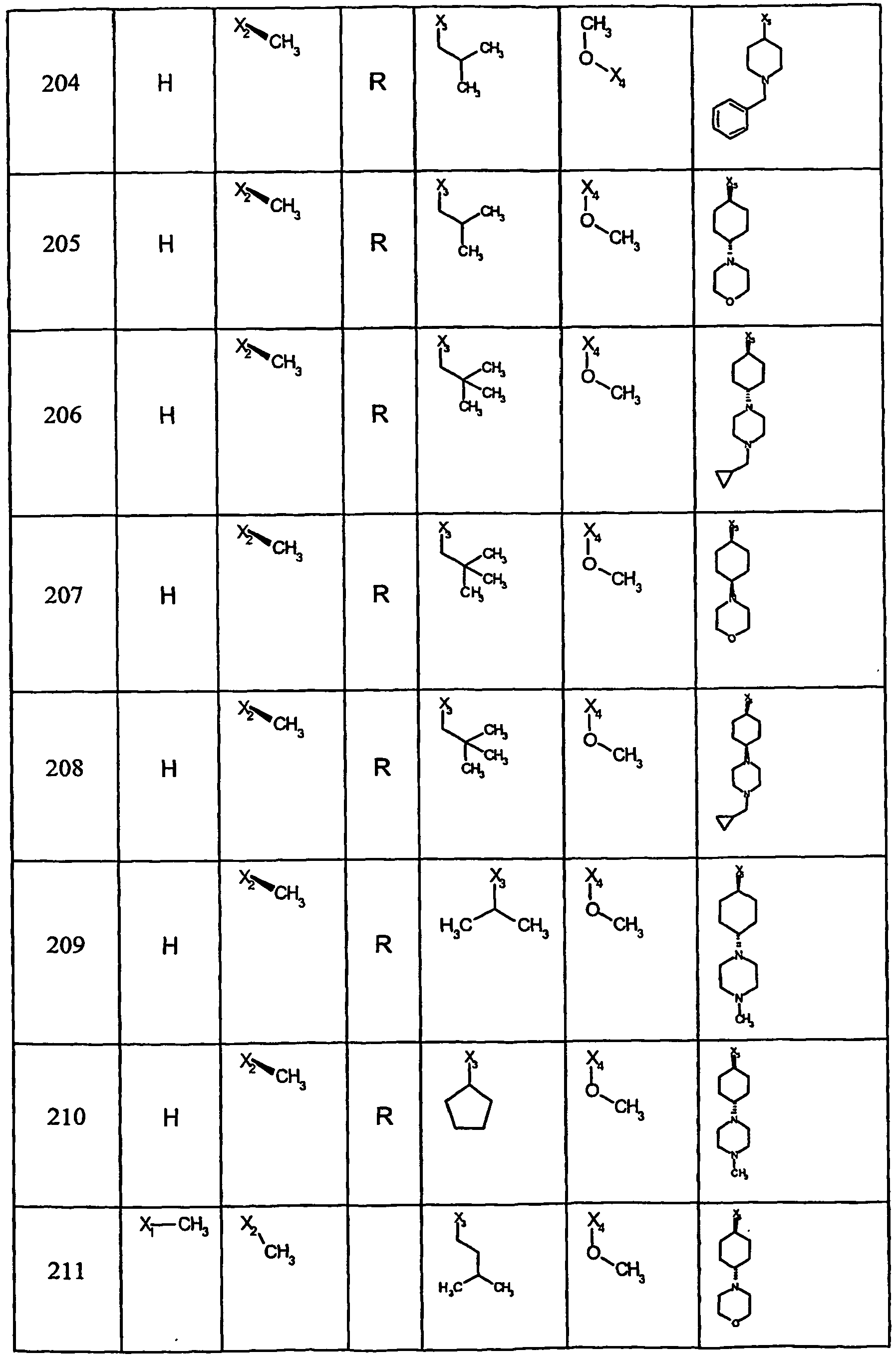

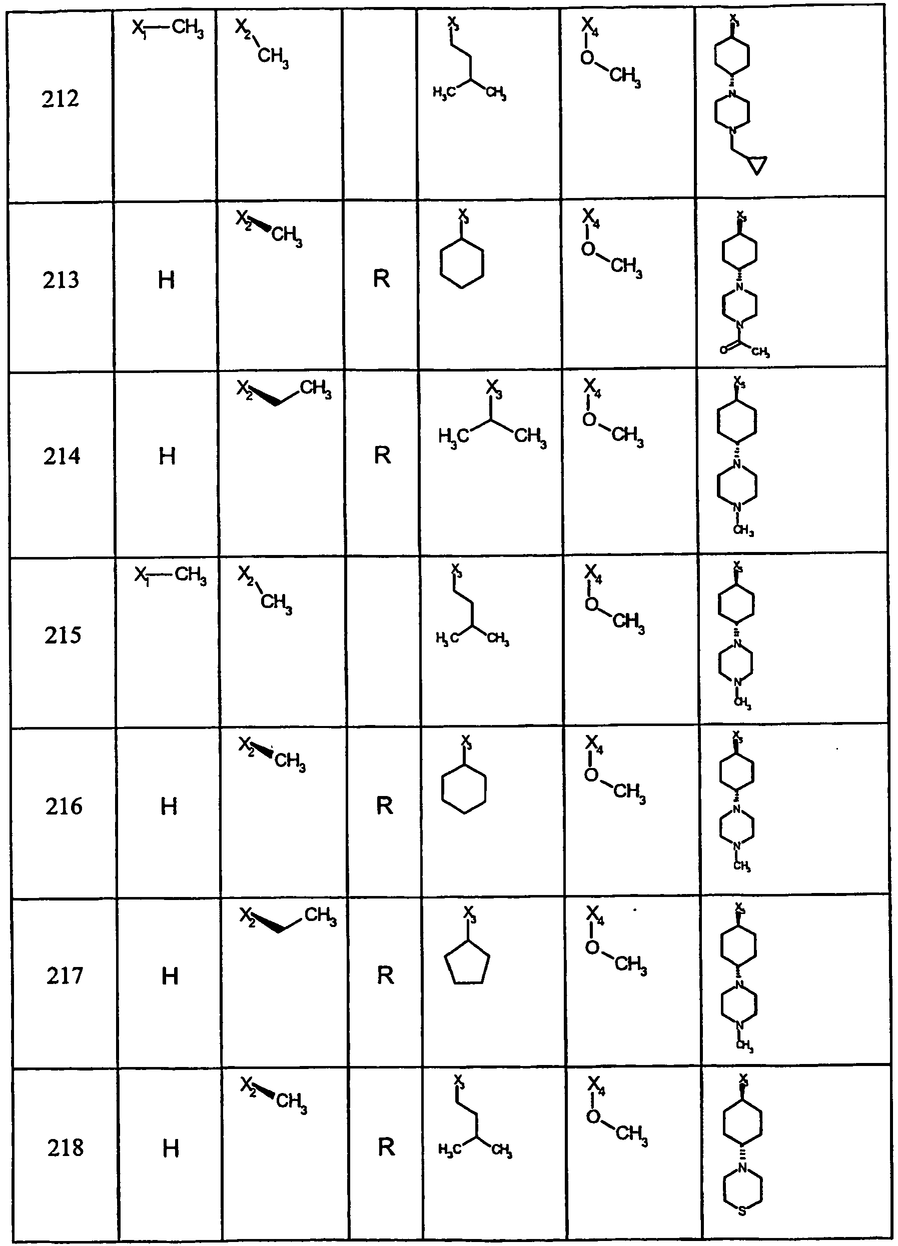

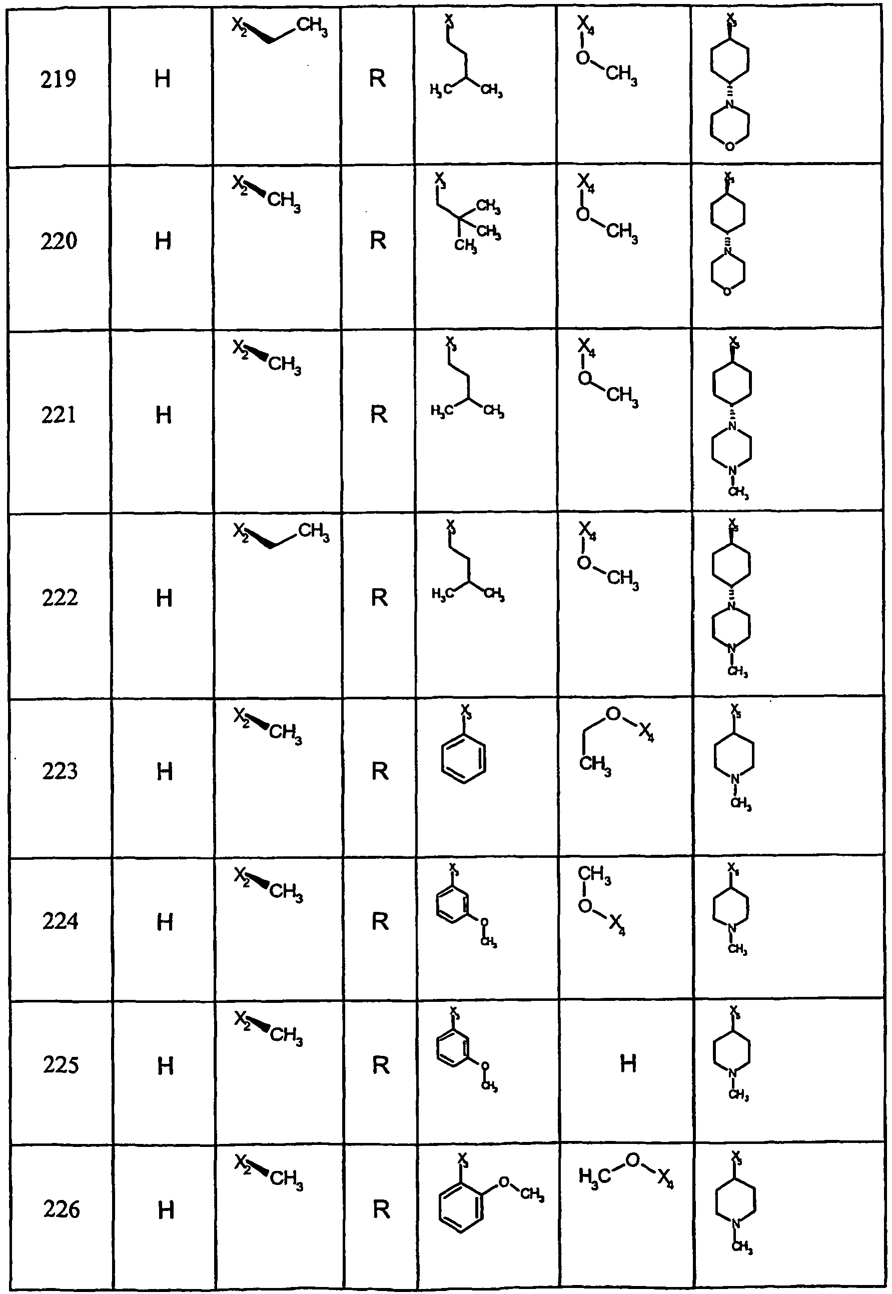

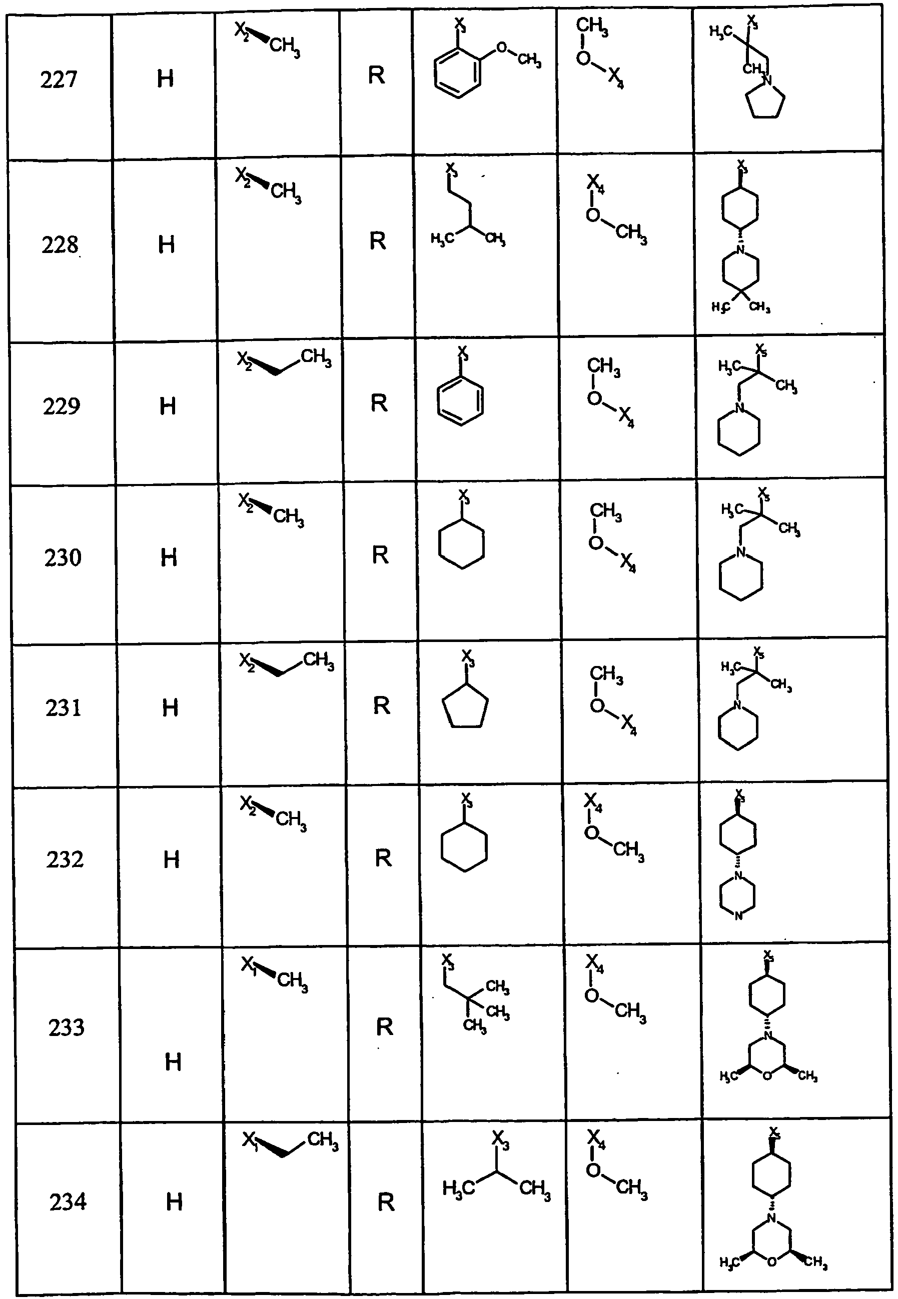

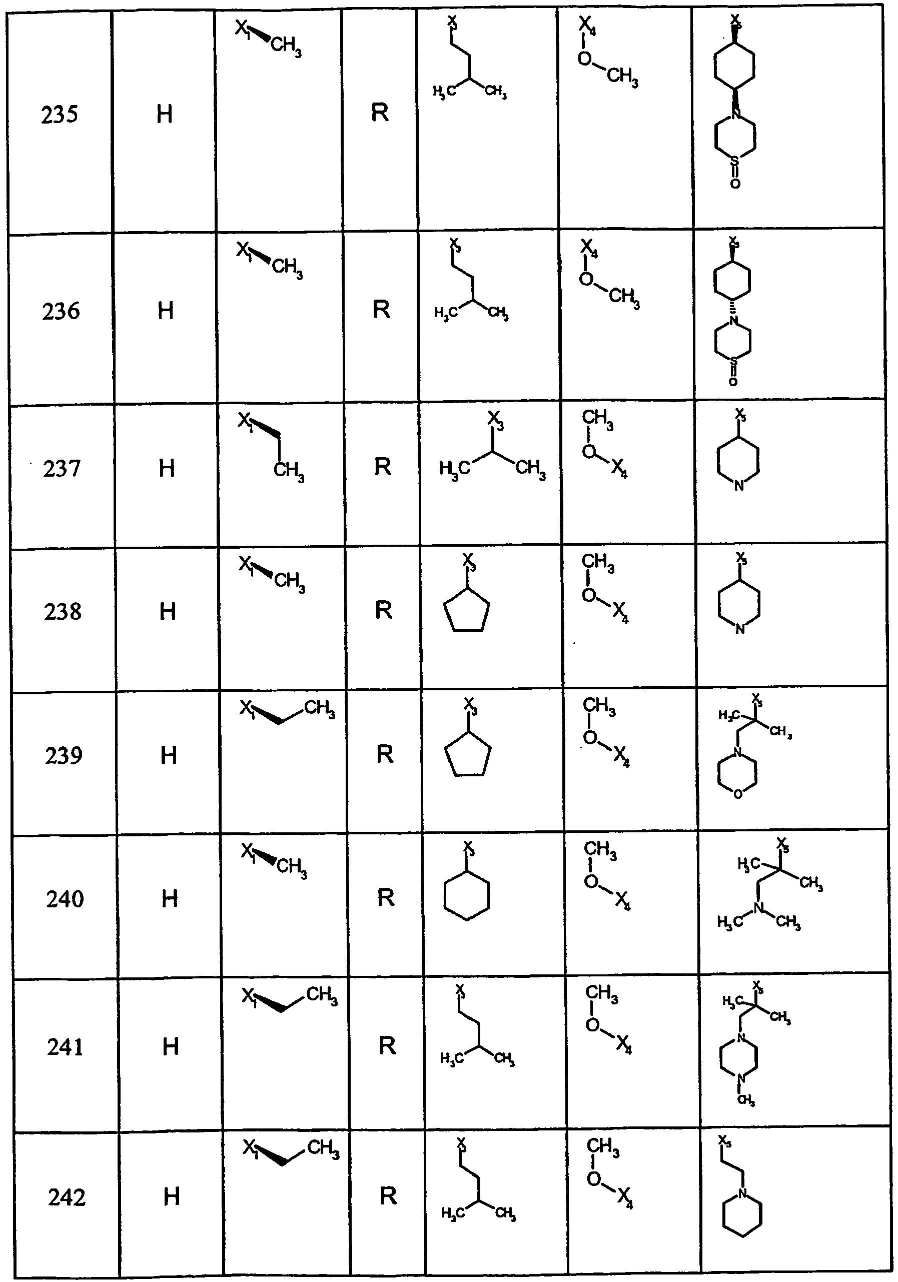

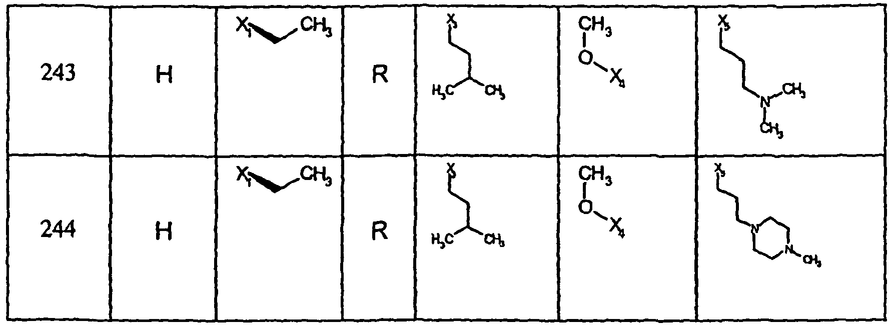

Die folgenden Synthesebeispiele sind als exemplarische Vorgehensweise zur weitergehenden Erläuterung der Erfindung zu verstehen, ohne selbige auf dessen Gegenstand zu beschränken. Die Synthesen sind in Schema (1) bis (3) dargestellt.

### Synthese von Beispielverbindung Nr. 46 (Syntheseschema 1)

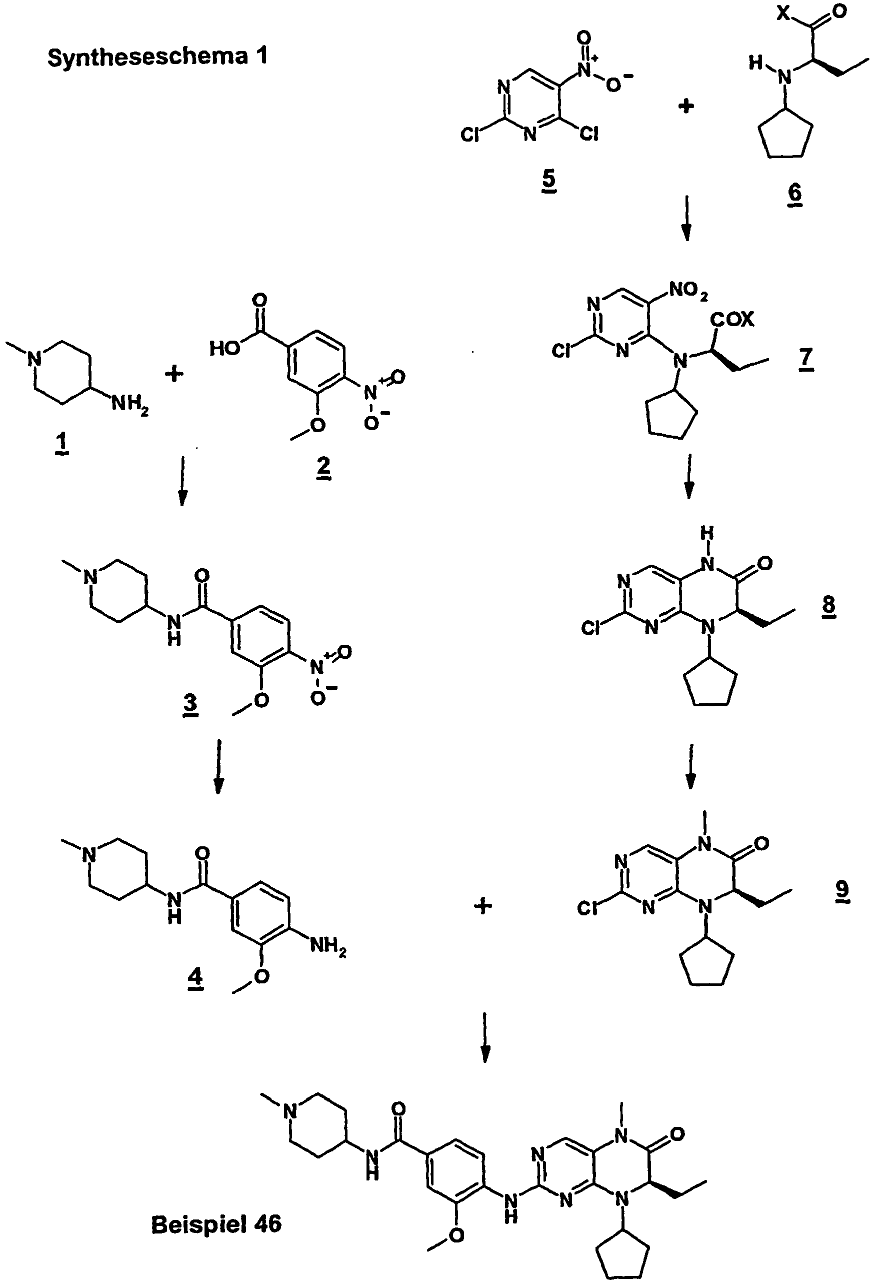

Herstellung des Anilin-Fragments **4** durch:

### ● Herstellung der Verbindung 3

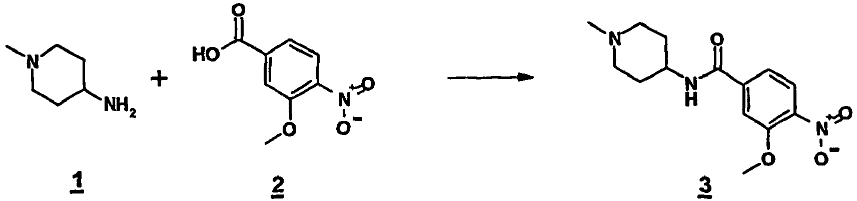

### Variante 1A:

Eine Suspension von 100 g (0.507 mol) 3-Methoxy-4-nitrobenzoesäure **2,** 163 g (0.508 mol) O-(Benzotriazol-1-yl)-N,N,N',N'-tetramethyluroniumtetrafluoroborat und 192 mL (1.1 mol) Ethyldiisopropylamin in 1.2 L Dichlormethan wird eine Stunde bei 25°C gerührt. Zur entstandenen Lösung gibt man 58 g (0.508 mol) 1-Methyl-4-aminopiperidin **1** und rührt 16 Stunden bei 20°C. Die Lösung wird auf 600 mL eingedampft und die organische Phase fünfmal mit 80 mL 1 molarer AmmoniakLösung gewaschen. Die organische Phase wird eingedampft und der Rückstand mit Dichlormethan / Methanol / konz. Ammoniak (15:1:0.1) über Kieselgel chromatographiert. Produktfraktionen werden vereinigt, das Lösungsmittel abgedampft und das Produkt aus Essigsäureethylester / Methanol kristallisiert. Man erhält 123 g Produkt **3**.

### Variante 1B:

4,00 kg (20,3 mol) 3-Methoxy-4-nitrobenzoesäure **2** werden in 54 L Toluol vorgelegt. Bei Normaldruck werden 16 L Toluol abdestilliert. Es wird auf 105°C abgekühlt und 40 ml Dimethylformamid in 2 L Toluol werden zugeben. Bei einer Manteltemperatur von 120°C lässt man 2,90 kg (24,3 mol) Thionylchlorid innerhalb 30 Min. zufließen und spült mit 4 L Toluol nach. Das Reaktionsgemisch wird 1 Stunde unter Rückfluß gerührt. Anschließend werden 12 L Toluol unter Normaldruck abdestilliert. Der Reaktorinhalt wird abgekühlt. Man lässt eine Lösung aus 2,55 kg (22,3 mol) 1-Methyl-4-aminopiperidin **1** in 2 L Toluol und 2,46 kg (24,3 mol) Triethylamin in 2 L Toluol bei 55 - 65°C zufließen. Man spült mit 4 L Toluol nach. Die Suspension wird 1 Stunde gerührt. Man lässt 20 L Wasser zufließen und gibt bei 35-40°C 3,08 kg (30,4 mol) Salzsäure konz. (36%) zu. Man spült mit 2 L Wasser nach. Bei 35 - 40°C entstehen 2 Phasen. Die organischen Phase wird abgetrennt und die produkthaltige Wasserphase wieder in den Reaktor überführt. Es wird mit 4 L Wasser nachgespült. Unter vermindertem Druck werden 3,2 L Wasser bei 50°C abdestilliert. Zur verbleibenden Lösung lässt man bei 40°C 4,87 kg (60,9 mol) Natriumhydroxid-Lsg. (50%) zufließen. Es wird mit 4 L Wasser nachgespült. Die Produktsuspension lässt man auf 22°C abkühlen und rührt 30 Min. bei dieser Temperatur. Die Suspension wird abgenutscht und der Filterkuchen mit 40 L Wasser gewaschen. Das Produkt wird im Vakuumtrockenschrank bei 40°C getrocknet. Man erhält 5.65 kg Produkt.

### • Herstellung der Verbindung 4:

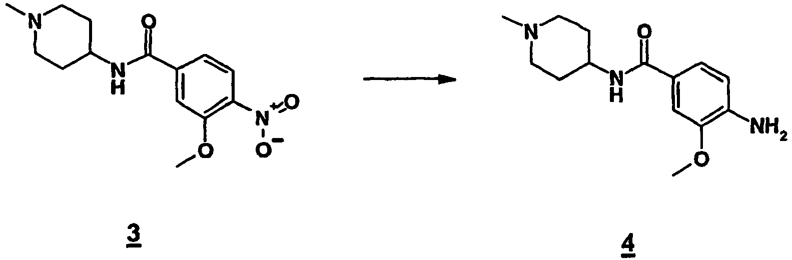

### Variante 2A

Eine Lösung von 145 g (0.494 mol) **3** in 2 L Methanol wird in Gegenwart von 2 g Palladium auf Kohle (10%) bei 4 bar hydriert. Der Katalysator wird abfiltriert und das Filtrat eingedampft. Man erhält 128 g Produkt **4.**

### Variante 2B

Zu 5,00 kg (17,0 mol) **3** und 600 g Aktivkohle (techn.) gibt man 25 L entmineralisiertes Wasser. Anschließend werden 2,05 kg (34,1 mol) Essigsäure zugeben. Die Suspension wird 15 Minuten bei 22-25°C gerührt. Man gibt 500 g Palladium auf Kohle (10%) aufgeschlämmt in 3 L entmineralisiertem Wasser zu und spült mit 2 L entmineralisiertem Wasser nach. Der Reaktorinhalt wird auf 40°C erwärmt und bei dieser Temperatur bis zum Stillstand der Wasserstoffaufnahme hydriert. Das Reaktionsgemisch wird filtriert und der Filterkuchen mit 10 L entmineralisiertem Wasser gewaschen

Zur Kristallisation wird das Filtrat in einen Reaktor transferiert und das Transportgefäß mit 5 L entmineralisiertem Wasser nachgespült. Der Reaktorinhalt wird auf 50°C erwärmt. Man gibt eine Mischung aus 5,45 kg (68,2 mol) Natriumhydroxid-Lsg. (50%, techn.) und 7 L entmineralisiertem Wasser zu. Man rührt 10 Minuten bei 45-50°C. Die Suspension wird auf 20°C abgekühlt und 1-1,5 Stunden bei dieser Temperatur gerührt. Das Produkt wird abgenutscht, mit 30 L entmineralisiertem Wasser gewaschen und bei 45°C im Vakuumtrockenschrank getrocknet. Man erhält 4.13 kg Produkt **4.**

Herstellung des Dihydropteridinon-Fragments **9,** durch:

### ● Herstellung der Aminosäure-Ester 6a-d

| | **6** | **a** | **b** | **c** | **d** |
|---|---|---|---|---|---|
| 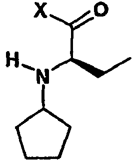 | **X** | 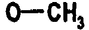 | 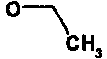 | 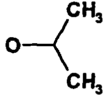 | 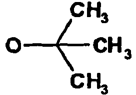 |
| **6** | | | | | |

Die Herstellung des Methylesters **6a,** des Ethylesters 6b und des 2-Propylesters **6c** erfolgt nach literaturbekannten Vorschriften, beispielsweise nach WO 03/020722 A1. Der tert.-Butylester **6d** wird durch Umesterung mit Essigsäure-tert.-butylester in Gegenwart von Perchlorsäure hergestellt (J. Med. Chem., Vol 37, No 20, 1994, 3294-3302).

Die Aminosäuren können in Form der Basen oder als Hydrochloride bei der nachfolgenden nucleophilen Substitutions-Reaktion eingesetzt werden.

### ● Herstellung der Aminosäure-Amide 6e,f

| | **6** | **e** | **f** |
|---|---|---|---|
| 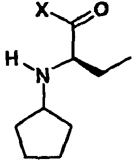 | **X** | 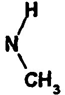 | 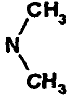 |
| **6** | | | |

Die Herstellung des Aminosäure-Amids **6e** erfolgt durch Aminolyse des Methylesters **6a** mit 40-prozentiger wässriger Methylamin-Lösung bei Raumtemperatur.

Die Herstellung des Aminosäure-Amids **6f** erfolgt durch Amidbildung der freien Aminosäure mit einem fünffachen Überschuß an 2 molarer Dimethylamin- Lösung in Tetrahydrofuran in Gegenwart von O-(Benzotriazol-1-yl)-N,N,N',N'-tetramethyluroniumtetrafluoroborat als Kopplungs-Reagenz.

### ● Herstellung der Verbindungen 7

| | | | | | | |
|---|---|---|---|---|---|---|
| 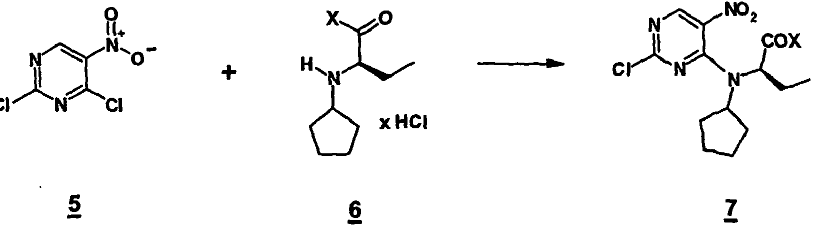 | | | | | | |

| **7** | **a** | **b** | **c** | **d** | **e** | **f** |
|---|---|---|---|---|---|---|
| **x** | O-CH₃ | 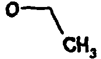 | 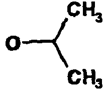 | 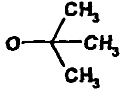 | 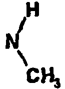 | 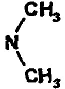 |

### ● Herstellung des Methylesters 7a

Eine Suspension von 457 g (2.06 mol) des Aminosäure-methylesters **6a** und 693 g (8.25 mol) pulverisiertem Natriumhydrogencarbonat in 10 L Cyclohexan rührt man 15 Minuten bei Raumtemperatur. Man gibt 440 g (2.27 mol) 2,4-Dichlor-5-nitropyrimidin **5** und 1.5 L Cyclohexan zu und rührt 3 Tage bei Raumtemperatur. Die Umsetzung wird mittels HPLC kontrolliert. Um auskristallisiertes Produkt wieder in Lösung zu bringen, gibt man zur Suspension 4 L Dichlormethan. Nach Zugabe von 335 g Magnesiumsulfat wird die Suspension abgenutscht und der anorganische Filterkuchen mit Dichlormethan nachgewaschen. Das Filtrat wird unter vermindertem Druck auf 3.1 kg eingedampft und die erhaltene Suspension zum Rückfluß erhitzt. Die Lösung lässt man langsam abkühlen und rührt eine Stunde bei 10-15°C. Die Suspension wird abgenutscht und der Filterkuchen mit Cyclohexan gewaschen. Das Produkt wird im Vakuumtrockenschrank bei 40°C getrocknet. Man erhält 582 g **7a** (X = OCH₃) als dunkelgelben Feststoff.

Analog dieser Herstellprozedur werden die Verbindungen **7b-f** hergestellt. Bei der Umsetzung der Aminosäure-Amide **7e,f** wird zur Verbesserung der Löslichkeit ein etwas polareres Lösungsmittel wie z.B. Ethylacetat oder Dichlormethan zugegeben.

### ● Herstellung von Verbindung 8

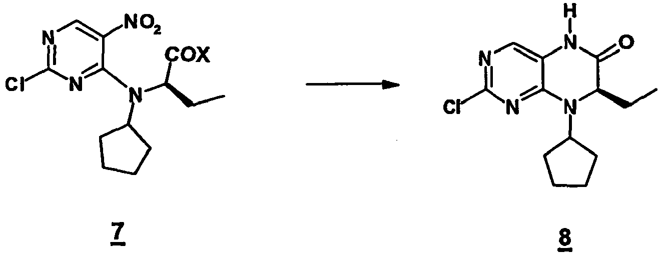

Eine frisch hergestellte Suspension von 560 g (1.63 mol) **7a** und 185 g Raney-Nickel in 2.8 L Essigsäure wird bei 75 °C hydriert. Nach beendeter Wasserstoffaufnahme wird der Katalysator abfiltriert und die Hydrierlösung unter vermindertem Druck eingedampft. Zum Rückstand gibt man 4 L entmineralisiertes Wasser und 4 L Essigsäureethylester. Zwischen den Phasen bildet sich ein Niederschlag, der Produkt enthält. Die wässrige Phase wird abgetrennt. Zur organischen Phase gibt man 2 L Essigsäureethylester und nutscht den Niederschlag ab. Der Niederschlag wird in 600 mL entmineralisiertem Wasser suspendiert, 1 Stunde bei Raumtemperatur gerührt, abgenutscht und mit entmineralisiertem Wasser gewaschen. Man erhält 110 g feuchtes Produkt A.

Das Filtrat wird dreimal mit Natriumchlorid-Lösung gewaschen. Die organische Phase wird eingedampft. Man erhält 380 g eines rötlich-braunen Rückstands B, der mit dem feuchten Produkt A vereinigt wird. Die vereinigten Rohprodukte A und B werden unter Rückfluß in 1.5 L Ethanol gelöst. Die Lösung wird klarfiltriert und der Filter mit 150 mL Ethanol nachgespült. Unter Rückfluß gibt man zur Lösung 550 mL entmineralisiertem Wasser. Man lässt abkühlen und rührt 16 Stunden bei Raumtemperatur und 3 Stunden bei 0-5°C. Der Niederschlag wird abgenutscht und mit entmineralisiertem Wasser/Methanol (1:1) und anschließend mit entmineralisiertem Wasser gewaschen. Das Produkt wird im Vakuumtrockenschrank bei 50°C getrocknet. Man erhält 266 g Produkt **8** als Feststoff.

### ● Herstellung der Verbindung 9

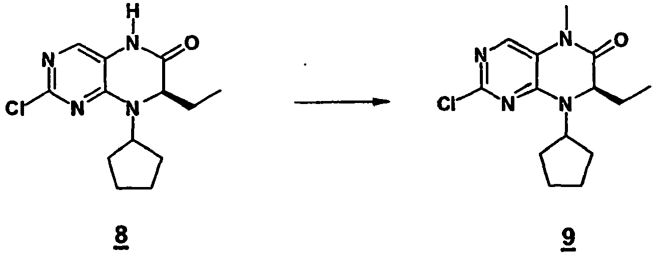

Zu einer Lösung von 264 g (0.94 mol) **8** und 161 g (1.13 mol) Methyliodid in 2 L Dimethylacetamid gibt man bei 4-10°C portionsweise innerhalb einer Stunde 38 g (0.95 mol) Natriumhydrid (60 prozentige Dispersion in Mineralöl). Man entfernt das Kühlbad und lässt innerhalb von 2 Stunden auf 20°C erwärmen. Man kühlt auf 10°C und gibt weitere 0.38 g (9.5 mmol) Natriumhydrid zu. Man rührt 4 Stunden bei 10-15°C. Zur Reaktionslösung gibt man 100 mL Essigsäureethylester und 1 kg Eis. Die entstehende Suspension wird mit 3 L entmineralisiertem Wasser verdünnt. Die Suspension wird 2 Stunden gerührt, der Niederschlag abgenutscht und der Filterkuchen mit entmineralisiertem Wasser gewaschen. Das Produkt wird im Vakuumtrockenschrank bei 50°C getrocknet. Man erhält 273 g Produkt **9** als farblose Kristalle.

Herstellung von Beispielverbindung Nr. 46 durch Umsetzung von **4** mit **9:**

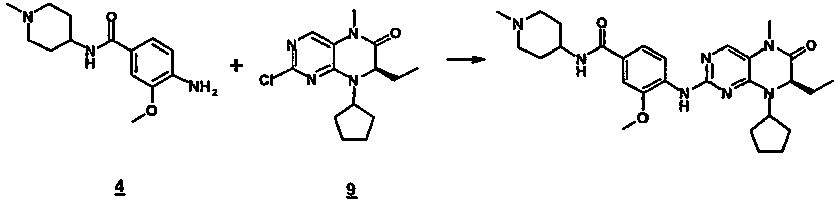

Eine Suspension aus 201 g (1.06 mol) para-Toluolsulfonsäure-Hydrat, 209 g (706 mmol) **9** und 183 g (695 mmol) **4** in 800 mL 2-Methyl-4-pentanol wird zum Rückfluß erhitzt. Es werden 100 mL Lösungsmittel abdestilliert. Man erwärmt 3 Stunden zum Rückfluß, gibt 200 mL 2-Methyl-4-pentanol zu und destilliert 120 mL Lösungsmittel ab. Nach 2 Stunden erwärmen unter Rückfluß werden weitere 280 mL Lösungsmittel abdestilliert. Man lässt auf 100°C abkühlen und gibt zur Reaktionslösung 1 L entmineralisiertes Wasser und anschließend 0.5 L Essigsäureethylester. Die organische Phase wird abgetrennt und die wässrige Phase nochmals mit 0.5 L Essigsäureethylester gewaschen. Zur sauren, wässrigen Phase gibt man 1.5 L Dichlormethan und 0.5 L Essigsäureethylester. Der pH-Wert der wässrigen Phase wird mit 260 mL 6 normaler Natronlauge auf pH 9.2 gebracht. Die wässrige Phase wird abgetrennt und die organische Phase dreimal mit jeweils 1 L 1 normaler wässriger Natriumhydrogencarbonat-Lösung gewaschen. Die organische Phase wird über Natriumsulfat getrocknet, filtriert und das Lösungsmittel unter vermindertem Druck eingedampft. Man erhält 406 g Rohprodukt.

Das Rohprodukt wird in 1.5 L Essigsäureethylester gelöst. Bei einer Temperatur von 50-55°C gibt man 2.5 L Methyl-tert.-butylether zu. Bei 45°C wird angeimpft und unter Abkühlen auf Raumtemperatur 16 Stunden gerührt. Man rührt die Suspension 3.5 Stunden bei 0-5°C und nutscht den Niederschlag ab. Der Filterkuchen wird mit Methyl-tert.-butylether /Essigsäureethylester (2:1) und Methyl-tert.-butylether nachgewaschen. Das Produkt wird im Vakuumtrockenschrank bei 50°C getrocknet. Man erhält 236 g kristallines Beispielverbindung Nr. 46 als Anhydrat (I).

### Kristallisation:

46.5 g des vorgehend beschriebenen kristallinen Anhydrats (I) werden in 310 mL 1-Propanol gelöst und klarfiltriert. Man erwärmt auf 70°C und gibt 620 mL entmineralisiertes Wasser hinzu. Die Lösung lässt man auf Raumtemperatur abkühlen, kühlt auf 0-10°C und gibt Impfkristalle zu. Die entstehende Suspension wird 3 Stunden bei 0-10°C gerührt. Man nutsch ab und wäscht mit kaltem 1-Propanol/entmineralisiertem Wasser (1:2) und entmineralisiertem Wasser nach. Das Produkt wird im Vakuumtrockenschrank bei 50°C getrocknet. Man erhält 40.5 g kristallines Beispielverbindung Nr. 46 als Monohydrat.

Das Rohprodukt der vorgehend beschriebenen Umsetzung kann auch direkt als kristallines Monohydrat aus 1-Propanol/entmineralisiertem Wasser kristallisiert werden.

### Synthese der Beispielverbindung Nr. 27, Beispielverbindung Nr. 110, und Beispielverbindung Nr. 234, (Syntheseschema 2)

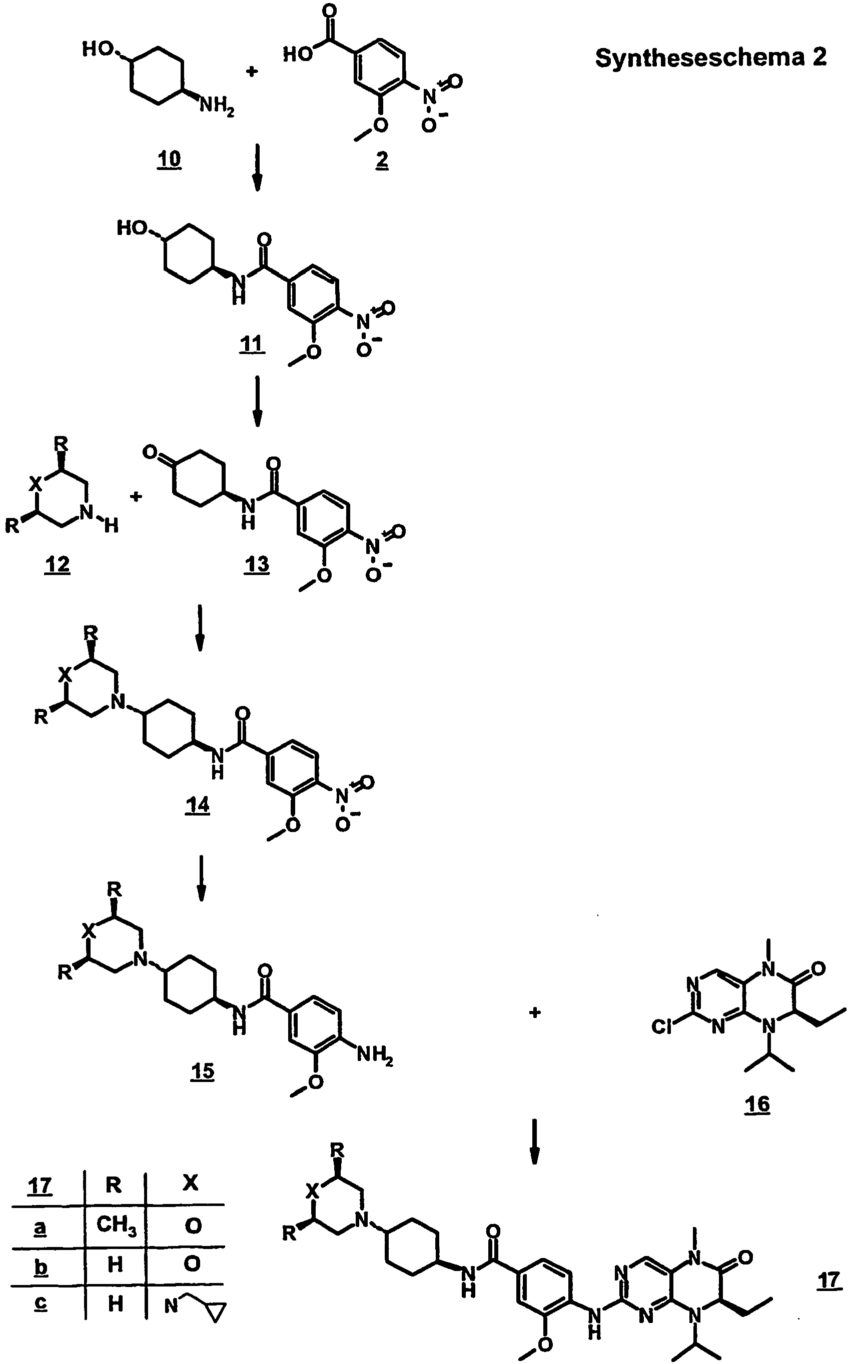

### Herstellung von 11

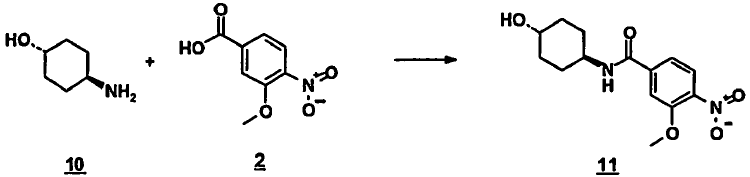

260g (1.32 mol) 3-Methoxy-4-nitrobenzoesäure **2** werden in 1.5 L Toluol vorgelegt. Man destilliert 300 mL Toluol ab. Zum Rückstand gibt man 5 mL Dimethylformamid und tropft 123 mL. (1.7 mol) Thionylchlorid zu. Die Reaktionslösung wird 2 Stunden zum Rückfluß erwärmt. Das Lösungsmittel wird am Rotationsverdampfer unter vermindertem Druck eingedampft. Der Rückstand wird in 500 mL Tetrahydrofuran gelöst und zu einer Suspension von 202g (1.33 mol) trans-4-Aminocyclohexanol **10** in 1.5 L Tetrahydrofuran und 1.38 L einer 30-prozentigen Kaliumcarbonat-Lösung zugetropft, so dass die Temperatur zwischen 5° bis 13°C gehalten wird. Man rührt 1 Stunde bei 20°C und gibt 5 L entmineralisiertes Wasser zu. Der Niederschlag wird abgenutscht und mit entmineralisiertem Wasser gewaschen. Der Feststoff wird bei 70°C im Umlufttrockenschrank getrocknet. Man erhält 380g (98% der Theorie) Produkt **11.** DC (Methylenchlorid/Ethanol = 9:1) R_{f} = 0.47

### Herstellung von 13

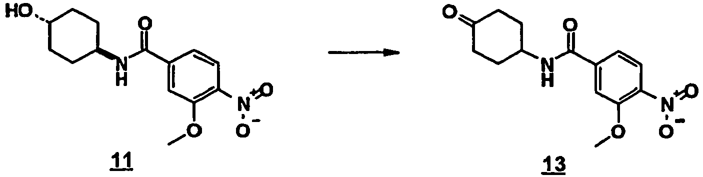

Zu 185g (0.63 mol) **11** und 234g N-Methylmorpholin-N-oxid in 1.8 L Acetonitril gibt man 1 g fein gepulvertes Ruthenium(III)-chloridhydrat und erwärmt 1 Stunde zum Rückfluß. Unter verminderten Druck werden 1.6 L Acetonitril abgedampft. Zum Rückstand gibt man 1.5 L entmineralisiertes Wasser und kühlt die Suspension auf 5°C. Der Niederschlag wird abgenutscht und mit viel entmineralisiertes Wasser gewaschen Der Feststoff wird bei 70°C im Umlufttrockenschrank getrocknet. Man erhält 168g (91% der Theorie) Produkt **13.**
DC (Methylenchlorid/Ethanol = 9:1) R_{f} = 0.64

### Herstellung von 14a

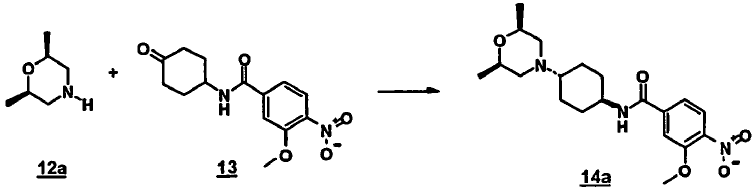

164g (0.51 mol) **13** (90%ig), 80.1 mL (0.65 mol) cis-2,6-Dimethylmorpholin **12** und 60g Natriumacetat in 1.4 L Tetrahydrofuran werden 1 Stunde zum Rückfluß erwärmt. Man kühlt auf 20°C und gibt portionsweise 120g (0.57 mol)
Natriumtriacetoxyborhydrid zu, so dass die Temperatur zwischen 18° bis 22°C gehalten wird. Man rührt 16 Stunden bei 20°C. Das Lösungsmittel wird unter vermindertem Druck eingedampft. Der Rückstand wird in 2 normaler Salzsäure gelöst. Zur Lösung gibt man 10g Aktivkohle und nutscht ab. Zum Filtrat gibt man 300 mL Diisopropylether und anschließend Ammoniaklösung, bis die wässrige Phase alkalisch ist. Es wird eine Stunde gerührt und die Suspension auf 5°C gekühlt. Die Suspension wird abgenutscht und der Feststoff mit entmineralisiertem Waser gewaschen. Das Rohprodukt wird aus 1.2 L Isopropanol kristallisiert. Das abgenutschte, kristalline Produkt wird im Umlufttrockenschrank bei 50°C getrocknet. Man erhält 84g (43% der Theorie) Verbindung **14a.**
DC (Methylenchlorid/Ethanol = 9:1) R_{f}= 0.45

### Herstellung von 14b

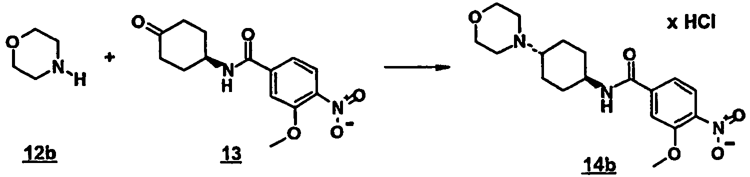

Eine Suspension aus 65.3g (223 mmol) **13,** 23.6 mL (268 mmol) Morpholin **12b** und 0.4 mL Methansulfonsäure in 600 mL Toluol wird am Wasserabscheider bis zur vollständigen Enamin-Bildung zum Rückfluß erwärmt. Das Lösungsmittel wird unter vermindertem Druck abdestilliert bis ein Rückstandsvolumen von 100 mL verbleibt. Der Rückstand wird mit 400 mL Ethanol bei 80°C gelöst und auf 0° bis 5°C abgekühlt. Bei dieser Temperatur gibt man portionsweise 10.1g Natriumborhyrid zu und rührt anschließend 16 Stunden bei 20°C. Man gibt zur Lösung Eis und stellt durch Zugabe von halbkonzentrierter Salzsäure auf pH = 8 bis 9. Die Lösungsmittel werden unter vermindertem Druck eingedampft. Der Rückstand wird in Methylenchlorid suspendiert und mit einem Lösungsmittelgemisch aus Methylenchlorid/Ethanol/Ammoniak (49:1:0.25 bis 19:1:0.25) über Kieselgel chromatographiert. Zuerst eluiert die cis-Verbindung [DC (Methylenchlorid/Ethanol/Ammoniak = 19:1:0.25) R_{f} = 0.23]. Fraktionen, die die trans-Verbindung [DC (Methylenchlorid/Ethanol/Ammoniak = 19:1:0.25) R_{f} = 0.12] enthalten, werden vereinigt und eingedampft. Der Rückstand wird in 350 mL Methanol in der Siedehitze suspendiert. Bei ca. 50°C gibt man 2 Moläquivalente Trimethylchlorsilan zu und anschließend 500 mL tert.-Butylmethylether zu. Die Suspension wird abgenutscht und der Feststoff getrocknet. Man erhält 24g (27% der Theorie) Verbindung **14b** als Hydrochlorid.
DC (Methylenchlorid/Ethanol/Ammoniak = 19:1:0.25) R_{f} = 0.12

### Herstellung von 14c

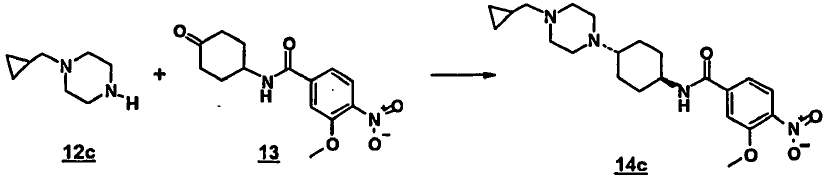

112g (383 mmol) **13**, 108g (770 mmol) N-(Cyclopropylmethyl)piperazin **12c** und 4.5 mL Methansulfonsäure in Toluol werden 3 Stunden am Wasserabscheider zum Rückfluß erwärmt (ca. 76 mL Wasser werden abgeschieden). Unter vermindertem Druck werden 900 mL Toluol abgedampft und der Rückstand in 1.2 L Ethanol suspendiert. Zu dieser Suspension gibt man portionsweise bei einer Temperatur von 15° bis 25°C 15g Natriumborhydrid innerhalb von einer Stunde. Man rührt 3 Stunden bei 20°C und gibt weitere 4g Natriumborhydrid zu. Man rührt 16 Sunden bei 20°C. Unter vermindertem Druck werden 650 mL Ethanol abgedampft. Man gibt 2 L gereinigtes Wasser und 300 mL Cyclohexan zu. Man kühlt auf 5°C und nutscht die Suspension ab. Der Rückstand wird in 1 normaler Salzsäure gelöst. Man gibt 5g Aktivkohle dazu und nutscht ab. Zum Fitrat gibt man 400 mL tert.-Butylmethylether und stellt mit Ammoniaklösung alkalisch. Man kühlt auf 4°C, nutscht den Niederschlag ab und wäscht mit entmineralisiertem Wasser nach. Der Rückstand wird in 400 mL tert.-Butylmethylether zum Rückfluß erwärmt. Es wird abgekühlt, der Feststoff abgenutscht und mit tert.-Butylmethylether nachgewaschen. Nach Trocknen im Umlufttrockenschrank bei 60°C erhält man 73g (46% der Theorie) Produkt 14c. DC (Methylenchlorid/Ethanol = 9:1) R_{f} = 0.2

### Herstellung von 15a

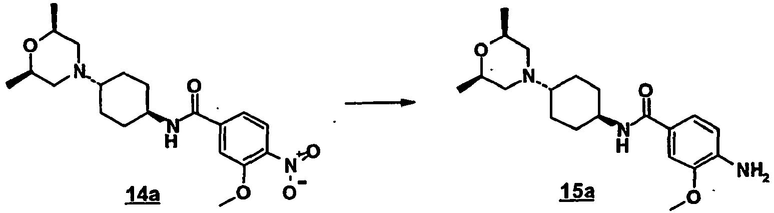

Eine Lösung von 108.5g (277 mmol) **14a** in 900 mL Essigsäure wird in Gegenwart von 10g Raney-Nickel bei einer Temperatur von 20°C und einem Wasserstoffdruck von 50psi hydriert. Der Katalysator wird abfiltriert und die Lösung unter vermindertem Druck eingedampft. Der Rückstand wird in 500 mL Isopropanol gelöst und durch Zugabe von Ammoniaklösung alkalisch gestellt. Man gibt soviel Eiswasser zu, dass ein Volumen von 1.5 L erreicht wird. Der Niederschlag wird abgenutscht und mit 400 mL entmineralisiertem Wasser, 160 mL Isopropanol und 300 mL tert.-Butylmethylether gewaschen. Der Feststoff wird im Umlufttrockenschrank bei 50°C getrocknet. Man erhält 92g (92% der Theorie) Produkt **15a.**
DC (Methylenchlorid/Ethanol = 9:1) R_{f} = 0.25

### Herstellung von 15b

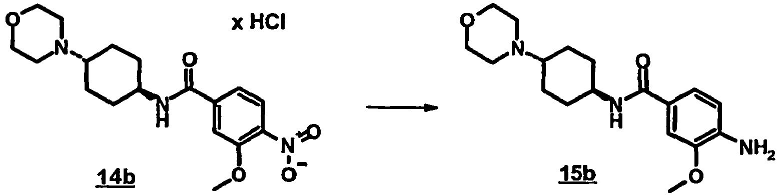

Eine Lösung von 23g (57.5 mmol) **14b** Hydrochlorid in 200 mL entmineralisiertem Wasser wird in Gegenwart von 5g Palladium auf Kohle (10%) bei einer Temperatur von 20°C und einem Wasserstoffdruck von 50psi hydriert. Der Katalysator wird abfiltriert und das Filtrat langsam durch Zugabe von 1 normaler Natronlauge auf pH = 11 gebracht. Die Suspension wird 2 Stunden bei 20°C gerührt, abgenutscht und der Feststoff mit entmineralisiertem Wasser gewaschen. Nach Trocknen im Vakuumtrockenschrank erhält man 17.5g Produkt **15b.**

### Herstellung von 15c

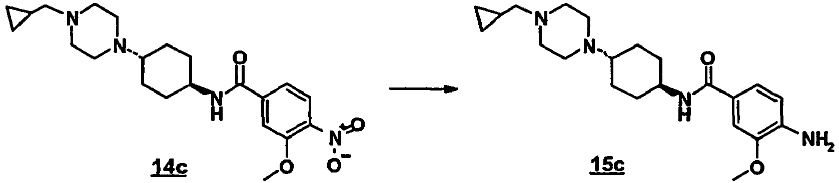

Eine Lösung von 72.5g (174 mmol) **14c** in 700 mL Methanol und 145 mL Dimethylformamid wird in Gegenwart von 10g Raney-Nickel bei einer Temperatur von 20°C und einem Wasserstoffdruck von 50psi hydriert. Der Katalysator wird abfiltriert und das Methanol unter vermindertem Druck eingedampft. Zum Rückstand gibt man 500 mL entmineralisiertes Wasser und kühlt die Suspension auf 5°C. Der Niederschlag wird abgenutscht und mit entmineralisiertes Wasser gewaschen. Nach dem Trocknen in Umlufttrockenschrank bei 60°C erhält man 60.5g (90% der Theorie) Produkt **15c.**
DC (Methylenchlorid/Ethanol/Ammoniak = 9:1:0.1) R_{f}= 0.58

### Herstellung von 17a (entspricht Beispielverbindung Nr. 234)

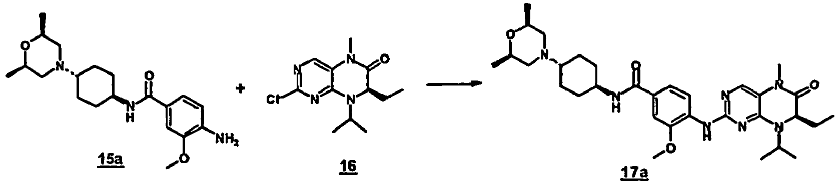

Eine Lösung aus 20.2g (55.9 mmol) **15a,** 16.5g (61.4 mmol) **16** und 15.9g (83.6 mmol) para-Toluolsulfonsäure Hydrat in 400 mL 2-Methyl-4-pentanol wird 9 Stunden zum Rückfluß erwärmt, wobei über die gesamte Zeitspanne insgesamt 360 mL Lösungsmittel abdestilliert werden. Den Rückstand lässt man abkühlen und löst das erstarrte Öl in 300 mL entmineralisiertem Wasser. Die wässrige Phase wird dreimal mit Essigsäureethylester gewaschen. Zur wässrigen Phase gibt man 400 mL Essigsäureethylester und gibt soviel Natronlauge zu, dass der pH Wert = 11 bis 12 erreicht. Die organische Phase wird zweimal mit entmineralisiertem Wasser gewaschen. Die organische Phase wird über Natriumsulfat getrocknet, filtriert und das Lösungsmittel unter vermindertem Druck abgedampft. Der Rückstand wird in 82 mL Dimethylacetamid gelöst und langsam unter gutem Rühren auf eine Lösung aus 60 mL konz. Ammoniak in 1.4 L entmineralisiertem Wasser getropft. Man rührt 4 Stunden bei 20°C, nutscht den Niederschlag ab und wäscht mit viel entmineralisiertem Wasser nach. Nach Trocknen bei 60°C im Vakuumtrockenschrank in Gegenwart von Natriumhydroxid-Plätzchen erhält man 30.3g Produkt **17a.**
Die so erhaltene Base kann aus Aceton / 1 normale Salzsäure als Monohydrochlorid mit einem Smp.: ca. 320°C (Zersetzung, DSC: 10 K/min) kristallisiert werden.

Herstellung von **17b** (entspricht Beispielverbindung Nr. 27)

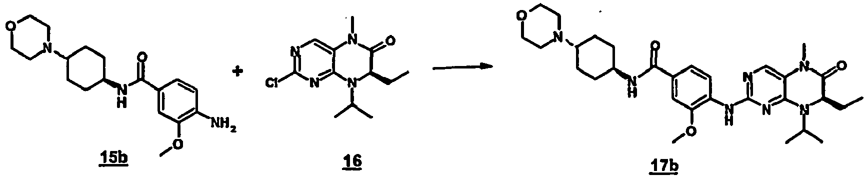

Eine Lösung aus 16.2g (48.6 mmol) **15b,** 14.5g (54 mmol) **16** und 13g (68.3 mmol) para-Toluolsulfonsäure Hydrat in 250 mL 2-Methyl-4-pentanol und 20 mL N-Methylpyrrolidinon wird zum Rückfluß erwärmt. Es werden innerhalb einer Stunde 180 mL Lösungsmittel abdestilliert. 100 mL 2-Methyl-4-pentanol werden wieder zugegeben und die Lösung wird 5 Stunden unter Rückfluß erwärmt. Man lässt auf 80°C abkühlen und gibt 40 mL Methanol und 12g Trimethylsilylchlorid zu. Bei 60°C lässt man 400 mL Aceton zufließen. Die Suspension wird zum Rückfluß erwärmt und auf 30°C abgekühlt. Der Niederschlag wird abgenutscht, mit Aceton / Methanol (85:15) und Aceton gewaschen. Nach Trocknen im Vakuumtrockenschrank bei 50°C erhält man 22.7g (78% der Theorie) Produkt **17b** als Hydrochlorid.
Nach Auflösen des Hydrochlorids in entmineralisiertem Wasser und Überführen in eine wässrige Lösung aus Kaliumcarbonat und Kochsalz wird die freie Base mit Methylenchlorid extrahiert. Die Base von **17b** wird aus Aceton / entmineralisiertem Wasser (1:1) kristallisiert (Smp. = 150°C, DSC: 10 K/min).

### Herstellung von 17c (entspricht Beispielverbindung Nr. 110)

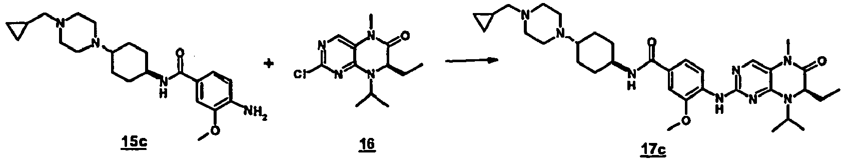

Eine Lösung aus 23g (59.5 mmol) **15c**, 16.8g (62.5 mmol) **16** und 28.3g (149 mmol) para-Toluolsulfonsäure Hydrat in 350 mL 2-Methyl-4-pentanol wird am Wasserabscheider 22 Stunden zum Rückfluß erwärmt. Nach Zugabe von 1g **16** erwärmt man weitere 2 Stunden zum Rückfluß. Man destilliert 300 mL Lösungsmittel ab und lässt das zähflüssige Öl auf 60°C abkühlen. Man gibt 300 mL Methylenchlorid und 300 mL entmineralisiertes Wasser zu und erhöht den pH-Wert durch Zugabe von ca. 20 mL 10 normaler Natronlauge auf pH = 9. Die organische Phase wird zweimal mit entmineralisiertem Wasser gewaschen und über Natriumsulfat getrocknet. Das Lösungsmittel wird unter vermindertem Druck abgedampft und der Rückstand bei 65°C in 200 mL Essigsäureethylester gelöst. Man lässt langsam aus 20°C abkühlen, nutscht den Niederschlag ab und wäscht mit kalten Essigsäureetylester nach. Nach Trocknen bei 60°C im Vakuumtrockenschrank erhält man 24.4g Produkt **17c** (Smp. = 182°C, DSC: 10 K/min, zusätzliche endotherme Effekte im DSC-Diagramm vor dem Schmelzen).

Die Verbindung **14c** kann alternativ auch nach folgendem Verfahren hergestellt werden (Syntheseschema 3).

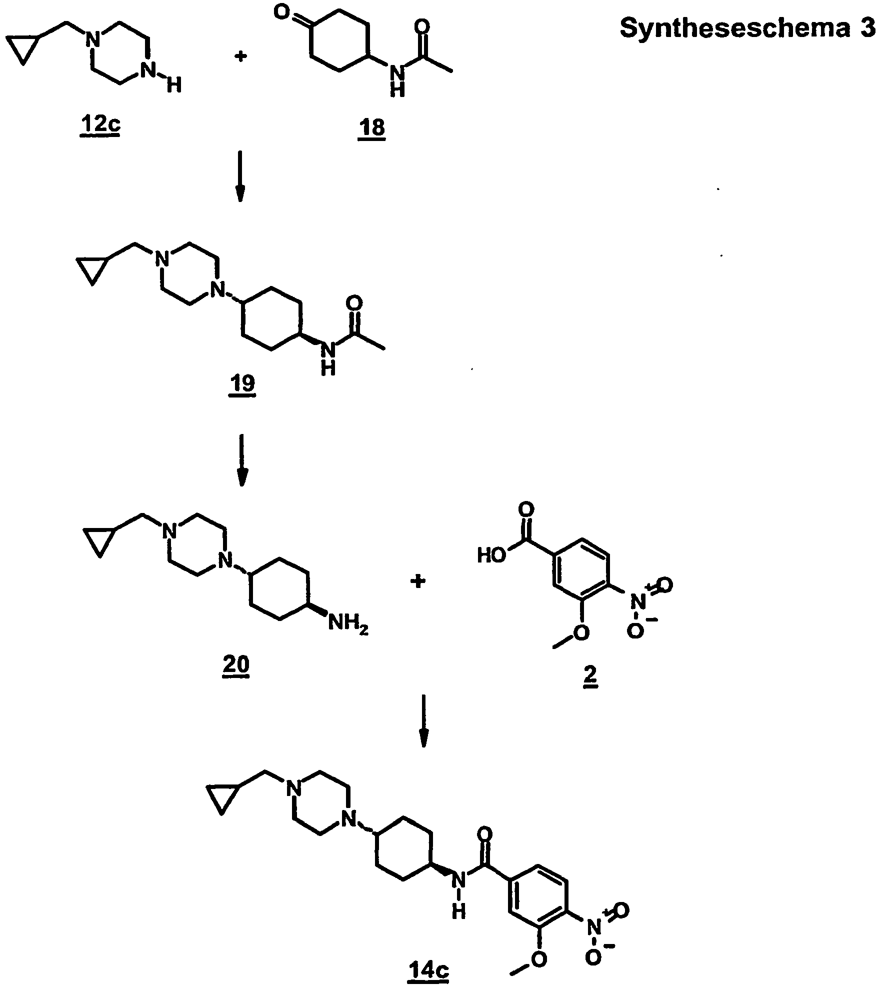

### Herstellung von 19

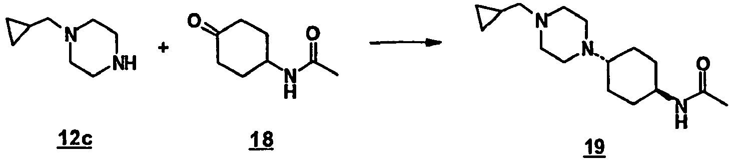

22g (142 mmol) 4-Acetamido-cyclohexanon **18**, 39.7g (283 mmol) N-Cyclopropylmethylpiperazin **12c** und 0.71 mL Methansulfonsäure in 175 mL Toluol werden am Wasserabscheider zum Rückfluß erwärmt, bis kein Wasser mehr abgeschieden wird. Man lässt abkühlen und gibt bei 50°C 175 mL Ethanol zu und kühlt auf 20°C. Unter gutem Rühren gibt man portionsweise 5.37g (142 mmol) Natriumborhydrid zu und rührt 16 Stunden bei 20°C. Zur Reaktionsmischung tropft man 200 mL 4 normale Salzsäure. Unter vermindertem Druck werden 200 mL Lösungsmittel abgedampft. Zum Rückstand gibt man 100 mL gesättigte Kaliumcarbonat-Lösung und 200 mL Methylisobutylketon. Unter gutem Rühren wird das 2 Phasengemisch auf 5°C gekühlt. Das Produkt wird abgenutscht und unter Rückfluß in 90 mL Methylisobutylketon gelöst. Nach Zugabe von Aktivkohle wird heiß filtriert. Man lässt abkühlen und nutscht den Niederschlag ab. Nach Trocknen erhält man 16.2g (41% der Theorie) an trans-Verbindung **19**
DC (Methylenchlorid/Ethanol/Ammoniak = 9:1:0.1) R_{f}= 0.39

### Herstellung von 20

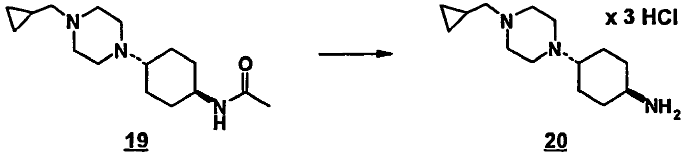

Eine Lösung von 44g (157 mmol) **19** in 500 mL 24prozentiger Salzsäure wird 6 Stunden zum Rückfluß erwärmt. Das Lösungsmittel wird unter vermindertem Druck eingedampft und der Rückstand aus 700 mL Isopropanol kristallisiert. Der Niederschlag wird abgenutscht, mit tert.-Butylmethylether gewaschen und bei 60°C im Vakuumtrockenschrank getrocknet. Man erhält 54.7g Produkt **20** als Trihydrochlorid (enthält 5% Wasser).

### Herstellung von 14c

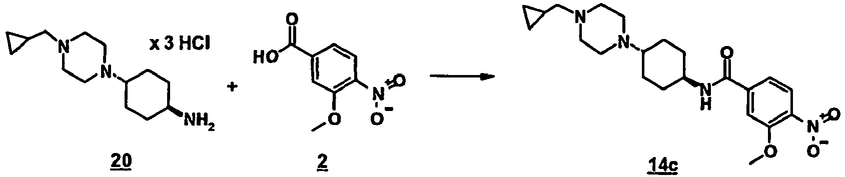

33g (90.4 mmol) 3-Methoxy-4-nitrobenzoesäure **2** werden in 80 mL Toluol suspendiert. Man gibt 0.5 mL Dimethylformamid und 16g (134 mmol) Thionylchlorid zu. Man erwärmt 1 Stunde unter Rückfluß. Die Lösung wird unter vermindertem Druck eingedampft und das rohe Säurechlorid in 50 mL Tetrahydrofuran gelöst. Die Lösung tropft man auf eine Suspension aus 18.7g (94.9 mmol, 95%ig) **20** Trihydrochlorid und 49g (397 mmol) Düsopropylethylamin in 150 mL Tetrahydrofuran unter Kühlung im Eisbad. Die Vollständigkeit der Umsetzung wird mittels DC kontrolliert. Nach beendeter Umsetzung gibt man Wasser zur Suspension und stellt durch Zugabe von Natronlauge einen pH-Wert von 10 ein. Die organische Phase wird abgetrennt und mit gesättigter Kochsalz-Lösung gewaschen. Die vereinigten wässrigen Phasen werden einmal mit Tetrahydrofuran extrahiert. Die vereinigten organischen Phasen werden unter vermindertem Druck eingedampft. Der Rückstand wird in 300 mL tert.-Butylmethylether zum Rückfluß erwärmt. Man läßt auf 20°C abkühlen und nutscht den Niederschlag ab. Nach Trocknen im Vakuumtrockenschrank bei 45°C erhält man 31.3g (83% der Theorie) **14c**.

## Patentansprüche

1. Verfahren zur Herstellung von Dihydropteridinonen der allgemeinen Formel (I) 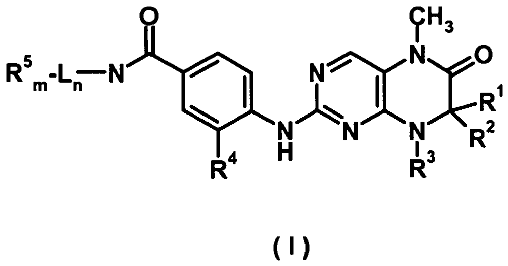 worin
R¹, R² gleich oder verschieden, Wasserstoff oder gegebenenfalls substituiertes C₁-C₆-Alkyl, oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktem C₃-C₁₂-Cycloalkyl, C₃-C₁₂-Cycloalkenyl, C₇-C₁₂-Polycycloalkyl, C₇-C₁₂-Polycycloalkenyl, C₅-C₁₂-Spirocycloalkyl, C₃-C₁₂-Heterocycloalkyl, das 1 bis 2 Heteroatome enthält, und C₃-C₁₂-Heterocycloalkenyl, das 1 bis 2 Heteroatome enthält, oder
R¹ und R³ oder R² und R³ gemeinsam eine gesättigte oder ungesättigte C₃-C₄-Alkylbrücke, die gegebenenfalls 1 Heteroatom enthalten kann,
R⁴ ein Rest ausgewählt aus der Gruppe bestehend aus Wasserstoff, -CN, Hydroxy, -NR₆R₇ und Halogen, oder
ein Rest ausgewählt aus der Gruppe bestehend aus gegebenenfalls substituiertem C₁-C₆-Alkyl, C₂-C₆-Alkenyl, C₂-C₆-Alkinyl, C₁-C₅-Alkyloxy, C₂-C₅-Alkenyloxy, C₂-C₅-Alkinyloxy, C₁-C₆-Alkylthio, C₁-C₆-Alkylsulfoxo und C₁-C₆-Alkylsulfonyl,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, -C₆-C₁₄-Aryl-C₁-C₄-Alkyl, gegebenenfalls verbrücktem C₃-C₁₂-Cycloalkyl und Heteroaryl, das gegebenenfalls 1 oder 2 Stickstoffatome enthält,
n 0 oder 1
m 1 oder 2
R⁵ ein Rest, ausgewählt aus der Gruppe bestehend aus gegebenenfalls substituiertem Morpholinyl, Piperidinyl, Piperazinyl, Piperazinylcarbonyl, Pyrrolidinyl, Tropenyl, R⁸-Diketomethylpiperazinyl, Sulfoxomorpholinyl, Sulfonylmorpholinyl, Thiomorpholinyl, -NR⁸R⁹ und Azacycloheptyl,
R⁶, R⁷ gleich oder verschieden, Wasserstoff oder C₁-C₄-Alkyl,
und
R⁸, R⁹ unsubstituierte Stickstoffsubstituenten an R⁵, gleich oder verschieden, entweder Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-,
bedeuten,
**dadurch gekennzeichnet, dass** eine Verbindung der Formel (II) 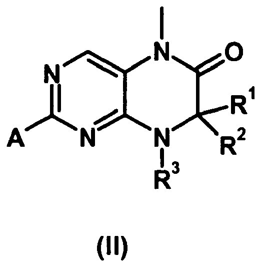 worin
R¹ bis R³ die angegebene Bedeutung aufweisen und A eine Abgangsgruppe ist,
mit einer Verbindung der Formel (III) 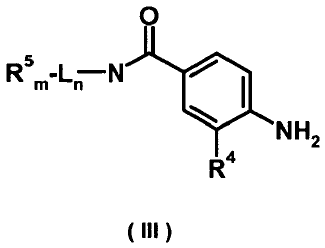 worin
R⁴, R⁵, L und m, n die angegebene Bedeutung aufweisen können,
in Gegenwart organischer Sulfonsäuren umgesetzt wird.

2. Verfahren nach Anspruch 1, **dadurch gekennzeichnet, dass** R₅ ein N-Cyclopropylmethylpiperazinyl Rest darstellt.

3. Verfahren nach Anspruch 1, **dadurch gekennzeichnet, dass** die Dihydropteridinone ausgewählt ist aus der Gruppe bestehend aus folgende Dihydropteridinone der allgemeinen Formel (I)
| | | | | | | |
|---|---|---|---|---|---|---|
| 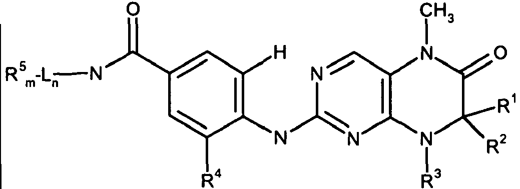 | | | | | | |
| Bsp. | R¹ | R² | Konfig. R¹ od. R² | R³ | R⁴ | Lₙ-R⁵ₘ |
|---|---|---|---|---|---|---|
| 27 | H | 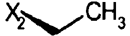 | R | 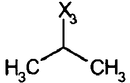 | 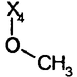 | 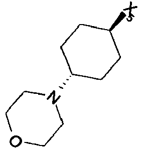 |
| 44 | H | 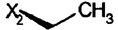 | R | 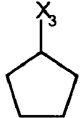 | H | 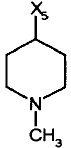 |
| 55 | H | 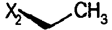 | R | 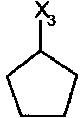 | 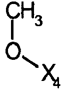 | 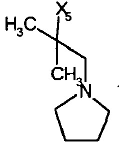 |
| 58 | H | 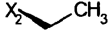 | R | 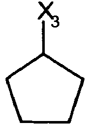 | 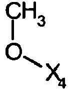 | 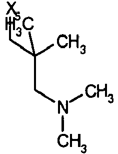 |
| 102 | H | 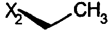 | R | 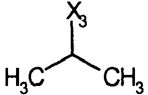 | 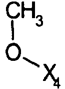 | 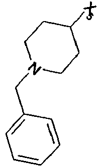 |
| 103 | H | 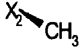 | R | 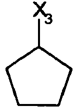 | 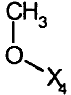 | 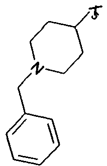 |
| 105 | H | 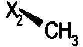 | R | 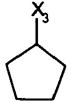 | 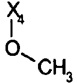 | 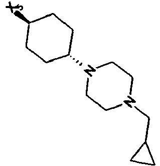 |
| 110 | H | 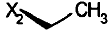 | R | 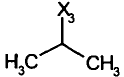 | 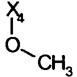 | 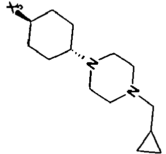 |
| 115 | H | 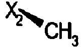 | R | 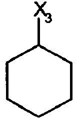 | 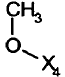 | 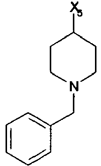 |
| 133 | H | 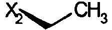 | R | 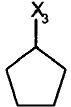 | 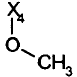 | 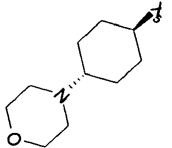 |
| 134 | H | 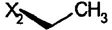 | R | 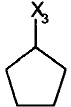 | 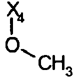 | 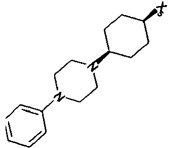 |
| 234 | H | 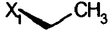 | R | 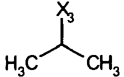 | 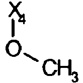 | 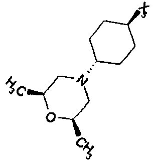 |
| 240 | H | 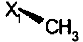 | R | 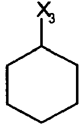 | 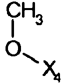 | 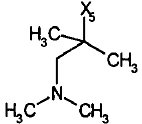 |
wobei die in der Tabelle verwendeten Abkürzungen X₁, X₂, X₃, X₄ und X₅ stehen jeweils für eine Verknüpfung mit einer Position in der unter der Tabelle aufgeführten allgemeinen Formel anstelle der entsprechenden Reste R¹, R², R³, R⁴ und L-R⁵.

4. Verfahren nach Anspruch 1, **dadurch gekennzeichnet, dass** die Dihydropteridinone ausgewählt ist aus der Gruppe bestehend aus folgende Dihydropteridinone der allgemeinen Formel (I)
| | | | | | | |
|---|---|---|---|---|---|---|
| 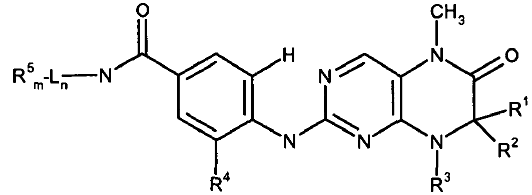 | | | | | | |
| Bsp. | R¹ | R² | Konfig. R¹ od. R² | R³ | R⁴ | Lₙ-R⁵ₘ |
|---|---|---|---|---|---|---|
| 110 | H | 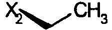 | R | 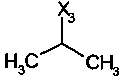 | 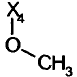 | 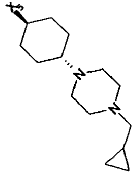 |
wobei die in der Tabelle verwendeten Abkürzungen X₁, X₂, X₃, X₄ und X₅ stehen jeweils für eine Verknüpfung mit einer Position in der unter der Tabelle aufgeführten allgemeinen Formel anstelle der entsprechenden Reste R¹, R², R³, R⁴ und L-R⁵.

5. Verfahren nach einem der Ansprüche 1 bis 4, **dadurch gekennzeichnet, dass** die Reaktion in einem Lösungsmittel wie Dimethylformanid, Dimethylacetamid, N-Methylpyrrolidinon, 1,3-Dimethyl-3,4,5,6-tetrahydro-2(1H)-pyrimidon (DMPU), Dimethylsulfoxid, Sulfolan, Methanol, Ethanol, 1-Propanol, 1-Butanol , 1-Pentanol, 2-Propanol, 2-Butanol, einem isomeren sekundären Alkohol des Pentans oder Hexans, einem tertiären Alkohol des Butans, Pentans oder Hexans, Acetonitril oder 2-Propylnitril durchgeführt wird.

6. Verfahren nach einem der Ansprüche 1 bis 5, **dadurch gekennzeichnet, dass** die Reaktionstemperatur zwischen 18 °C und 180 °C liegt.

7. Verbindung der Formel

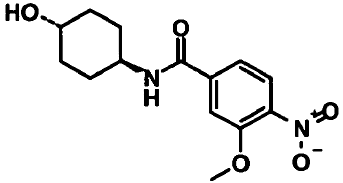

8. Verbindung der Formel

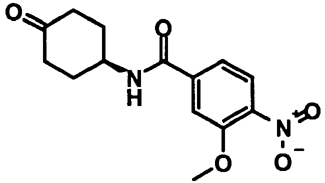

9. Verbindung der Formel 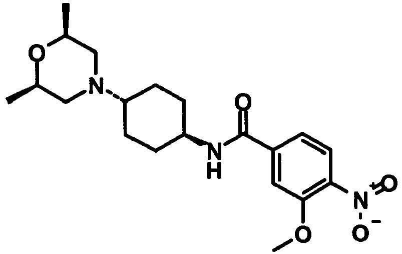 oder deren Salze.

10. Verbindung der Formel 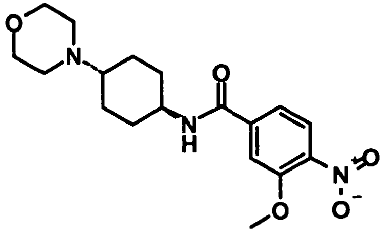 oder deren Salze.

11. Verbindung der Formel 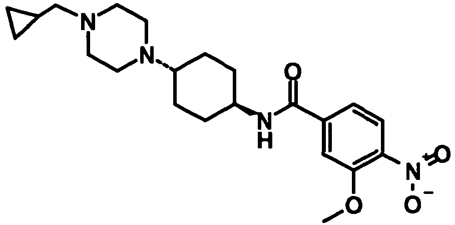 oder deren Salze.

12. Verbindung der Formel 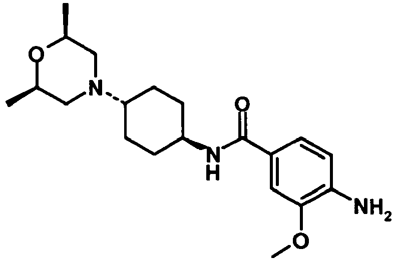 oder deren Salze.

13. Verbindung der Formel 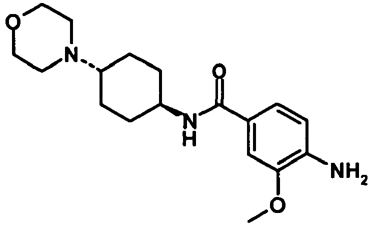 oder deren Salze.

14. Verbindung der Formel 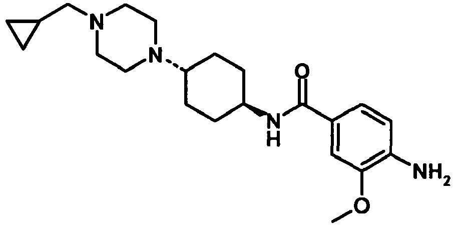 oder deren Salze.

15. Verbindung der Formel 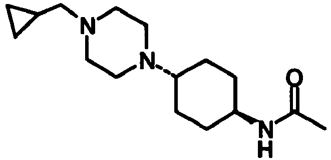 oder deren Salze.

## Claims

1. Method for the production of dihydropteridinones of general formula (I) 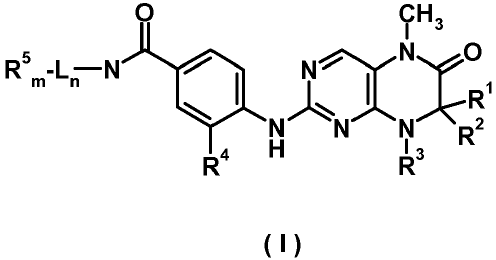 wherein
R¹, R² which may be identical or different, denote hydrogen or optionally substituted C₁-C₆-alkyl,
or
R¹ and R² together denote a 2- to 5-membered alkyl bridge which may contain 1 to 2 heteroatoms,
R³ denotes hydrogen or a group selected from among optionally substituted C₁-C₁₂-alkyl, C₂-C₁₂-alkenyl, C₂-C₁₂-alkynyl and C₆-C₁₄-aryl, or
a group selected from among optionally substituted and/or bridged C₃-C₁₂-cycloalkyl, C₃-C₁₂-cycloalkenyl, C₇-C₁₂-polycycloalkyl, C₇-C₁₂-polycycloalkenyl, C₅-C₁₂-spirocycloalkyl, C₃-C₁₂-heterocycloalkyl which contains 1 to 2 heteroatoms, and C₃-C₁₂-heterocycloalkenyl which contains 1 to 2 heteroatoms, or
R¹ and R³ or R² and R³ together denote a saturated or unsaturated C₃-C₄-alkyl bridge which may optionally contain 1 heteroatom,
R⁴ denotes a group selected from among hydrogen, -CN, hydroxy, -NR₆R₇ and halogen, or
a group selected from among optionally substituted C₁-C₆-alkyl, C₂-C₆-alkenyl, C₂-C₆-alkynyl, C₁-C₅-alkyloxy, C₂-C₅-alkenyloxy, C₂-C₅-alkynyloxy, C₁-C₆-alkylthio, C₁-C₆-alkylsulphoxo and C₁-C₆-alkylsulphonyl,
L denotes a linker selected from among optionally substituted C₂-C₁₀-alkyl, C₂C₁₀-alkenyl, C₆-C₁₄-aryl, -C₂-C₄-alkyl-C₆-C₁₄-aryl, -C₆-C₁₄-aryl-C₁-C₄-alkyl, optionally bridged C₃-C₁₂-cycloalkyl and heteroaryl which optionally contains 1 or 2 nitrogen atoms,
n denotes 0 or 1
m denotes 1 or 2
R⁵ denotes a group selected from among optionally substituted morpholinyl, piperidinyl, piperazinyl, piperazinylcarbonyl, pyrrolidinyl, tropenyl, R⁸-diketomethylpiperazinyl, sulphoxomorpholinyl, sulphonylmorpholinyl, thiomorpholinyl, -NR³R⁹ and azacycloheptyl, R⁶, R⁷, which may be identical or different, denote hydrogen or C₁-C₄-alkyl,
and
R⁸, R⁹ denotes unsubstituted nitrogen substituents at R⁵, which may be identical or different, either hydrogen or a group selected from among C₁-C₆-alkyl, -C₁-C₄-alkyl-C₃-C₁₀-cycloalkyl, C₃-C₁₀-cycloalkyl, C₆-C₁₄-aryl, -C₁-C₄-alkyl-C₆-C₁₄-aryl, pyranyl, pyridinyl, pyrimidinyl, C₁-C₄-alkyloxycarbonyl, C₆-C₁₄-arylcarbonyl-, C₁-C₄-alkylcarbonyl-, C₆-C₁₄-arylmethyloxycarbonyl-, C₆-C₁₄-arylsulphonyl-, C₁-C₄-alkylsulphonyl- and C₆-C₁₄-aryl-C₁-C₄-alkylsulphonyl-,
**characterised in that** a compound of formula (II) 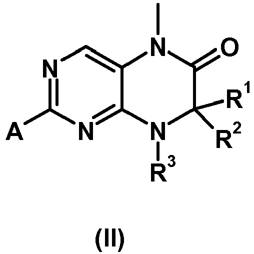 wherein
R¹ to R³ are defined as specified and A is a leaving group,
is reacted with a compound of formula (III) 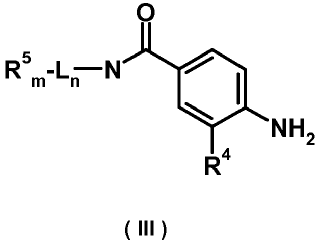 wherein
R⁴, R⁵, L and m, n may be defined as specified,
in the presence of organic sulphonic acids.

2. Method according to claim 1, **characterised in that** R₅ denotes an N-cyclopropylmethylpiperazinyl group.

3. Method according to claim 1, **characterised in that** the dihydropteridinone is selected from among the following dihydropteridinones of general formula (I)
| | | | | | | |
|---|---|---|---|---|---|---|
| 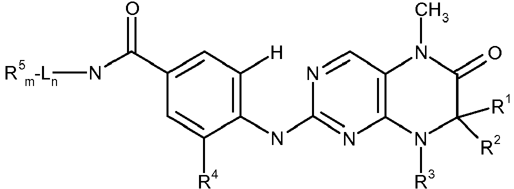 | | | | | | |
| Ex. | R¹ | R² | config. R¹ or R² | R³ | R⁴ | Lₙ-R⁵ₘ |
|---|---|---|---|---|---|---|
| 27 | H | 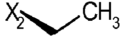 | R | 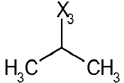 | 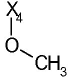 | 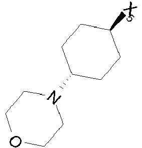 |
| 44 | H | 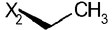 | R | 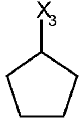 | H | 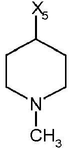 |
| 55 | H | 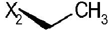 | R | 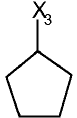 | 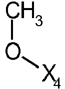 | 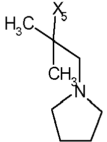 |
| 58 | H | 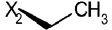 | R | 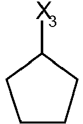 | 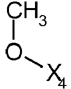 | 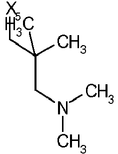 |
| 102 | H | 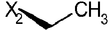 | R | 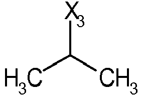 | 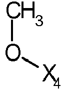 | 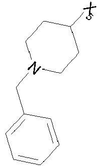 |
| 103 | H | 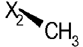 | R | 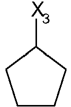 | 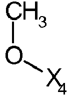 | 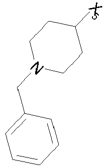 |
| 105 | H | 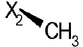 | R | 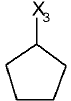 | 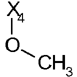 | 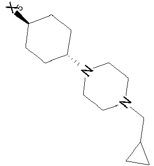 |
| 110 | H | 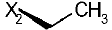 | R | 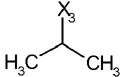 | 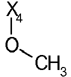 | 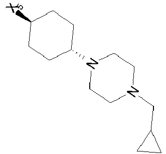 |
| 115 | H | 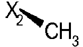 | R | 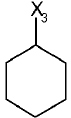 | 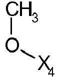 | 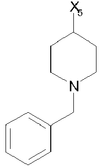 |
| 133 | H | 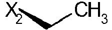 | R | 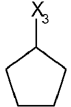 | 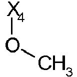 | 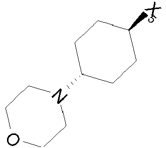 |
| 134 | H | 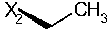 | R | 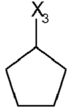 | 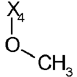 | 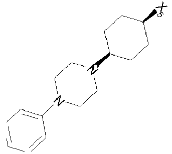 |
| 234 | H | 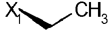 | R | 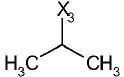 | 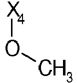 | 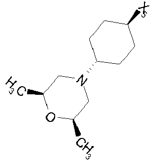 |
| 240 | H | 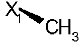 | R | 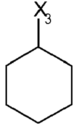 | 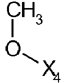 | 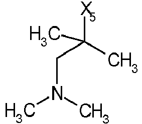 |
while the abbreviations X₁, X₂, X₃, X₄ and X₅ used in the Table each denote a link to a position in the general formula shown in the Table instead of the corresponding groups R¹, R², R³, R⁴ and L-R⁵.

4. Method according to claim 1, **characterised in that** the dihydropteridinone is selected from among the following dihydropteridinones of general formula (I)
| | | | | | | |
|---|---|---|---|---|---|---|
| 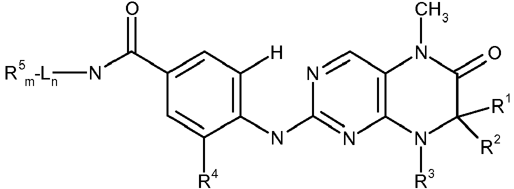 | | | | | | |
| Ex. | R¹ | R² | config. R¹ or R² | R³ | R⁴ | Lₙ-R⁵ₘ |
|---|---|---|---|---|---|---|
| 110 | H | 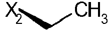 | R | 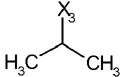 | 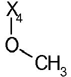 | 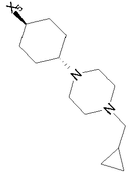 |
while the abbreviations X₁, X₂, X₃, X₄ and X₅ used in the Table each denote a link to a position in the general formula shown in the Table instead of the corresponding groups R¹, R², R³, R⁴ and L-R⁵.

5. Method according to one of claims 1 to 4, **characterised in that** the reaction is carried out in a solvent such as dimethylformamide, dimethylacetamide, N-methylpyrrolidinone, 1,3-dimethyl-3,4,5,6-tetrahydro-2(1H)-pyrimidone (DMPU), dimethylsulphoxide, sulpholane, methanol, ethanol, 1-propanol, 1-butanol, 1-pentanol, 2-propanol, 2-butanol, an isomeric secondary alcohol of pentane or hexane, a tertiary alcohol of butane, pentane or hexane, acetonitrile or 2-propylnitrile.

6. Method according to one of claims 1 to 5, **characterised in that** the reaction temperature is between 18°C and 180°C.

7. Compound of the formula

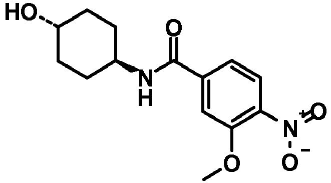

8. Compound of the formula

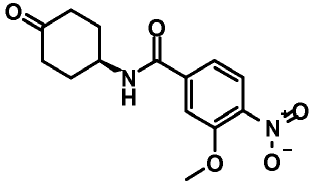

9. Compound of the formula 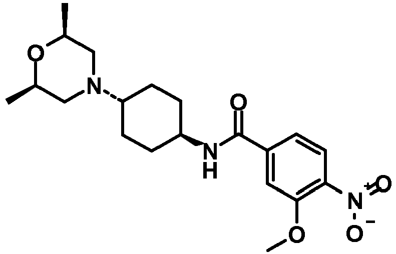 or the salts thereof.

10. Compound of the formula 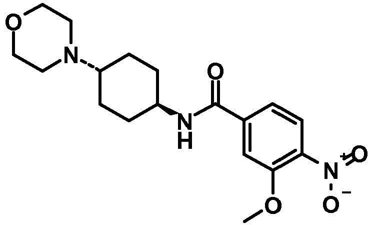 or the salts thereof.

11. Compound of the formula 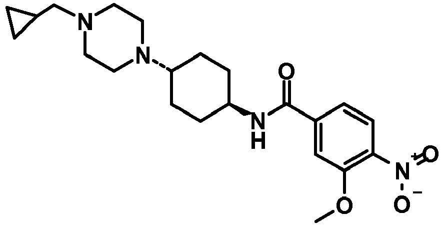 or the salts thereof.

12. Compound of the formula 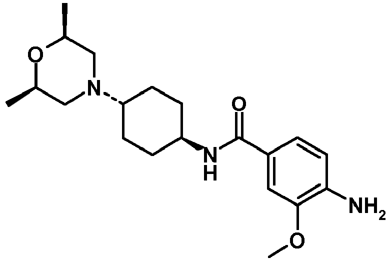 or the salts thereof.

13. Compound of the formula 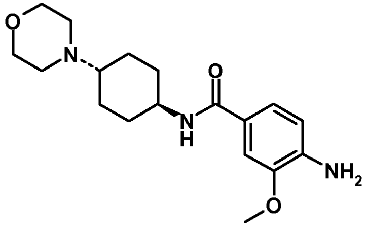 or the salts thereof.

14. Compound of the formula 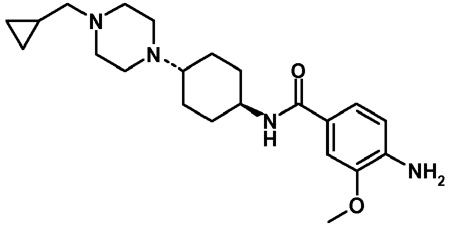 or the salts thereof.

15. Compound of the formula 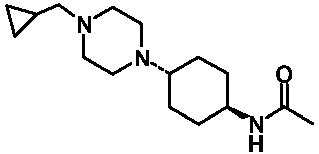 or the salts thereof.

## Revendications

1. Procédé de production de dihydroptéridinones de formule générale (I) 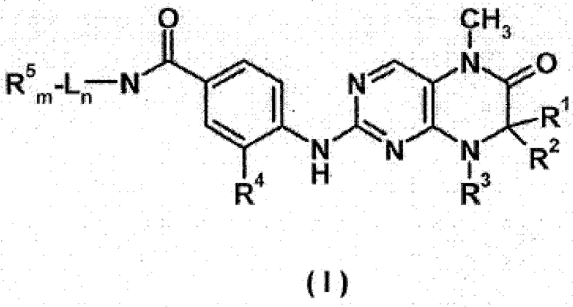 dans laquelle
R¹, R², identiques ou différents, représentent un atome d'hydrogène ou un groupe alkyle en C₁ à C₆ éventuellement substitué,
ou
R¹ et R² représentent conjointement un pont alkyle de 2 à 5 chaînons qui peut contenir 1 à 2 hétéroatomes,
R³ représente un atome d'hydrogène ou un radical choisi dans le groupe consistant en groupes éventuellement substitués alkyle en C₁ à C₁₂, alcényle en C₂ à C₁₂, alcinyle en C₂ à C₁₂ et aryle en C₆ à C₁₄, ou un radical choisi dans le groupe consistant en groupes éventuellement substitués et/ou pontés, cycloalkyle en C₃ à C₁₂, cycloalcényle en C₃ à C₁₂, polycycloalkyle en C₇ à C₁₂, polycycloalcényle en C₇ à C₁₂, spirocycloalkyle en C₅ à C₁₂, hétérocycloalkyle en C₃ à C₁₂ qui contient 1 à 2 hétéroatomes, et hétérocycloalcényle en C₃ à C₁₂ qui contient 1 à 2 hétéroatomes, ou
R¹ et R³ ou R² et R³ représentent conjointement un pont alkyle en C₃ à C₄ saturé ou insaturé qui peut contenir éventuellement 1 hétéroatome,
R⁴ représente un radical choisi dans le groupe consistant en atome d'hydrogène, -CN, un groupe hydroxy, -NR₆R₇ et un atome d'halogène, ou
un radical choisi dans le groupe consistant en groupes entuellement substitués alkyle en C₁ à C₆, alcényle en C₂ à C₆, alcinyle en C₂ à C₆, alkyloxy en C₁ à C₅, alcényloxy en C₂ à C₅, alcinyloxy en C₂ à C₅, alkylthio en C₁ à C₆, alkylsulfoxo en C₁ à C₆ et alkylsulfonyle en C₁ à C₆ év,
L représente un segment de liaison choisi dans le groupe consistant en groupes éventuellement substitués alkyle en C₂ à C₁₀, alcényle en C₂ à C₁₀, aryle en C₆ à C₁₄, alkyle en C₂ à C₄-aryle en C₆ à C₁₄, aryle en C₆ à C₁₄-alkyle en C₁ à C₄, cycloalkyle en C₃ à C₁₂ éventuellement ponté et hétéroaryle qui contient éventuellement 1 ou 2 atomes d'azote,
n est 0 ou 1
m est 1 ou 2
R⁵ représente un radical choisi dans les groupes éventuellement substitués consistant en groupes morpholinyle, pipéridinyle, pipérazinyle, pipérazinylcarbonyle, pyrrolidinyle, propényle, R⁸-dicétométhylpipérazinyle, sulfoxo-morpholinyle, sulfonylmorpholinyle, thiomorpholinyle, -NR⁸R⁹ et azacycloheptyle,
R⁶, R⁷ identiques ou différents, représentent un atome d'hydrogène ou un groupe alkyle en C₁ à C₄,
et
R⁸, R⁹ représentent des substituants de l'azote non substitués sur R⁵, identiques ou différents, soit un atome d'hydrogène soit un radical choisi dans le groupe consistant en groupes alkyle en C₁ à C₆, (alkyle en C₁ à C₄)-(cycloalkyle en C₃ à C₁₀), cycloalkyle en C₃ à C₁₀, aryle en C₆ à C₁₄, (alkyle en C₁ à C₄)-(aryle en C₆ à C₁₄), pyranyle, pyridinyle, pyrimidinyle, alkyloxycarbonyle en C₁ à C₄, arylcarbonyle en C₆ à C₁₄, alkylcarbonyle en C₁ à C₄, arylméthyloxycarbonyle en C₆ à C₁₄, arylsulfonyle en C₆ à C₁₄, alkylsulfonyle en C₁ à C₄ et (aryle en C₆ à C₁₄)-(alkylsulfonyle en C₁ à C₄),
**caractérisé en ce que** l'on fait réagir un composé de formule (II) 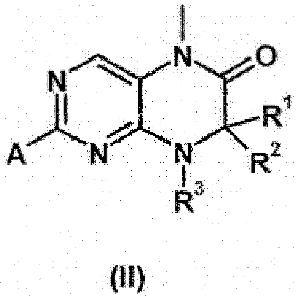 dans laquelle
R¹ à R³ présentent la signification indiquée et A est un groupe partant
avec un composé de formule (III) 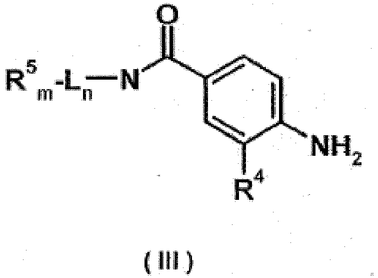 dans laquelle
R⁴, R⁵, L et m, n peuvent présenter la signification indiquée,
en présence d'acides sulfoniques organiques.

2. Procédé selon la revendication 1, **caractérisé en ce que** R⁵ désigne un radical N-cyclopropylméthylpipérazinyle.

3. Procédé selon la revendication 1, **caractérisé en ce que** la dihydroptéridinone est choisie dans le groupe consistant en dihydroptéridinones de formule générale (I) suivantes
| | | | | | | |
|---|---|---|---|---|---|---|
| 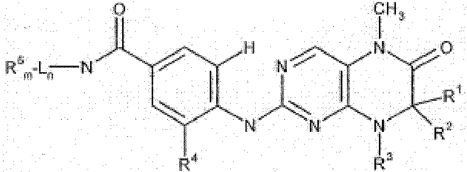 | | | | | | |
| Ex. | R¹ | R² | Config. R¹ ou R² | R³ | R⁴ | Lₙ-R⁵ₘ |
|---|---|---|---|---|---|---|
| 27 | H | 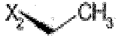 | R | 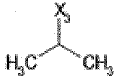 | 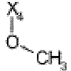 | 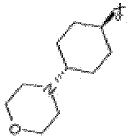 |
| 44 | H | 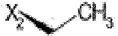 | R | 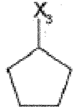 | H | 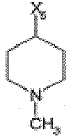 |
| 55 | H | 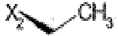 | R | 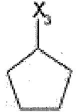 | 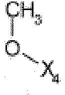 | 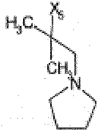 |
| 58 | H | 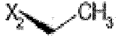 | R | 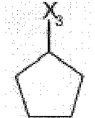 | 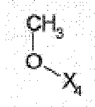 | 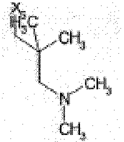 |
| 102 | H | 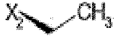 | R | 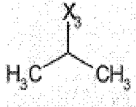 | 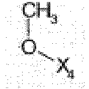 | 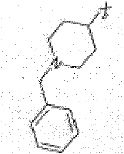 |
| 103 | H | 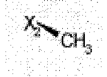 | R | 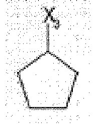 | 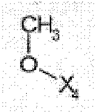 | 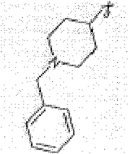 |
| 105 | H | 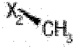 | R | 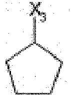 | 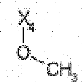 | 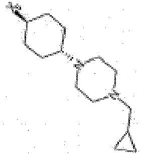 |
| 110 | H | 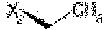 | R | 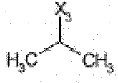 | 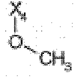 | 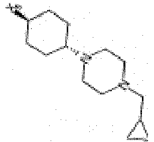 |
| 115 | H | 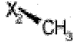 | R | 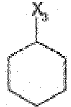 | 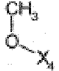 | 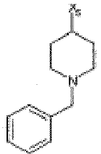 |
| 133 | H | 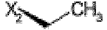 | R | 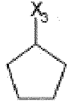 | 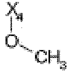 | 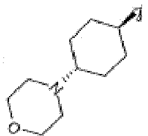 |
| 134 | H | 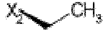 | R | 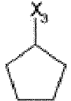 | 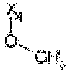 | 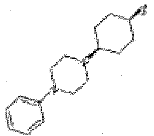 |
| 234 | H | 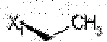 | R | 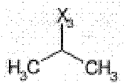 | 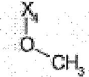 | 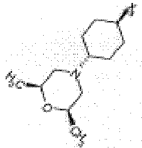 |
| 240 | H | 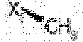 | R | 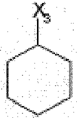 | 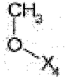 | 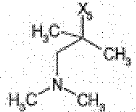 |
les abréviations X₁, X₂, X₃, X₄ et X₅ utilisées dans le tableau désignant respectivement une liaison avec une position dans la formule générale indiquée dans le tableau au lieu des radicaux R¹, R², R³, R⁴ et L-R⁵ correspondants.

4. Procédé selon la revendication 1, **caractérisé en ce que** la dihydroptéridinone est choisie dans le groupe consistant en les dihydroptéridinones de formule générale (I) suivantes
| | | | | | | |
|---|---|---|---|---|---|---|
| 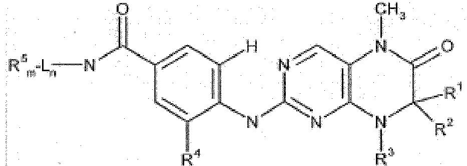 | | | | | | |
| Ex. | R¹ | R² | Config. R¹ ou R² | R³ | R⁴ | Lₙ-R⁵ₘ |
|---|---|---|---|---|---|---|
| 110 | H | 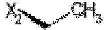 | R | 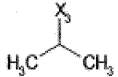 | 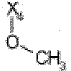 | 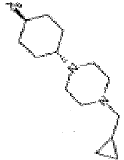 |
les abréviations X₁, X₂, X₃, X₄ et X₅ utilisées dans le tableau désignant respectivement une liaison avec une position dans la formule générale indiquée dans le tableau au lieu des radicaux R¹, R², R³, R⁴ et L-R⁵ correspondants.

5. Procédé selon l'une des revendications 1 à 4, **caractérisé en ce que** la réaction est réalisée dans un solvant tel que le diméthylformamide, le diméthylacétamide, la N-méthylpyrrolidone, la 1,3-diméthyl-3,4,5,6-tétrahydro-2(1H)-pyrimidone (DMPU), le diméthylsulfoxyde, le sulfolane, le méthanol, l'éthanol, le 1-propanol, le 1-butanol, le 1-pentanol, le 2-propanol, le 2-butanol, un alcool secondaire isomère du pentane ou de l'hexane, un alcool tertiaire du butane, du pentane ou de l'hexane, l'acétonitrile ou le 2-propylniltrile.

6. Procédé selon l'une des revendications 1 à 5, **caractérisé en ce que** la température réactionnelle se situe entre 18° C et 180° C.

7. Composé de formule

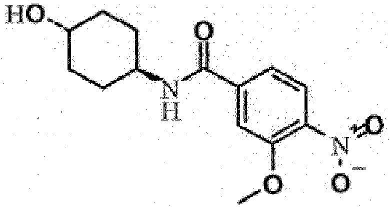

8. Composé de formule

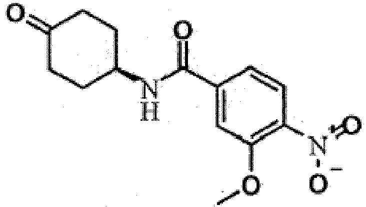

9. Composé de formule 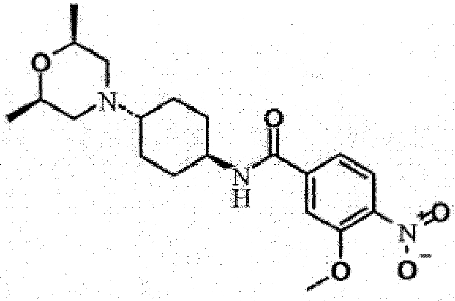 ou ses sels.

10. Composé de formule 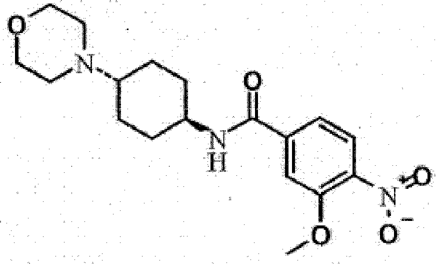 ou ses sels.

11. Composé de formule 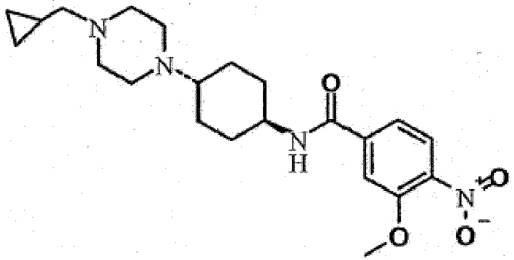 ou ses sels.

12. Composé de formule 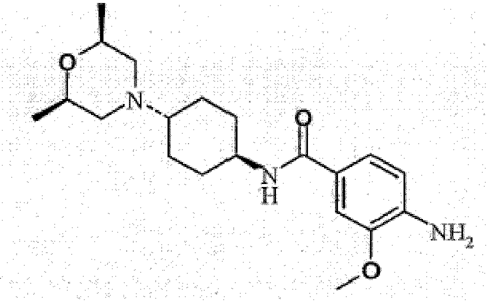 ou ses sels.

13. Composé de formule 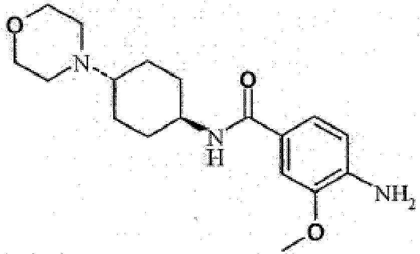 ou ses sels.

14. Composé de formule 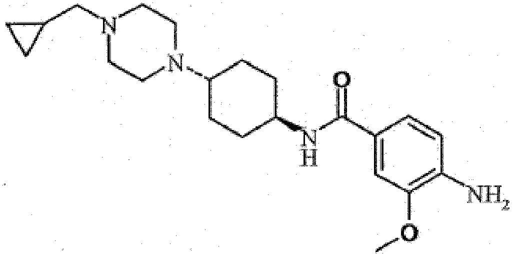 ou ses sels.

15. Composé de formule 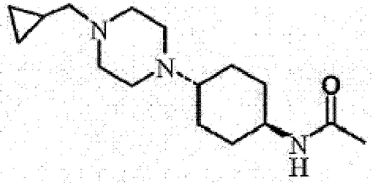 ou ses sels.
